# EUROPÄISCHE PATENTSCHRIFT

(11) **EP 1 399 423 B1**
(45) Veröffentlichungstag und Bekanntmachung des Hinweises auf die Patenterteilung: **14.11.2007**
(21) Anmeldenummer: 02745333.1
(22) Anmeldetag: 31.05.2002
(51) Int. Cl.: C07D 215/36, A61K 31/33, A61P 9/06, C07D 401/12, C07D 333/34, C07D 405/12, C07D 261/10, C07D 271/12, C07D 213/71

(54) **ANTHRANILSÄUREAMIDE MIT HETEROARYLSULFONYL-SEITENKETTE UND IHRE VERWENDUNG ALS ANTIARRHYTHMISCHE WIRKSTOFFE**
ANTHRANILIC ACID AMIDES WITH A HETEROARYLSULFONYL SIDE CHAIN, METHOD FOR THE PRODUCTION THEREOF, USE THEREOF AS A MEDICAMENT OR DIAGNOSTIC AGENT AND PHARMACEUTICAL PREPARATIONS CONTAINING SAID COMPOUNDS
AMIDES D'ACIDE ANTHRANILIQUE COMPORTANT UNE CHAINE LATERALE HETEROARYLSULFONYLE, PROCEDE DE FABRICATION, UTILISATION EN TANT QU'AGENT PHARMACEUTIQUE OU DIAGNOSTIQUE, ET PREPARATIONS PHARMACEUTIQUES CONTENANT CES AMIDES

(30) Priorität: 12.06.2001 DE 10128331
(43) Veröffentlichungstag der Anmeldung: 24.03.2004
(73) Patentinhaber: Sanofi-Aventis Deutschland GmbH, 65929 Frankfurt am Main (DE)
(72) Erfinder: BRENDEL, Joachim, 61118 Bad Vilbel (DE); BÖHME, Thomas, 65428 Rüsselsheim (DE); PEUKERT, Stefan, 60313 Frankfurt (DE); KLEEMANN, Heinz-Werner, 65474 Bischofsheim (DE)
(86) Internationale Anmeldenummer: PCT/EP2002/005956
(87) Internationale Veröffentlichungsnummer: WO 2002/100825

(56) Entgegenhaltungen:
- DE-A- 19 947 457
- US-A- 6 150 356

## Beschreibung

Anthranilsäureamide mit Heteroarylsulfonyl-Seitenkette, Verfahren zu ihrer Herstellung, ihre Verwendung als Medikament oder Diagnostikum sowie sie enthaltende pharmazeutische Zubereitungen

Die Erfindung betrifft Verbindungen der Formel I, 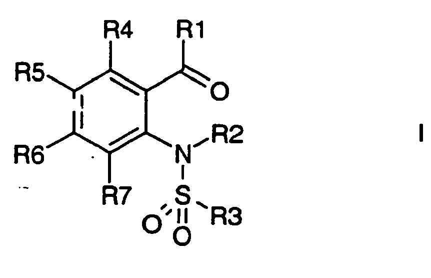 worin R(1), R(2), R(3), R(4), R(5), R(6) und R(7) die im folgenden angegebenen Bedeutungen haben, sowie deren Verwendung, insbesondere in Arzneimitteln,
- R(1): 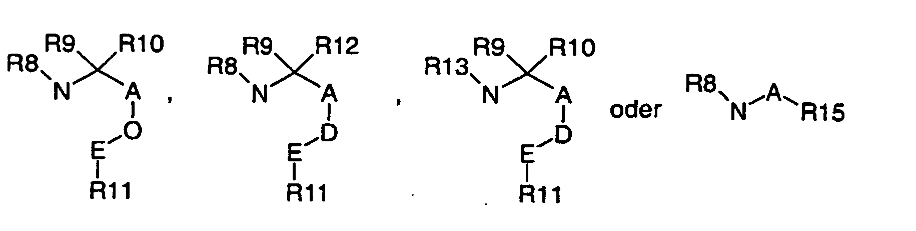 A -CₙH₂ₙ-;
n = 0, 1, 2, 3, 4 oder 5;
D eine Bindung oder -O-;
E -CₘH₂ₘ-;
m = 0, 1, 2, 3, 4 oder 5;
R(8) Wasserstoff , Alkyl mit 1, 2, 3 oder 4 C-Atomen oder CₚH₂ₚ-R(14);
p 0, 1, 2, 3, 4 oder 5;
R(14) Phenyl, Naphthyl oder Heteroaryl,
wobei Phenyl, Naphthyl und Heteroaryl unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(9) Wasserstoff oder Alkyl mit 1, 2, 3, 4, 5 oder 6 C-Atomen;
R(10) Wasserstoff, Alkyl mit 1, 2, 3 oder 4 C-Atomen, Phenyl, Naphthyl oder Heteroaryl
wobei Phenyl, Naphthyl und Heteroaryl unsubstituiert sind oder substituiert mit 1, 2 oder 3 Substituenten ausgewählt aus der Gruppe bestehend aus F, Cl, Br, l, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(11) Cycloalkyl mit 3, 4, 5 oder 6 C-Atomen, Phenyl, Naphthyl, Thienyl, Furyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl,
wobei Phenyl, Naphthyl, Thienyl, Furyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl unsubstituiert sind oder substituiert mit 1, 2 oder 3 Substituenten ausgewählt aus der Gruppe bestehend aus F, Cl, Br, I, CF₃, OCF₃, NO₂, CN, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(12) Alkyl mit 1, 2, 3 oder 4 C-Atomen, Alkinyl mit 1, 2, 3 oder 4 C-Atomen, Cycloalkyl mit 3, 4, 5 oder 6 C-Atomen, Phenyl, Naphthyl oder Heteroaryl,
wobei Phenyl, Naphthyl und Heteroaryl unsubstituiert sind oder substituiert mit 1, 2 oder 3 Substituenten ausgewählt aus der Gruppe bestehend aus F, Cl, Br, I, CF₃, OCF₃, NO₃, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(13) CₚH₂ₚ-R(14);
p 0, 1, 2, 3, 4 oder 5;
R(15) Cycloalkyl mit 3, 4, 5, 6, 7 oder 8 C-Atomen;
- R(2): Wasserstoff oder Alkyl mit 1, 2, 3 oder 4 C-Atomen;
- R(3): Heteroaryl,
wobei Heteroaryl unsubstituiert ist oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
- R(4), R(5), R(6) und R(7): unabhängig voneinander Wasserstoff, F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
sowie ihre pharmazeutisch verträglichen Salze.

Bevorzugt sind Verbindungen der Formel I, worin bedeuten:
- R(1): 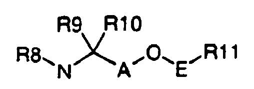 A -CₙH₂ₙ-;
n 0, 1, 2 oder 3;
E -CₘH₂m-;
m 0, 1, 2 oder 3;
R(8) Wasserstoff, Alkyl mit 1, 2 oder 3 C-Atomen oder CₚH₂ₚ-R(14);
p 0, 1. 2, oder 3;
R(14) Phenyl, Naphthyl oder Heteroaryl,
wobei Phenyl, Naphthyl und Heteroaryl unsubstituiert sind oder substituiert mit 1, 2 oder 3 Substituenten ausgewählt aus der Gruppe bestehend aus F, Cl, CF₃, OCF₃, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(9) Wasserstoff oder Alkyl mit 1, 2, 3 oder 4 C-Atomen;
R(10) Wasserstoff, Alkyl mit 1, 2 oder 3 C-Atomen, Phenyl, Naphthyl oder Heteroaryl,
wobei Phenyl, Naphthyl und Heteroaryl unsubstituiert sind oder substituiert mit 1, 2 oder 3 Substituenten ausgewählt aus der Gruppe bestehend aus F, Cl, CF₃, OCF₃, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(11) Phenyl, Naphthyl, Thienyl, Furyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl,
wobei Phenyl, Naphthyl, Thienyl, Furyl , Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl unsubstituiert sind oder substituiert mit 1, 2 oder 3 Substituenten ausgewählt aus der Gruppe bestehend aus F, Cl, CF₃, OCF₃, CN, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
- R(2): Wasserstoff oder Alkyl mit 1, 2 oder 3 C-Atomen;
- R(3): Heteroaryl,
wobei Heteroaryl unsubstituiert ist oder substituiert mit 1, 2 oder 3 Substituenten ausgewählt aus der Gruppe bestehend aus F, Cl, CF₃, OCF₃, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
- R(4), R(5), R(6) und R(7): unabhängig voneinander Wasserstoff, F, Cl, CF₃, OCF₃, CN, COMe, OH, Alkyl mit 1, 2, 3 oder 4 C-Atomen, Methoxy, Ethoxy, Dimethylamino, Sulfamoyl, Methylsulfonyl und Methylsulfonylamino;
sowie ihre pharmazeutisch verträglichen Salze.

### Besonders bevorzugt sind Verbindungen der Formel I, worin bedeuten:

- R(1): 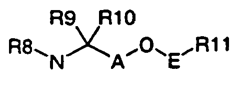 A -CₙH₂ₙ-;
n 0 oder 1;
E -CₘH₂ₘ-;
m 0 oder 1;
R(8) Wasserstoff , Alkyl mit 1, 2 oder 3 C-Atomen oder CₚH₂ₚ-R(14);
p 0 oder 1;
R(14) Phenyl, Naphthyl oder Heteroaryl,
wobei Phenyl, Naphthyl und Heteroaryl unsubstituiert sind oder substituiert mit 1 oder 2 Substituenten ausgewählt aus der Gruppe bestehend aus F, Cl, CF₃, OCF₃, CN, COMe, Alkyl mit 1, 2, 3 oder 4 C-Atomen, Methoxy, Ethoxy, Dimethylamino, Sulfamoyl, Methylsulfonyl und Methylsulfonylamino;
R(9) Wasserstoff, Methyl oder Ethyl;
R(10) Wasserstoff, Alkyl mit 1,2 oder 3 C-Atomen, Phenyl, Naphthyl oder Heteroaryl,
wobei Phenyl, Naphthyl und Heteroaryl unsubstituiert sind oder substituiert mit 1 oder 2 Substituenten ausgewählt aus der Gruppe bestehend aus F, Cl, CF₃, OCF₃, CN, COMe, Alkyl mit 1, 2, 3 oder 4 C-Atomen, Methoxy, Ethoxy, Dimethylamino, Sulfamoyl, Methylsulfonyl und Methylsulfonylamino;
R(11) Phenyl, Naphthyl, Pyridyl, Pyrazinyl, Pyrimidinyl, .Pyridazinyl, Indolyl, Indazolyl, Chinolyl, Isochinolyl, Phthalazinyl, Chinoxalinyl, Chinazolinyl oder Cinnolinyl,
wobei Phenyl, Naphthyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl unsubstituiert sind oder substituiert mit 1 oder 2 Substituenten ausgewählt aus der Gruppe bestehend aus F, Cl, CF₃, OCF₃, CN, COMe, Alkyl mit 1, 2, 3 oder 4 C-Atomen, Methoxy, Ethoxy, Dimethylamino, Sulfamoyl, Methylsulfonyl und Methylsulfonylamino;
- R(2): Wasserstoff, Methyl oder Ethyl;
- R(3): Heteroaryl,
wobei Heteroaryl unsubstituiert ist oder substituiert mit 1 oder 2 Substituenten ausgewählt aus der Gruppe bestehend aus F, Cl, CF₃, OCF₃, CN, COMe, Alkyl mit 1, 2, 3 oder 4 C-Atomen, Methoxy, Ethoxy, Dimethylamino, Sulfamoyl, Methylsulfonyl und Methylsulfonylamino;
- R(4), R(5), R(6) und R(7): unabhängig voneinander Wasserstoff, F, Cl, CF₃, OCF₃, CN, COMe, OH, Alkyl mit 1, 2, 3 oder 4 C-Atomen, Methoxy, Ethoxy, Dimethylamino, Sulfamoyl, Methylsulfonyl und Methylsulfonylamino;
sowie ihre pharmazeutisch verträglichen Salze.

Ganz besonders bevorzugt sind Verbindungen I, worin bedeuten:
- R(1): 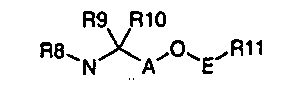 A -CₙH₂ₙ-;
n 0 oder 1;
E -CₘH₂ₘ-;
m 0 oder 1;
R(8) Wasserstoff , Alkyl mit 1, 2 oder 3 C-Atomen oder CₚH₂ₚ-R(14);
p 0 oder 1;
R(14) Phenyl, Naphthyl oder Heteroaryl,
wobei Phenyl, Naphthyl und Heteroaryl unsubstituiert sind oder substituiert mit 1 oder 2 Substituenten ausgewählt aus der Gruppe bestehend aus F, Cl, CF₃, OCF₃, CN, COMe, Methyl, Methoxy, Ethoxy, Dimethylamino, Sulfamoyl oder Methylsulfonyl;
R(9) Wasserstoff, Methyl oder Ethyl;
R(10) Wasserstoff, Alkyl mit 1, 2 oder 3 C-Atomen, Phenyl, Naphthyl oder Heteroaryl,
wobei Phenyl, Naphthyl und Heteroaryl unsubstituiert sind oder substituiert mit 1 oder 2 Substituenten ausgewählt aus der Gruppe bestehend aus F, Cl, CF₃, OCF₃, CN, COMe, Methyl, Methoxy, Ethoxy, Dimethylamino, Sulfamoyl oder Methylsulfonyl;
R(11) Phenyl, Naphthyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl,
wobei Phenyl, Naphthyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl unsubstituiert sind oder substituiert mit 1 oder 2 Substituenten ausgewählt aus der Gruppe bestehend aus F, Cl, CF₃, OCF₃, CN, COMe, Methyl, Methoxy, Ethoxy, Dimethylamino, Sulfamoyl oder Methylsulfonyl;
- R(2): Wasserstoff;
- R(3): Heteroaryl,
wobei Heteroaryl unsubstituiert ist oder substituiert mit 1 oder 2 Substituenten ausgewählt aus der Gruppe bestehend aus F, Cl, CF₃, OCF₃, CN, COMe, Methyl, Methoxy, Ethoxy, Dimethylamino, Sulfamoyl oder Methylsulfonyl;
- R(4): Wasserstoff, F, Cl, CF₃, Methyl oder Methoxy;
- R(5): Wasserstoff, F, Cl, CF₃, Methyl, Methoxy, COMe, OCF₃, CN oder OH;
- R(6): Wasserstoff, F, Cl, CF₃, Methyl, Methoxy oder OH;
- R(7): Wasserstoff, F, Cl, CF₃, Methyl, Ethyl, Methoxy oder OH;
sowie ihre pharmazeutisch verträglichen Salze.

Ebenfalls bevorzugt sind Verbindungen der Formel I, worin bedeuten:
- R(1): 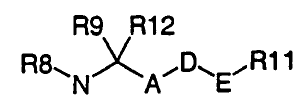 A -CₙH₂ₙ-;
n 0, 1, 2 oder 3;
D eine Bindung oder -O-;
E -CₘH₂ₘ-;
m 0, 1, 2 oder 3;
R(8) Wasserstoff , Alkyl mit 1, 2 oder 3 C-Atomen oder CₚH₂p-R(14)
p 0, 1, 2, oder 3;
R(14) Phenyl, Naphthyl oder Heteroaryl,
wobei Phenyl, Naphthyl und Heteroaryl unsubstituiert sind oder substituiert mit 1, 2 oder 3 Substituenten ausgewählt aus der Gruppe bestehend aus F, Cl, CF₃, OCF₃, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(9) Wasserstoff oder Alkyl mit 1, 2, 3 oder 4 C-Atomen;
R(11) Phenyl, Naphthyl, Pyridyl, Pyrazinyl, Pyrimidinyl, Pyridazinyl, Indolyl, Indazolyl, Chinolyl, lsochinolyl, Phthalazinyl, Chinoxalinyl, Chinazolinyl oder Cinnolinyl
wobei Phenyl, Naphthyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl unsubstituiert sind oder substituiert mit 1, 2 oder 3 Substituenten ausgewählt aus der Gruppe bestehend aus F, Cl, CF₃, OCF₃, CN, COMe, NH₂, OH, Alkyl mit 1, 2,3 oder 4 C-Atomen, Alkoxy mit 1, 2, 3 oder 4 C-Atomen, Dimethylamino, Sulfamoyl, Methylsulfonyl und Methylsulfonylamino;
R(12) Alkyl mit 1, 2 oder 3 C-Atomen, Alkinyl mit 1, 2 oder 3 C-Atomen, Cycloalkyl mit 3, 4, 5 oder 6 C-Atomen, Phenyl, Naphthyl oder Heteroaryl,
wobei Phenyl, Naphthyl und Heteroaryl unsubstituiert sind oder substituiert mit 1, 2 oder 3 Substituenten ausgewählt aus der Gruppe bestehend aus F, Cl, CF₃, OCF₃, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
- R(2): Wasserstoff oder Alkyl mit 1, 2 oder 3 C-Atomen;
- R(3): Heteroaryl,
wobei Heteroaryl unsubstituiert ist oder substituiert mit 1, 2 oder 3 Substituenten ausgewählt aus der Gruppe bestehend aus F, Cl, CF₃, OCF₃, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
- R(4), R(5), R(6) und R(7): unabhängig voneinander Wasserstoff, F, Cl, CF₃, OCF₃, CN, COMe, OH, Alkyl mit 1, 2, 3 oder 4 C-Atomen, Methoxy, Ethoxy, Dimethylamino, Sulfamoyl, Methylsulfonyl und Methylsulfonylamino;
und ihre pharmakologisch verträglichen Salze.

Ebenfalls besonders bevorzugt sind Verbindungen I, worin bedeuten:
- R(1): 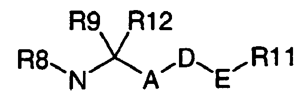 A -CₙH₂ₙ-;
n 0 oder 1;
D eine Bindung oder -O-;
E -CₘH₂ₘ-;
m 0 oder 1;
R(8) Wasserstoff , Alkyl mit 1, 2 oder 3 C-Atomen oder CₚH₂ₚ-R(14)
p 0 oder 1;
R(14) Phenyl, Naphthyl oder Heteroaryl,
wobei Phenyl, Naphthyl und Heteroaryl unsubstituiert sind oder substituiert mit 1 oder 2 Substituenten ausgewählt aus der Gruppe bestehend aus F, Cl, CF₃, OCF₃, CN, COMe, Alkyl mit 1, 2, 3 oder 4 C-Atomen, Methoxy, Ethoxy, Dimethylamino, Sulfamoyl, Methylsulfonyl und Methylsulfonylamino;
R(9) Wasserstoff, Methyl oder Ethyl;
R(11) Phenyl, Naphthyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl,
wobei Phenyl, Naphthyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl unsubstituiert sind oder substituiert mit 1 oder 2 Substituenten ausgewählt aus der Gruppe bestehend aus F, Cl, CF₃, OCF₃, CN, COMe, Alkyl mit 1,2,3 oder 4 C-Atomen, Methoxy, Ethoxy, Dimethylamino, Sulfamoyl, Methylsulfonyl und Methylsulfonylamino;
R(12) Alkyl mit 1, 2 oder 3 C-Atomen, Ethinyl, Cyclopropyl, Phenyl, Naphthyl oder Heteroaryl,
wobei Phenyl, Naphthyl und Heteroaryl unsubstituiert sind oder substituiert mit 1 oder 2 Substituenten ausgewählt aus der Gruppe bestehend aus F, Cl, CF₃, OCF₃, CN, COMe, Alkyl mit 1, 2, 3 oder 4 C-Atomen, Methoxy, Ethoxy, Dimethylamino, Sulfamoyl, Methylsulfonyl und Methylsulfonylamino;
- R(2): Wasserstoff, Methyl oder Ethyl;
- R(3): Heteroaryl,
wobei Heteroaryl unsubstituiert ist oder substituiert mit 1 oder 2 Substituenten ausgewählt aus der Gruppe bestehend aus F, Cl, CF₃, OCF₃, CN, COMe, Alkyl mit 1, 2, 3 oder 4 C-Atomen, Methoxy, Ethoxy, Dimethylamino, Sulfamoyl, Methylsulfonyl und Methylsulfonylamino;
- R(4), R(5), R(6) und R(7): unabhängig voneinander Wasserstoff, F, Cl, CF₃, OCF₃, CN, COMe, OH, Alkyl mit 1, 2, 3 oder 4 C-Atomen, Methoxy, Ethoxy, Dimethylamino, Sulfamoyl, Methylsulfonyl und Methylsulfonylamino;
und ihre pharmakologisch verträglichen Salze.

Ebenfalls ganz besonders bevorzugt sind Verbindungen der Formel 1, worin bedeuten:
- R(1): 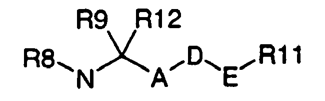 A -CₙH₂ₙ-;
n 0 oder 1;
D eine Bindung oder -O-;
E -CₘH₂ₘ-;
m 0 oder 1;
R(8) Wasserstoff , Alkyl mit 1, 2 oder 3 C-Atomen oder CₚH₂ₚ-R(14)
p 0 oder 1;
R(14) Phenyl, Naphthyl oder Heteroaryl,
wobei Phenyl, Naphthyl und Heteroaryl unsubstituiert sind oder substituiert mit 1 oder 2 Substituenten ausgewählt aus der Gruppe bestehend aus F, Cl, CF₃, OCF₃, CN, COMe, Methyl, Methoxy, Ethoxy, Dimethylamino, Sulfamoyl, Methylsulfonyl und Methylsulfonylamino;
R(9) Wasserstoff, Ethyl oder Methyl;
R(11) Phenyl, Naphthyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl,
wobei Phenyl, Naphthyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl unsubstituiert sind oder substituiert mit 1 oder 2 Substituenten ausgewählt aus der Gruppe bestehend aus F, Cl, CF₃, OCF₃, CN, COMe, Methyl, Methoxy, Ethoxy, Dimethylamino, Sulfamoyl und Methylsulfonyl;
R(12) Alkyl mit 1, 2 oder 3 C-Atomen, Ethinyl, Cyclopropyl, Phenyl, Naphthyl oder Heteroaryl,
wobei Phenyl, Naphthyl und Heteroaryl unsubstituiert sind oder substituiert mit 1 oder 2 Substituenten ausgewählt aus der Gruppe bestehend aus F, Cl, CF₃, OCF₃, CN, COMe, Methyl, Methoxy, Ethoxy, Dimethylamino, Sulfamoyl, Methylsulfonyl und Methylsulfonylamino;
- R(2): Wasserstoff;
- R(3): Heteroaryl,
wobei Heteroaryl unsubstituiert ist oder substituiert mit 1 oder 2 Substituenten ausgewählt aus der Gruppe bestehend aus F, Cl, CF₃, OCF₃, CN, COMe, Methyl, Methoxy, Ethoxy, Dimethylamino, Sulfamoyl und Methylsulfonyl;
- R(4): Wasserstoff, F, Cl, CF₃, Methyl oder Methoxy;
- R(5): Wasserstoff, F, Cl, CF₃, Methyl, Methoxy, COMe, OCF₃, CN oder OH;
- R(6): Wasserstoff, F, Cl, CF₃, Methyl oder Methoxy oder OH;
- R(7): Wasserstoff, F, Cl, CF₃, Methyl, Ethyl, Methoxy oder OH;
und ihre pharmakologisch verträglichen Salze.

Desgleichen bevorzugt sind Verbindungen der Formel I, worin bedeuten:
- R(1): 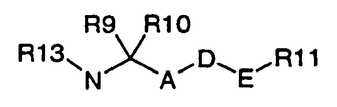 A -CₙH₂ₙ-
n = 0,1, 2 oder 3;
D eine Bindung oder -O-;
E -CₘH₂ₘ-
m 0, 1, 2 oder 3;
R(9) Wasserstoff oder Alkyl mit 1, 2, 3 oder 4 C-Atomen;
R(10) Wasserstoff, Alkyl mit 1, 2 oder 3 C-Atomen, Phenyl, Naphthyl oder Heteroaryl
wobei Phenyl, Naphthyl und Heteroaryl unsubstituiert sind oder substituiert mit 1, 2 oder 3 Substituenten ausgewählt aus der Gruppe bestehend aus F, Cl, CF₃, OCF₃, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(11) Phenyl, Naphthyl, Thienyl, Furanyl , Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl,
wobei Phenyl, Naphthyl, Thienyl, Furanyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl unsubstituiert sind oder substituiert mit 1, 2 oder 3 Substituenten ausgewählt aus der Gruppe bestehend aus F, Cl, CF₃, OCF₃, CN, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(13) CₚH₂ₚ-R(14);
p 0, 1, 2 oder 3;
R(14) Phenyl, Naphthyl oder Heteroaryl,
wobei Phenyl, Naphthyl und Heteroaryl unsubstituiert sind oder substituiert mit 1, 2 oder 3 Substituenten ausgewählt aus der Gruppe bestehend aus F, Cl, CF₃, OCF₃, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(2) Wasserstoff oder Alkyl mit 1, 2 oder 3 C-Atomen;
R(3) Heteroaryl,
wobei Heteroaryl unsubstituiert ist oder substituiert mit 1, 2 oder 3. Substituenten ausgewählt aus der Gruppe bestehend aus F, Cl, CF₃, OCF₃, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
- R(4), R(5), R(6) und R(7): unabhängig voneinander Wasserstoff, F, Cl, CF₃, OCF₃, CN, COMe, OH, Alkyl mit 1, 2, 3 oder 4 C-Atomen, Methoxy, Ethoxy, Dimethylamino, Sulfamoyl, Methylsulfonyl und Methylsulfonylamino;
sowie ihre pharmazeutisch verträglichen Salze.

Desgleichen besonders bevorzugt sind Verbindungen der Formel 1, worin bedeuten:
- R(1): 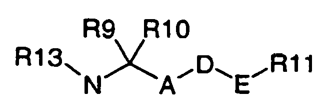 A -CₙH₂ₙ-;
n 0 oder 1;
D eine Bindung oder -O-;
E -CₘH₂ₘ-
m 0 oder 1;
R(9) Wasserstoff, Methyl oder Ethyl;
R(10) Wasserstoff, Alkyl mit 1, 2 oder 3 C-Atomen, Phenyl, Naphthyl oder Heteroaryl,
wobei Phenyl, Naphthyl und Heteroaryl unsubstituiert sind oder substituiert mit 1 oder 2 Substituenten ausgewählt aus der Gruppe bestehend aus F, Cl, CF₃, OCF₃, CN, COMe, Alkyl mit 1,2,3 oder 4 C-Atomen, Methoxy, Ethoxy, Dimethylamino, Sulfamoyl, Methylsulfonyl und Methylsulfonylamino;
R(11) Phenyl, Naphthyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl,
wobei Phenyl, Naphthyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl unsubstituiert sind oder substituiert mit 1 oder 2 Substituenten ausgewählt aus der Gruppe bestehend aus F, Cl, CF₃, OCF₃, CN, COMe. Alkyl mit 1, 2, 3 oder 4 C-Atomen, Methoxy, Ethoxy, Dimethylamino, Sulfamoyl und Methylsulfonyl und Methylsulfonylamino;
R(13) CₚH₂ₚ-R(14);
p 0 oder 1;
R(14) Phenyl, Naphthyl oder Heteroaryl,
wobei Phenyl, Naphthyl und Heteroaryl unsubstituiert sind oder substituiert mit 1 oder 2 Substituenten ausgewählt aus der Gruppe bestehend aus F, Cl, CF₃, OCF₃, CN, COMe, Alkyl mit 1, 2, 3 oder 4 C-Atomen, Methoxy, Ethoxy, Dimethylamino, Sulfamoyl und Methylsulfonyl und Methylsulfonylamino;
- R(2): Wasserstoff, Methyl oder Ethyl;
- R(3): Heteroaryl,
wobei Heteroaryl unsubstituiert ist oder substituiert mit 1 oder 2 Substituenten ausgewählt aus der Gruppe bestehend aus F, Cl, CF₃, OCF₃, CN, COMe, Alkyl mit 1, 2, 3 oder 4 C-Atomen, Methoxy, Ethoxy, Dimethylamino, Sulfamoyl und Methylsulfonyl oder Methylsulfonylamino;
- R(4), R(5), R(6) und R(7): unabhängig voneinander Wasserstoff, F, Cl, CF₃, OCF₃, CN, COMe, OH, Alkyl mit 1, 2, 3 oder 4 C-Atomen, Methoxy, Ethoxy, Dimethylamino, Sulfamoyl, Methylsulfonyl und Methylsulfonylamino;
sowie ihre pharmazeutisch verträglichen Salze.

Desgleichen ganz besonders bevorzugt sind Verbindungen der Formel I, worin bedeuten:
- R(1): 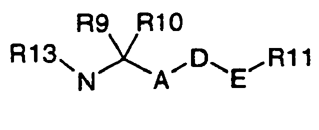 A -CₙH₂ₙ-;
n 0 oder 1;
D eine Bindung oder -O-;
E -CₘH₂ₘ-;
m 0 oder 1;
R(9) Wasserstoff, Ethyl oder Methyl;
R(10) Wasserstoff, Alkyl mit 1, 2 oder 3 C-Atomen, Phenyl, Naphthyl oder Heteroaryl,
wobei Phenyl, Naphthyl und Heteroaryl unsubstituiert sind oder substituiert mit 1 oder 2 Substituenten ausgewählt aus der Gruppe bestehend aus F, Cl, CF₃, OCF₃, CN, COMe, Methyl, Methoxy, Ethoxy, Dimethylamino, Sulfamoyl, Methylsulfonyl;
R(11) Phenyl, Naphthyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl
wobei Phenyl, Naphthyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl unsubstituiert sind oder substituiert mit 1 oder 2 Substituenten ausgewählt aus der Gruppe bestehend aus F, Cl, CF₃, OCF₃, CN, COMe, Methyl, Methoxy, Ethoxy, Dimethylamino, Sulfamoyl, Methylsulfonyl;
R(13) CₚH₂ₚ-R(14);
p 0 oder 1;
R(14) Phenyl, Naphthyl oder Heteroaryl,
wobei Phenyl, Naphthyl und Heteroaryl unsubstituiert sind oder substituiert mit 1 oder 2 Substituenten ausgewählt aus der Gruppe bestehend aus F, Cl, CF₃, OCF₃, CN, COMe, Methyl, Methoxy, Ethoxy, Dimethylamino, Sulfamoyl, Methylsulfonyl;
- R(2): Wasserstoff;
- R(3): Heteroaryl,
wobei Heteroaryl unsubstituiert ist oder substituiert mit 1 oder 2 Substituenten ausgewählt aus der Gruppe bestehend aus F, Cl, CF₃, OCF₃, CN, COMe, Methyl, Methoxy, Ethoxy, Dimethylamino, Sulfamoyl, Methylsulfonyl;
- R(4): Wasserstoff, F, Cl, CF₃, Methyl, Methoxy;
- R(5): Wasserstoff, F, Cl, CF₃, Methyl, Methoxy, COMe, OCF₃, CN, OH;
- R(6): Wasserstoff, F, Cl, CF₃, Methyl, Methoxy, OH;
- R(7): Wasserstoff, F, Cl, CF₃, Methyl, Ethyl, Methoxy, OH;
sowie ihre pharmazeutisch verträglichen Salze.

Desgleichen bevorzugt sind Verbindungen der Formel I, worin bedeuten:
- R(1): 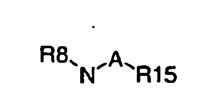 A -CₙH₂ₙ-;
n =0, 1, 2 oder 3
R(8) Wasserstoff , Alkyl mit 1, 2 oder 3 C-Atomen oder CₚH₂ₚ-R(14);
p 0, 1, 2, oder 3;
R(14) Phenyl, Naphthyl oder Heteroaryl,
wobei Phenyl, Naphthyl und Heteroaryl unsubstituiert sind oder substituiert mit 1, 2 oder 3 Substituenten ausgewählt aus der Gruppe bestehend aus F, Cl, CF₃, OCF₃, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
- R(2): Wasserstoff oder Alkyl mit 1, 2 oder 3 C-Atomen;
- R(3): Heteroaryl,
wobei Heteroaryl unsubstituiert ist oder substituiert mit 1, 2 oder 3 Substituenten ausgewählt aus der Gruppe bestehend aus F, Cl, CF₃, OCF₃, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
- R(4), R(5), R(6) und R(7): unabhängig voneinander Wasserstoff, F, Cl, CF₃, OCF₃, CN, COMe, OH, Alkyl mit 1, 2, 3 oder 4 C-Atomen, Methoxy, Ethoxy, Dimethylamino, Sulfamoyl, Methylsulfonyl und Methylsulfonylamino;
- R(15): Cycloalkyl mit 3, 4, 5, 6 oder 7 C-Atomen;
sowie ihre pharmazeutisch verträglichen Salze.

Desgleichen besonders bevorzugt sind Verbindungen der Formel I, worin bedeuten:
- R(1): 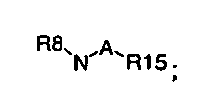 A -CₙH₂ₙ-;
n 0 oder 1;
R(8) Wasserstoff , Alkyl mit 1, 2 oder 3 C-Atomen oder CₚH₂ₚ-R(14);
p 0 oder 1;
R(14) Phenyl, Naphthyl oder Heteroaryl,
wobei Phenyl, Naphthyl und Heteroaryl unsubstituiert sind oder substituiert mit 1 oder 2 Substituenten ausgewählt aus der Gruppe bestehend aus F, Cl, CF₃, OCF₃, CN, COMe, Alkyl mit 1, 2, 3 oder 4 C-Atomen, Methoxy, Ethoxy, Dimethylamino, Sulfamoyl, Methylsulfonyl und Methylsulfonylamino;
- R(2): Wasserstoff, Methyl oder Ethyl;
- R(3): Heteroaryl,
wobei Heteroaryl unsubstituiert ist oder substituiert mit 1 oder 2 Substituenten ausgewählt aus der Gruppe bestehend aus F, Cl, CF₃, OCF₃, CN, COMe, Alkyl mit 1, 2, 3 oder 4 C-Atomen, Methoxy, Ethoxy, Dimethylamino, Sulfamoyl, Methylsulfonyl und Methylsulfonylamino;
- R(4), R(5), R(6) und R(7): unabhängig voneinander Wasserstoff, F, Cl, CF₃, OCF₃, CN, COMe, OH, Alkyl mit 1, 2, 3 oder 4 C-Atomen, Methoxy, Ethoxy, Dimethylamino, Sulfamoyl, Methylsulfonyl und Methylsulfonylamino;
R(15) Cycloalkyl mit 3, 4, 5, 6 oder 7 C-Atomen;
sowie ihre pharmazeutisch verträglichen Salze.

Desgleichen ganz besonders bevorzugt sind Verbindungen der Formel 1, worin bedeuten:
- R(1): 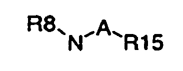 A -CₙH₂n-;
n 0 oder 1;
R(8) Wasserstoff , Alkyl mit 1, 2 oder 3 C-Atomen oder CₚH₂ₚ-R(14);
p 0 oder 1;
R(14) Phenyl, Naphthyl oder Heteroaryl,
wobei Phenyl, Naphthyl und Heteroaryl unsubstituiert sind oder substituiert mit 1 oder 2 Substituenten ausgewählt aus der Gruppe bestehend aus F, Cl, CF₃, OCF₃, CN, COMe, Methyl, Methoxy, Ethoxy, Dimethylamino, Sulfamoyl und Methylsulfonyl;
- R(2): Wasserstoff;
- R(3): Heteroaryl,
wobei Heteroaryl unsubstituiert ist oder substituiert mit 1 oder 2 Substituenten ausgewählt aus der Gruppe bestehend aus F, Cl, CF₃, OCF₃, CN, COMe, Methyl, Methoxy, Ethoxy, Dimethylamino, Sulfamoyl oder Methylsulfonyl;
- R(4): Wasserstoff, F, Cl, CF₃, Methyl oder Methoxy;
- R(5): Wasserstoff, F, Cl, CF₃, Methyl, Methoxy, COMe, OCF₃; CN oder OH;
- R(6): Wasserstoff, F, Cl, CF₃, Methyl, Methoxy oder OH;
- R(7): Wasserstoff, F, Cl, CF₃, Methyl, Ethyl, Methoxy oder OH;
- R(15): Cycloalkyl mit 3, 4, 5, 6 oder 7 C-Atomen;
sowie ihre pharmazeutisch verträglichen Salze.

Unter Heteroaryl werden insbesondere Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind. Des weiteren können auch ein oder beide Atome der Kondensationsstelle bicyclischer Reste (wie im Indolizinyl) N-Atome sein.

Als Heteroaryl gelten insbesondere Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl.
Alkylreste und Alkylenreste können geradkettig oder verzweigt sein. Dies gilt auch für die Alkylenreste der Formeln CₘH₂ₘ, CₙH₂ₙ, und CₚH₂ₚ. Alkylreste und Alkylenreste können auch geradkettig oder verzweigt sein, wenn sie substituiert sind oder in anderen Resten enthalten sind, z. B. in einem Alkoxyrest oder in einem fluorierten Alkylrest. Beispiele für Alkylreste sind Methyl, Ethyl, n-Propyl, Isopropyl, n-Butyl, Isobutyl, sec-Butyl, tert-Butyl, n-Pentyl, Isopentyl, Neopentyl, n-Hexyl, 3,3-Dimethylbutyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Eicosyl. Die von diesen Resten abgeleiteten zweiwertigen Reste, z. B. Methylen, 1,1-Ethylen, 1,2-Ethylen, 1,1-Propylen, 1,2-Propylen, 2,2-Propylen, 1,3-Propylen, 1,1-Butylen, 1,4-Butylen, 1,5-Pentylen, 2,2-Dimethyl-1,3-propylen, 1,6-Hexylen, usw. sind Beispiele für Alkylenreste.

Enthalten die Verbindungen der Formel I eine oder mehrere saure oder basische Gruppen bzw. einen oder mehrere basische oder saure Heterocyclen, so gehören auch die entsprechenden physiologisch oder toxikologisch verträglichen Salze zur Erfindung, insbesondere die pharmazeutisch verwendbaren Salze. So können die Verbindungen der Formel I, die saure Gruppen, z. B. eine oder mehrere COOH-Gruppen, tragen, beispielsweise als Alkalimetallsalze, vorzugsweise Natrium- oder Kaliumsalze, oder als Erdalkalimetallsalze, z. B. Calcium- oder Magnesiumsalze, oder als Ammoniumsalze, z. B. als Salze mit Ammoniak oder organischen Aminen oder Aminosäuren, verwendet werden. Verbindungen der Formel I, die eine oder mehrere basische, d. h. protonierbare, Gruppen tragen oder einen oder mehrere basische heterocyclische Ringe enthalten, können auch in Form ihrer physiologisch verträglichen Säureadditionssalze mit anorganischen oder organischen Säuren verwendet werden, beispielsweise als Hydrochloride, Phosphate, Sulfate, Methansulfonate, Acetate, Lactate, Maleinate, Fumarate, Malate, Gluconate usw. Enthalten die Verbindungen der Formel I gleichzeitig saure und basische Gruppen im Molekül, so gehören neben den geschilderten Salzformen auch innere Salze, sogenannte Betaine, zu der Erfindung. Salze können aus den Verbindungen der Formel I nach üblichen Verfahren erhalten werden, beispielsweise durch Vereinigung mit einer Säure bzw. Base in einem Lösungs- oder Dispergiermittel oder auch durch Anionenaustausch aus anderen Salzen.

Die Verbindungen der Formel I können bei entsprechender Substitution in stereoisomeren Formen vorliegen. Enthalten die Verbindungen der Formel I ein oder mehrere Asymmetriezentren, so können diese unabhängig voneinander die S-Konfiguration oder die R-Konfiguration aufweisen. Zur Erfindung gehören alle möglichen Stereoisomeren, z. B. Enantiomere oder Diastereomere, und Mischungen von zwei oder mehr stereoisomeren Formen, z. B. Enantiomeren und/oder Diastereomeren, in beliebigen Verhältnissen. Enantiomere z. B. gehören also in enantiomerenreiner Form, sowohl als links- als auch als rechtsdrehende Antipoden, und auch in Form von Mischungen der beiden Enantiomeren in unterschiedlichen Verhältnissen oder in Form von Racematen zu der Erfindung. Die Herstellung von einzelnen Stereoisomeren kann gewünschtenfalls durch Auftrennung eines Gemisches nach üblichen Methoden oder z. B. durch stereoselektive Synthese erfolgen. Bei Vorliegen von beweglichen Wasserstoffatomen umfasst die vorliegende Erfindung auch alle tautomeren Formen der Verbindungen der Formel I.

Die erfindungsgemäßen Verbindungen der Formel I und ihre physiologisch verträglichen Salze können am Tier, bevorzugt am Säugetier, und insbesondere am Menschen als Arzneimittel für sich allein, in Mischungen untereinander oder in Form von pharmazeutischen Zubereitungen verwendet werden. Gegenstand der vorliegenden Erfindung sind auch die Verbindungen der Formel I und ihre physiologisch verträglichen Salze zur Anwendung als Arzneimittel, ihre Verwendung in der Therapie und Prophylaxe der genannten Krankheitsbilder und ihre Verwendung zur Herstellung von Medikamenten dafür und von Medikamenten mit K⁺-Kanal-blockierender Wirkung. Weiterhin sind Gegenstand der vorliegenden Erfindung pharmazeutische Zubereitungen, die als aktiven Bestandteil eine wirksame Dosis mindestens einer Verbindung der Formel I und/oder eines physiologisch verträglichen Salzes davon neben üblichen, pharmazeutisch einwandfreien Träger- und Hilfsstoffen enthalten. Die pharmazeutischen Zubereitungen enthalten normalerweise 0,1 bis 90 Gewichtsprozent der Verbindungen der Formel I und/oder ihrer physiologisch verträglichen Salze. Die Herstellung der pharmazeutischen Zubereitungen kann in an sich bekannter Weise erfolgen. Dazu werden die Verbindungen der Formel I und/oder ihre physiologisch verträglichen Salze zusammen mit einem oder mehreren festen oder flüssigen galenischen Trägerstoffen und/oder Hilfsstoffen und, wenn gewünscht, in Kombination mit anderen Arzneimittelwirkstoffen in eine geeignete Darreichungsform bzw. Dosierungsform gebracht, die dann als Arzneimittel in der Humanmedizin oder Veterinärmedizin verwendet werden kann.

Arzneimittel, die erfindungsgemäße Verbindungen der Formel I und/oder ihre physiologisch verträglichen Salze enthalten, können oral, parenteral, z. B intravenös, rektal, durch Inhalation oder topisch appliziert werden, wobei die bevorzugte Applikation vom Einzelfall, z. B. dem jeweiligen Erscheinungsbild der zu behandelnden Erkrankung, abhängig ist.

Welche Hilfsstoffe für die gewünschte Arzneimittelformulierung geeignet sind, ist dem Fachmann auf Grund seines Fachwissens geläufig. Neben Lösemitteln, Gelbildnern, Suppositoriengrundlagen, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Mittel zur Erzielung eines Depoteffekts, Puffersubstanzen oder Farbstoffe verwendet werden.

Die Verbindungen der Formel I können zur Erzielung einer vorteilhaften therapeutischen Wirkung auch mit anderen Arzneiwirkstoffen kombiniert werden. So sind in der Behandlung von Herz-Kreislauferkrankungen vorteilhafte Kombinationen mit herz-kreislaufaktiven Stoffen möglich. Als derartige, für Herz-Kreislauferkrankungen vorteilhafte Kombinationspartner kommen beispielsweise andere Antiarrhythmika, so Klasse I-, Klasse II- oder Klasse III-Antiarrhythmika, in Frage, wie beispielsweise IK_{S}- oder IK_{I}-Kanalblocker, z.B. Dofetilid, oder weiterhin blutdrucksenkende Stoffe wie ACE-Inhibitoren (beispielsweise Enalapril, Captopril, Ramipril), Angiotensin-Antagonisten, K⁺-Kanalaktivatoren, sowie alpha- und beta-Rezeptorenblocker, aber auch sympathomimetische und adrenerg wirkende Verbindungen, sowie Na⁺/H⁺-Austausch-Inhibitoren, Calciumkanalantagonisten, Phosphodiesterasehemmer und andere positiv inotrop wirkende Stoffe, wie z. B. Digitalisglykoside, oder Diuretika.

Für eine orale Anwendungsform werden die aktiven Verbindungen mit den dafür geeigneten Zusatzstoffen, wie Trägerstoffen, Stabilisatoren oder inerten Verdünnungsmittel, vermischt und durch die üblichen Methoden in die geeigneten Darreichungsformen gebracht, wie Tabletten, Dragees, Steckkapseln, wässrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie. Sonnenblumenöl oder Lebertran. Als Lösungsmittel für wässrige oder alkoholische Lösungen kommen z. B. Wasser, Ethanol oder Zuckerlösungen oder Gemische davon, in Betracht. Weitere Hilfsstoffe, auch für andere Applikationsformen, sind z. B. Polyethylenglykole und Polypropylenglykole.

Zur subkutanen oder intravenösen Applikation werden die aktiven Verbindungen, gewünschtenfalls mit den dafür üblichen Substanzen wie Lösungsvermittlern, Emulgatoren oder weiteren Hilfsstoffen, in Lösung, Suspension oder Emulsion gebracht. Die Verbindungen der Formel I und ihre physiologisch verträglichen Salze können auch lyophilisiert werden und die erhaltenen Lyophilisate z. B. zur Herstellung von Injektions- oder Infusionspräparaten verwendet werden. Als Lösungsmittel kommen z. B. Wasser, physiologische Kochsalzlösung oder Alkohole, z. B. Ethanol, Propanol, Glycerin, in Betracht, daneben auch Zuckerlösungen wie Glucose- oder Mannitlösungen, oder auch Mischungen aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen der Wirkstoffe der Formel I oder ihrer physiologisch verträglichen Salze in einem pharmazeutisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gewichtsprozent.

Die Dosierung des zu verabreichenden Wirkstoffs der Formel I bzw. der physiologisch verträglichen Salze davon hängt vom Einzelfall ab und ist wie üblich für eine optimale Wirkung den Gegebenheiten des Einzelfalls anzupassen. So hängt sie natürlich ab von der Häufigkeit der Verabreichung und von der Wirkstärke und Wirkdauer der jeweils zur Therapie oder Prophylaxe eingesetzten Verbindungen, aber auch von Art und Stärke der zu behandelnden Krankheit sowie von Geschlecht, Alter, Gewicht und individueller Ansprechbarkeit des zu behandelnden Menschen oder Tieres und davon, ob akut oder prophylaktisch therapiert wird. Üblicherweise beträgt die tägliche Dosis einer Verbindung der Formel I bei Verabreichung an einem etwa 75 kg schweren Patienten 0.001 mg/kg Körpergewicht bis 100 mg/kg Körpergewicht, bevorzugt 0.01 mg/kg Körpergewicht bis 20 mg/kg Körpergewicht. Die Dosis kann in Form einer Einzeldosis verabreicht werden oder in mehrere, z. B. zwei, drei oder vier Einzeldosen aufgeteilt werden. Insbesondere bei der Behandlung akuter Fälle von Herzrhythmusstörungen, beispielsweise auf einer Intensivstation, kann auch eine parenterale Verabreichung durch Injektion oder Infusion, z. B. durch eine intravenöse Dauerinfusion, vorteilhaft sein.

Die erfindungsgemäßen Verbindungen der Formel I wirken auf den sogenannten Kv1.5-Kalium-Kanal und inhibieren einen als "ultra-rapidly activating delayed rectifier" bezeichneten Kaliumstrom im humanen Herzvorhof. Die Verbindungen sind deshalb ganz besonders geeignet als neuartige antiarrhythmische Wirkstoffe, insbesondere zur Behandlung und Prophylaxe von Vorhof-Arrhythmien, z.B. Vorhofflimmern (atriale Fibrillation, AF) oder Vorhofflattern (atriales Flattern).

Vorhofflimmern (AF) und Vorhofflattern sind die häufigsten anhaltenden Herzarrhythmien. Das Auftreten erhöht sich mit zunehmenden Alter und führt häufig zu fatalen Folgeerscheinungen, wie zum Beispiel Gehirnschlag. AF betrifft ca. 1 Millionen Amerikaner jährlich und führt zu mehr als 80.000 Schlaganfällen jedes Jahr in den USA. Die zur Zeit gebräuchlichen Antiarrhythmika der Klasse I und III reduzieren die Wiederauftrittsrate von AF, finden aber wegen ihrer potentiellen proarrhythmischen Nebenwirkungen nur eingeschränkte Anwendung. Deshalb besteht eine hohe medizinische Notwendigkeit für die Entwicklung besserer. Medikamente zur Behandlung atrialer Arrhythmien (S. Nattel, Am. Heart J. 130, 1995, 1094 - 1106; "Newer developments in the management of atrial fibrillation").

Es wurde gezeigt, dass den meisten supraventrikulären Arrhythmien sogenannte "Reentry" Erregungswellen unterliegen. Solche Reentries treten dann auf, wenn das Herzgewebe eine langsame Leitfähigkeit und gleichzeitig sehr kurze Refraktärperioden besitzt. Das Erhöhen der myokardialen Refraktärzeit durch Verlängerung des Aktionspotentials ist ein anerkannter Mechanismus, um Arrhythmien zu beenden bzw. deren Entstehen zu verhindern (T. J. Colatsky et al, Drug Dev. Res. 19, 1990, 129 - 140; "Potassium channels as targets for antiarrhythmic drug action"). Die Länge des Aktionspotentials wird im wesentlichen bestimmt durch das Ausmaß repolarisierender K⁺-Ströme, die über verschiedene K⁺-Kanäle aus der Zelle herausfließen. Eine besonders große Bedeutung wird hierbei dem sogenannten "delayed rectifier" I_{K} zugeschrieben, der aus 3 verschiedenen Komponenten besteht: IKᵣ, IKₛ und IKᵤᵣ.

Die meisten bekannten Klasse III- Antiarrhythmika (z.B. Dofetilide, E4031 und d-Sotalol) blockieren überwiegend oder ausschließlich den schnell aktivierenden Kaliumkanal IKᵣ, der sich sowohl in Zellen des menschlichen Ventrikel als auch im Vorhof nachweisen lässt. Es hat sich jedoch gezeigt, dass diese Verbindungen bei geringen oder normalen Herzfrequenzen ein erhöhtes proarrhythmisches Risiko aufweisen, wobei insbesondere Arrhythmien, die als "Torsades de pointes" bezeichnet werden, beobachtet wurden (D. M. Roden, Am. J. Cardiol. 72, 1993, 44B - 49B; "Current status of class III antiarrhythmic drug therapy"). Neben diesem hohen, zum Teil tödlichen Risiko bei niedriger Frequenz, wurde für die l_{Kr}-Blocker ein Nachlassen der Wirksamkeit unter den Bedingungen von Tachykardie, in der die Wirkung gerade benötigt wird, festgestellt ("negative use-dependence").

Während einige dieser Nachteile durch Blocker der langsam aktivierenden Komponente (IKₛ) möglicherweise überwunden werden können, wurde deren Wirksamkeit bisher nicht bewiesen, da keine klinischen Untersuchungen mit lK_{S}-Kanalblockem bekannt sind.

Die "besonders schnell" aktivierende und sehr langsam inaktivierende Komponente des delayed Rectifier IKᵤᵣ (=ultra-rapidly activating delayed rectifier), die dem Kv1.5-Kanal entspricht, spielt eine besonders große Rolle für die Repolarisationsdauer im menschlichen Vorhof. Eine Inhibierung des IKᵤᵣ-Kaliumauswärtsstroms stellt somit im Vergleich zur Inhibierung von lKᵣ bzw. IKₛ eine besonders effektive Methode zur Verlängerung des atrialen Aktionspotentials und damit zur Beendigung bzw. Verhinderung von atrialen Arrhythmien dar. Mathematische Modelle des menschlichen Aktionspotentials legen nahe, dass der positive Effekt einer Blockade des IKᵤᵣ gerade unter den pathologischen Bedingungen einer chronischen atrialen Fibrillation besonders ausgeprägt sein sollte (M. Courtemanche, R. J. Ramirez, S. Nattel, Cardiovascular Research 1999, 42, 477-489: "Ionic targets for drug therapy and atrial fibrillation-induced electrical remodeling: insights from a mathematical model").

Im Gegensatz zu IKᵣ und IKₛ, die auch im menschlichen Ventrikel vorkommen, spielt der IKᵤᵣ zwar eine bedeutende Rolle im menschlichen Vorhof, jedoch nicht im Ventrikel. Aus diesem Grunde ist bei Inhibierung des IKᵤᵣ-Stroms im Gegensatz zur Blockade von IKᵣ oder IKₛ das Risiko einer proarrhythmischen Wirkung auf den Ventrikel von vornherein ausgeschlossen.(Z. Wang et al, Circ. Res. 73, 1993, 1061 - 1076: "Sustained Depolarisation-Induced Outward Current in Human Atrial Myocytes"; G.-R. Li et al, Circ. Res. 78, 1996, 689 - 696: "Evidence for Two Components of Delayed Rectifier K+-Current in Human Ventricular Myocytes"; G. J. Amos et al, J. Physiol. 491, 1996, 31 - 50: "Differences between outward currents of human atrial and subepicardial ventricular myocytes").

Antiarrhythmika, die über eine selektive Blockade des IKᵤᵣ-Stroms bzw. Kv1.5-Kanals wirken, sind auf dem Markt bisher jedoch nicht verfügbar. Für zahlreiche pharmazeutische Wirkstoffe (z.B. Tedisamil, Bupivacaine oder Sertindole) wurde zwar eine blockierende Wirkung auf den Kv1.5-Kanal beschrieben, doch stellt die Kv1.5-Blockade hier jeweils nur eine Nebenwirkung neben anderen Hauptwirkungen der Substanzen dar.

In WO 98 04 521 werden Aminoindane als Kaliumkanalblocker beansprucht, die den Kv1.5-Kanal blockieren. In den Anmeldungen WO 98 18 475 und WO 98 18 476 wird die Verwendung verschiedener Pyridazinone und Phosphinoxide als Antiarrhythmika beansprucht, die über eine Blockade des IKᵤᵣ wirken sollen. Die gleichen Verbindungen wurden ursprünglich jedoch auch als Immunsuppressiva beschrieben (WO 96 25 936). Die in diesen genannten Anmeldungen beschriebenen Verbindungen sind strukturell völlig andersartig als die erfindungsgemäßen Verbindungen dieser Anmeldung.

In der Anmeldung DE19947457 werden 2'-substituierte 1,1'-Biphenyl-2-carbonamide als Kv1.5 Blocker beansprucht. In dem Patent US6150356 werden Indan- und Benzopyran-Derivate als Kv1.5 Inhibitoren beschrieben. Die in DE19947457 und US6150356 beschriebenen Verbindungen sind strukturell sehr verschieden von den erfindungsgemäßen Verbindungen der Formel 1.

Es wurde nun überraschenderweise gefunden, dass die hier beschriebenen Heteroarylsulfonyl-Anthranilsäureamide potente Blocker des humanen Kv1.5-Kanals sind. Sie können deshalb verwendet werden als neuartige Antiarrhythmika mit besonders vorteilhaftem Sicherheitsprofil. Insbesondere eignen sich die Verbindungen zur Behandlung supraventrikulärer Arrhythmien, z.B. Vorhofflimmern oder Vorhofflattern.

Die erfindungsgemäßen Verbindungen sind bisher nicht bekannt. Einige strukturell verwandte Verbindungen sind beschrieben in WO 0002851, EP 0 686 625 A1 und EP 0 947 500 A1. Für die dort beschriebenen Anthranilsäure-Derivate ist jedoch keine Kaliumkanal blockierende Aktivität bekannt.

Gemäß dem Schema 1 können erfindungsgemäße Verbindungen beispielsweise hergestellt werden, indem zunächst eine Aminocarbonsäure der Formel VI in einem Lösungsmittel wie Wasser, Pyridin oder einem Ether in Gegenwart einer Base mit einem Sulfonylchlorid der Formel R(3)-SO₂-Cl oder einem Sulfonsäureanhydrid umgesetzt wird. Als Base kommen anorganische Basen wie zum Beispiel Natriumcarbonat oder organische Basen wie zum Beispiel Pyridin oder Triethylamin in Betracht. Die erhaltene Sulfonylaminocarbonsäure der Formel VII kann dann zum Beispiel durch Umsetzung mit einem Chlorierungsagenz wie zum Beispiel Phosphorpentachlorid, Phosphoroxychlorid oder Thionylchlorid in einem inerten Lösungsmittel zu einem Säurechlorid der Formel VIII aktiviert werden und dann mit einem Amin H-R(1) zu den Titelverbindungen der Formel I umgesetzt. werden. Die Aktivierung der Carbonsäuregruppe in der Verbindung der Formel VII kann aber auch auf andere Art erfolgen, zum Beispiel durch eine der zahlreichen, dem Fachmann geläufigen Methoden, die in der Peptidchemie zur Knüpfung von Amidbindungen angewandt werden, zum Beispiel durch Überführung in ein gemischtes Anhydrid oder einen aktivierten Ester oder unter Verwendung eines Carbodiimids wie Dicyclohexylcarbodiimid.

Die Umsetzung der aktivierten Sulfonylaminocarbonsäure mit einem Amin H-R(1) wird vorteilhaft in einem inerten Lösungsmittel wie zum Beispiel Pyridin, Tetrahydrofuran oder Toluol ohne Zusatz oder unter Zusatz einer inerten Hilfsbase, zum Beispiel eines tertiären Amins oder von Pyridin, durchgeführt.

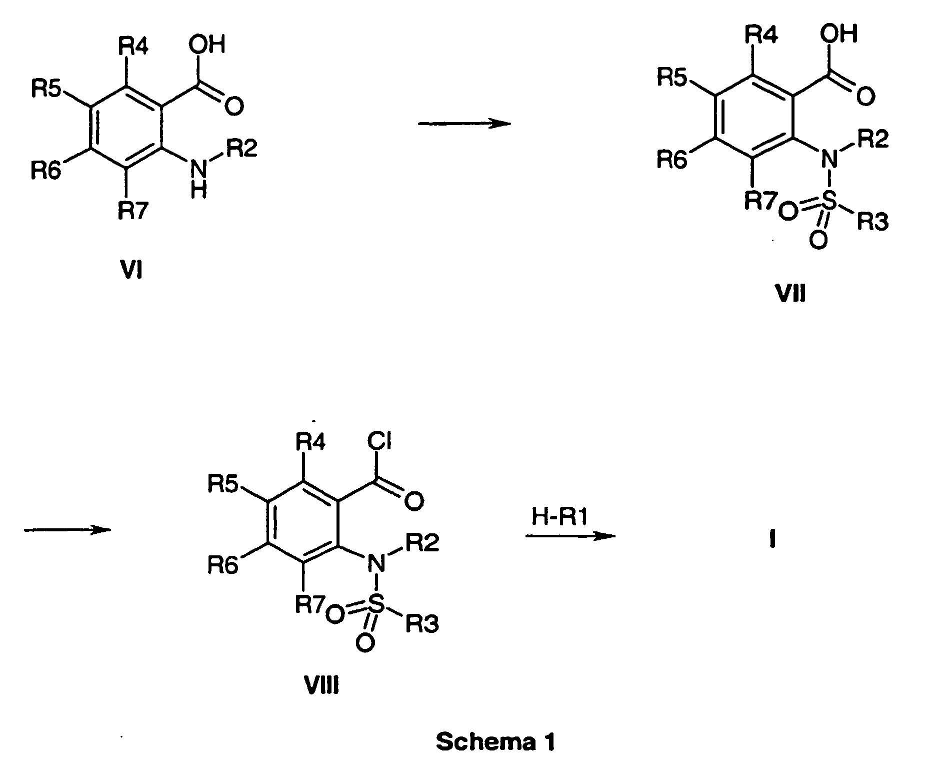

### Liste der Abkürzungen

- BuLi: Butyllithium
- CDI: Carbonyldiimidazol
- DIC: Diisopropylcarbodiimid
- DIP: Diisopropylether
- DIPEA: Diisopropylethylamin
- DMAP: 4-Dimethylaminopyridin
- DMF: N,N-Dimethylformamid
- EDAC: N-Ethyl-N'-(3-dimethylaminopropyl)-carbodiimid Hydrochlorid
- EE: Essigsäureethylester
- Fp.: Schmelzpunkt (Wenn nicht anders angegeben sind die Schmelzpunkte der ungereinigten Rohprodukte angegeben; die Schmelzpunkte der jeweiligen Reinsubstanzen können durchaus deutlich höher liegen)
- HOBT: 1-Hydroxy-1 H-benzotriazol
- LM: Lösungsmittel
- Me: Methyl
- MTB: t-Butylmethylether
- RT: Raumtemperatur
- THF: Tetrahydrofuran
- TOTU: O-[(Cyano(ethoxycarbonyl)methylen)amino]-1,1,3,3-tetramethyluronium-tetrafluoroborat

### Allgemeine Vorschrift 1: Umsetzung von Anthranilsäuren mit Sulfonylchloriden zu o-Sulfonylaminobenzoesäuren (analog Organic Syntheses 1952, 32, 8)

Zu einer Lösung von 260 g (2,4 mol) Natriumcarbonat und 1 mol der entsprechenden Anthranilsäure in 1,5 I Wasser werden bei 60°C 1,2 mol des entsprechenden Sulfonsäurechlorids portionsweise zugegeben. Die Reaktionsmischung wird bis zur vollständigen Umsetzung (ca. 1 - 6 h) bei 60 - 80°C erhitzt, wobei falls erforderlich weiteres Sulfonsäurechlorid nachgesetzt wird. Nach dem Abkühlen wird die Reaktionsmischung in 500 ml 6 molare Salzsäure gegossen und der ausgefallene Niederschlag wird abgesaugt und im Trockenschrank bei 45°C im Vakuum getrocknet. Falls das Produkt nicht kristallin anfällt wird es durch Extraktion mit Essigsäureethylester isoliert.

### Vorstufe 1 a: 4-Fluor-2-(chinolin-8-sulfonylamino)-benzoesäure

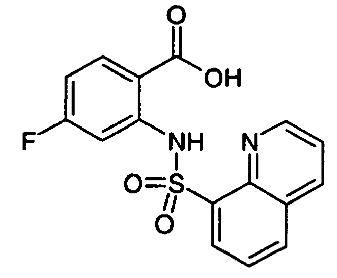

Gemäß der allgemeinen Vorschrift 1 wurden aus 5,0 g 2-Amino-4-Fluorbenzoesäure und 8,8 g 8-Chinolinsulfonylchlorid 7,6 g der Titelverbindung als weisser Feststoff erhalten.
Fp.: 248°C; MS (ES): 347 (M+1).

### Vorstufe 1 b: 6-Chlor-2-(chinolin-8-sulfonylamino)-benzoesäure

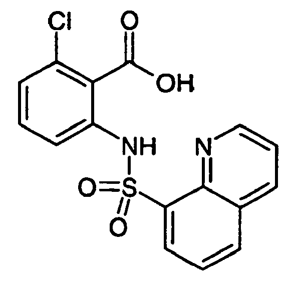

Gemäß der allgemeinen Vorschrift 1 wurden aus 5,0 g 2-Amino-6-Chlorbenzoesäure und 8,0 g 8-Chinolinsulfonylchlorid 8,3 g der Titelverbindung als Feststoff erhalten. Fp.: 88°C; MS (ES): 363 (M+1).

### Vorstufe 1 c:3-Chlor-2-(chinolin-8-sulfonylamino)-benzoesäure

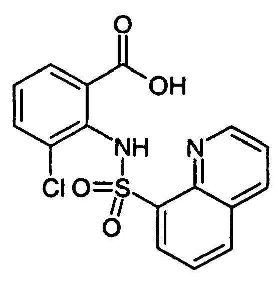

Gemäß der allgemeinen Vorschrift 1 wurden aus 5,0 g 2-Amino-6-Chlorbenzoesäure und 8,0 g 8-Chinolinsulfonylchlorid 4,1 g der Titelverbindung erhalten.
MS (ES): 363 (M+1).

Gemäss der allgemeinen Vorschrift 1 wurden unter anderem folgende weitere Vorstufen synthetisiert:

| Vorstufe | Struktur | Masse (ES) |
|---|---|---|
| 1 d | 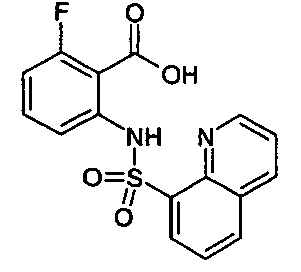 | 347 (M+1) |
| 1 e | 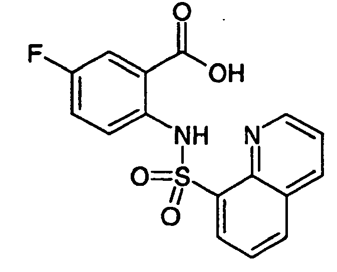 | 347 (M+1) |
| 1 f | 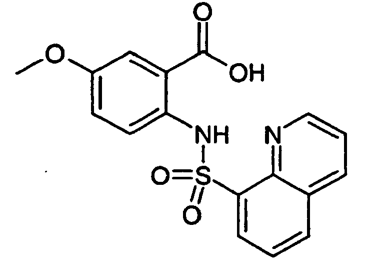 | 359 (M+1) |

### Allgemeine Vorschrift 2: Umsetzung von Sulfonylaminobenzoesäuren zu den entsprechenden Säurechloriden

### A) mit Phosphorpentachlorid

8 mmol der Sulfonylaminobenzoesäure werden in 15 ml trockenem Toluol suspendiert und bei Raumtemperatur langsam 9,6 mmol Phosphorpentachlorid eingetragen. Die Mischung wird 3h bei 50°C gerührt, auf 0°C abgekühlt, das Säurechlorid abgesaugt, mit wenig Toluol gewaschen und bei 45°C im Vakuumtrockenschrank getrocknet.

### B) mit Thionylchlorid

8 mmol der Sulfonylaminobenzoesäure werden in 6 ml Thionylchlorid 3 h auf 60°C erhitzt, eingeengt und der Rückstand zweifach mit Toluol koevaporiert.

Allgemeine Vorschrift 3 A: Darstellung sekundärer Amine durch reduktive Aminierung 0,18 mol primäres Amin werden in 200 ml Methanol gelöst, mit 0,09 mol Aldehyd, 0,18 mol Natriumcyanborhydrid sowie 0,18 mol Eisessig versetzt und 6h bei Raumtemperatur gerührt. Die Lösung wird eingeengt, mit Essigester aufgenommen und zweimal mit NaHCO₃-Lösung gewaschen. Die organische Phase wird eingeengt und der Rückstand im Hochvakuum destilliert. Bei schwer flüchtigen sekundären Aminen werden flüchtige Bestandteile abdestilliert und der Rückstand in Ether/THF gelöst und mit etherischer HCl-Lösung versetzt und das ausgefallene Hydrochlorid abgesaugt, mit Ether gewaschen und getrocknet. Die hergestellten sekundären Amine wurde ohne weitere Reinigung für die Umsetzungen mit den Sulfonylaminobenzoylchloriden oder Sulfonylaminobenzoesäuren eingesetzt.

### Vorstufe 3 a: Benzyl-(1-methyl-1 H-imidazol-2-ylmethyl)-amin

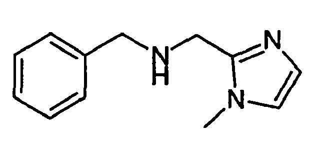

Gemäss allgemeiner Arbeitsvorschrift 3 A wurde aus 19,4 g Benzylamin und 10 g 2-Formyl-1-methylimidazol das Hydrochlorid (20,5 g) hergestellt.
MS (ES+): m/z = 202 (M+1).

### Vorstufe 3 b: Benzyl-pyridin-3-ylmethylamin

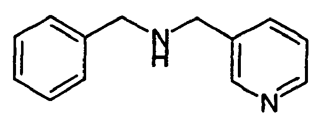

Gemäss allgemeiner Arbeitsvorschrift3 A wurde aus 4,32 g 3-Pyridylmethylamin und 2,12 g Benzaldehyd nach Kugelrohrdestillation bei 0,1 mbar und 130°C das sekundäre Amin (2,8 g) hergestellt.

MS (ES+): m/z = 199 (M+1).

### Vorstufe 3 c: Benzyl-(3-imidazol-1-yl-propyl)-amin

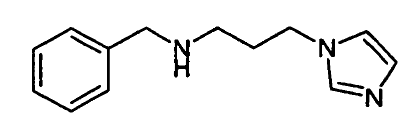

Gemäss allgemeiner Arbeitsvorschrift 3 A wurden aus 12.5 g 3-Imidazol-1-yl-propylamin und 5.3 g Benzaldehyd nach Kugelrohrdestillation bei 0.1 mbar und 130°C das sekundäre Amin (3.5 g) hergestellt.
MS (ES+): m/z = 216 (M+1).

Gemäss der allgemeinen Arbeitsvorschrift 3A wurden unter anderem folgende weitere Vorstufen hergestellt:

| Vorstufe | Struktur | Masse |
|---|---|---|
| 3d | 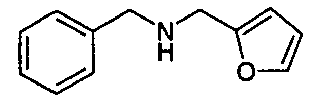 | 188 (M+1) |
| 3e | 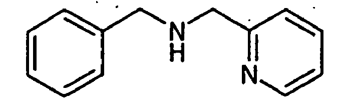 | 199 (M+1) |
| 3f | 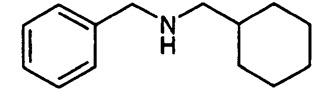 | 204 (M+1) |
| 3g | 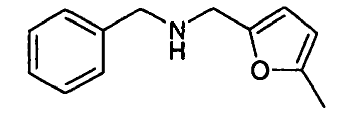 | 202 (M+1) |
| 3h | 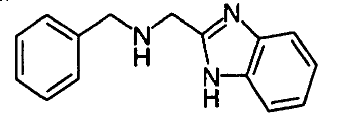 | 238 (M+1) |
| 3i | 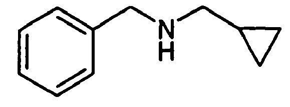 | 162 (M+1) |
| 3j | 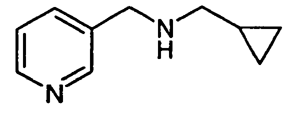 | 163 (M+1) |
| 3k | 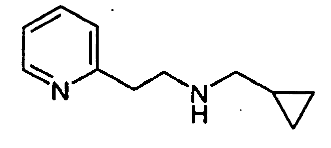 | 177 (M+1) |

### Allgemeine Vorschrift 3 B: Darstellung von α-verzweigten Aminen aus Ketonen

Zu einer Lösung von 200 mmol Hydroxylammoniumchlorid und 200 ml Natriumacetat in 120 ml Wasser wird bei 30°C eine Lösung von 67 mmol des entsprechenden Ketons in 120 ml Ethanol zugetropft und es wird bis zur vollständigen Umsetzung (1 - 3 h) auf 60°C erhitzt. Nach dem Abkühlen wird die Reaktionsmischung mit Wasser verdünnt und das ausgefallene Oxim wird abgesaugt oder falls erforderlich durch Extraktion isoliert. Das erhaltene Produkt wird in 100 ml Methanol, 100 ml THF und 10 ml konzentrierter Ammoniaklösung gelöst und in Gegenwart von Raney-Nickel bei RT und Normaldruck bis zum Ende der Wasserstoffaufnahme hydriert. Nach Abfiltrieren des Katalysators und Einengen der Reaktionsmischung erhält man das entsprechende Amin, das falls erforderlich, chromatographisch gereinigt wird.

### Vorstufe 3 l: 1-Benzyl-propylamin

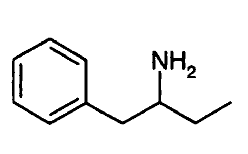

Gemäß der allgemeinen Vorschrift 3 B wurden aus 10 g 1-Phenyl-2-butanon 4,5 g der Titelverbindung erhalten.

### Vorstufe 3 m: 1-Pyridin-4-yl-propylamin

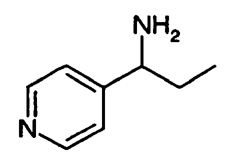

Gemäß der allgemeinen Vorschrift 3 B wurden aus 10 g 4-Propionylpyridin 10,2 g der Titelverbindung erhalten.

### Vorstufe 3 n : 1-Pyridin-3-yl-propylamin

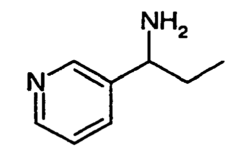

Gemäß der allgemeinen Vorschrift 3 B wurden aus 1 g 1-Pyridin-3-yl-propan-1-on 0,9 g der Titelverbindung erhalten.

### Vorstufe 3o: 1-Cyclopropyl-1-phenylmethylamin Hydrochlorid

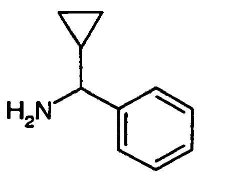

### a) N-(Cyclopropylphenylmethyl)-formamid

14,8 g (0,1 mol) Cyclopropylphenylketon, 11,4 ml (0,3 mol) Ameisensäure und 20 ml (0,5 mol) Formamid wurden 18 h auf 160°C erhitzt. Nach dem Abkühlen wurde mit 100 ml Wasser versetzt und 2x mit je 50 ml Ether extrahiert. Die etherische Phase wurde mit 50 ml 10 % Na₂CO₃ - Lösung gewaschen, über Na₂SO₄ getrocknet und eingeengt. Man erhielt 13,6 g (77,4 mmol) eines gelben Öls.

### b) 1-Cyclopropyl-1-phenylmethylamin Hydrochlorid

13,6 g (77,4 mmol) N-(Cyclopropylphenylmethyl)-formamid (siehe a) wurden 18 h in 100 ml 2N HCl zum Rückfluss erhitzt. Nach dem Abkühlen wurde 2x mit je 50 ml Dichlormethan extrahiert und die wässrige Phase eingeengt. Der Rückstand wurde in 30 ml 2-Propanol aufgenommen, zum Sieden erhitzt und über Nacht im Kühlschrank abgekühlt. Die ausgefallenen Kristalle an 1-Cyclopropyl-1-phenylmethylamin Hydrochlorid (3,85 g, 21 mmol) wurden abgesaugt und im Vakuumtrockenschrank getrocknet.

### Vorstufe 3p: Cyclopropylpyridin-2-yl-methylamin Hydrochlorid

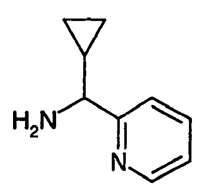

### a) Cyclopropylpyridin-2-yl-methylenamin

Zu 100 ml (160 mmol) n-BuLi-Lösung in 300 ml Diethylether wurden bei -70°C 25 g (157,5 mmol) 2-Brompyridin in 100 ml Diethylether innerhalb von 20 min zugetropft. Die dunkelrote Lösung wurde 5 h gerührt und dann mit 8,8 g (131 mmol) Cyclopropancarbonsäurenitril in 100 ml Ether versetzt. Die Mischung wurde bei -70°C 30 min gerührt, auf Raumtemperatur erwärmt und weitere 30 min gerührt. Anschliessend wurden 15 g Na₂SO₄ x 10 H₂O zugesetzt und 1 h weitergerührt. Die rote Lösung wurde mit Na₂SO₄ versetzt, abfiltriert und eingeengt. In der Kugelrohrdestille wurde das Produkt bei 75°C-120°C /0.3 mbar als hellgelbes Öl (18,6 g, 127 mmol) destilliert und bei -18°C aufbewahrt.

### b) Cyclopropylpyridin-2-yl-methylamin Hydrochlorid

2,72 g (18,6 mmol) Cyclopropylpyridin-2-yl-methylenamin (siehe a) wurden in 35 ml trockenem Methanol gelöst. Bei 0°C wurde portionsweise 0,69 g (18,6 mmol) NaBH₄ zugesetzt. Nach 30 min bei 0°C wurde 2 h bei Raumtemperatur gerührt, mit 1M HCl auf pH 3 gestellt, das Methanol am Rotationsverdampfer abgezogen und der Rückstand gefriergetrocknet. Man erhielt 8,8 g Cyclopropylpyridin-2-yl-methylamin Hydrochlorid das mit anorganischen Salzen und Borsäure vermischt ist.

### Vorstufe 3 q: Cyclopropylpyridin-3-yl-methylamin Hydrochlorid

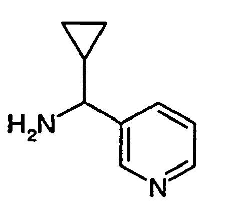

### a) Cyclopropylpyridin-3-yl-methylenamin

Entsprechend der Vorschrift für Vorstufe 3p wurden aus 8,8 g (131 mmol) Cyclopropancarbonsäurenitril, 25 g (157,5 mmol) 3-Brompyridin und 173 mmol n-BuLi-Lösung nach Kugelrohrdestillation (130°C/0,2 mbar) 7,5 g (51 mmol) des Imins als gelbes Öl isoliert.

### b) Cyclopropylpyridin-3-yl-methylamin Hydrochlorid

Entsprechend der Vorschrift für Vorstufe 3p wurden aus 7,5 g (51,5 mmol) Imin (siehe a) und 1,9 g (51,4 mmol) NaBH₄ 16,6 g Cyclopropylpyridin-3-yl-methylamin Hydrochlorid erhalten die mit anorganischen Salzen und Borsäure vermischt ist.

### Vorstufe 3 r: 1-(5-Methyl-furan-2-yl)-propylamin

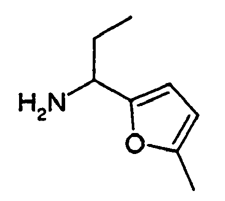

Zu 5 g (36 mmol) 2-Methyl-5-propionylfuran und 28,2 g (366 mmol) Ammoniumacetat in 300 ml Methanol wurde portionsweise 11,35 g (180 mmol) Natriumcyanborhydrid unter Rühren eingetragen und 18 h bei RT reagieren gelassen. Die Mischung wurde weitgehend eingeengt, mit 200 ml Dichloromethan versetzt und die organische Phase 3x mit je 50 ml NaHCO₃-Lösung gewaschen, über Na₂SO₄ getrocknet und eingeengt. Man erhielt 3,9 g (28 mmol) des Amins in Form eines hellgelben Öls.

### Vorstufe 3s: 1-Phenyl-prop-2-ynylamin Hydrochlorid

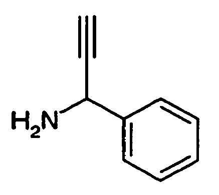

Die Verbindung wurde entsprechend der Vorschrift von Bjorn M. Nilsson et al J. Heterocycl. Chem. (1989), 26(2), 269-75 ausgehend von 1-Phenyl-2-propynytalkohol durch Ritter-Reaktion und anschliessende salzsaure Hydrolyse hergestellt.

### Vorstufe 3t: C-Cyclopropyl-C-(6-methoxy-pyridin-2-yl)-methylamin

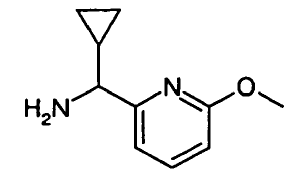

### a) Cyclopropancarbaldehyd- O-benzyloxim

6,7 g (95,6 mmol) Cyclopropancarbaldehyd wurde zusammen mit 15,3 g (95,6 mmol) O-Benzylhydroxylamin und 15,7 g (191,2 mmol) Natriumacetat in 250 ml Ethanol bei Raumtemperatur 18h gerührt, eingeengt und mit Na₂SO₄ versetzt. Der Rückstand wurde 3x mit je 50 ml Dichloromethan extrahiert, die organische Phase eingeengt und das Rohprodukt an Kieselgel chromatographisch gereinigt. Man erhielt 5 g (28,6 mmol) einer farblosen Flüssigkeit.

### b) O-Benzyl-N-[cyclopropyl-(6-methoxypyridin-2-yl)-methyl]-hydroxylamin

3,76 g (20 mmol) 2-Bromo-6-methoxypyridin wurden in 20 ml THF bei -78°C mit 8,8 ml (22 mmol) n-BuLi (2,5 M in Toluol) versetzt. Nach 30 min wurde diese dunkelrote Lösung zu einer Lösung von 1,4 g (8 mmol) Cyclopropancarbaldehyd- O-benzyloxim (siehe a) und 2,52 ml (20 mmol) BF₃-Etherat in 40 ml Toluol, die bei -78°C 15 min gerührt wurde, zugegeben. Es wurde 4 h bei -78°C gerührt, langsam auf RT erwärmt, mit Wasser versetzt und dann mit gesättigter Na₂CO₃-Lösung alkalisch gestellt. Die organische Phase wurde abgetrennt, die wässrige Phase mit Toluol extrahiert und die vereinigten organischen Phasen über Na₂SO₄ getrocknet und eingeengt. Das Rohprodukt wurde in 12 ml Acetonitril aufgenommen, unlösliche Bestandteile abgetrennt und das Produkt mittels präparativer HPLC isoliert (650 mg, rotes Öl).

### c) C-Cyclopropyl-C-(6-methoxy-pyridin-2-yl)-methylamin

650 mg (2,3 mmol) O-Benzyl-N-[cyclopropyl-(6-methoxypyridin-2-yl)-methyl]-hydroxylamin (siehe a) wurden in 18 ml Eisessig gelöst und mit 18 ml Wasser verdünnt. 3,3 g Zinkstaub wurden dazugegeben und die Suspension 24 h im Ultraschallbad zur Reaktion gebracht Die Mischung wurde über Kieselgur filtriert, mit halbkonzentrierter Essigsäure nachgewaschen , das Filtrat teilweise eingeengt und mit gesättigter Na₂CO₃-Lösung auf pH 11 gebracht. Es wurde 3x mit je 100 ml Dichloromethan extrahiert, über Na₂SO₄ getrocknet und eingeengt. Man erhielt 0,4 g (2,2 mmol) des Produkts in Form eines dunkelroten Öls.

### Allgemeine Vorschrift 4 A: Darstellung von 2-Aminobenzoesäureamiden aus 2-Nitrobenzoesäuren

Die entsprechende 2-Nitrobenzoesäure wird zunächst analog der allgemeinen Vorschriften 2 und 5 mit dem jeweiligen Amin zu einem 2-Nitrobenzoesäureamid umgesetzt. Anschließend werden 4 mmol des 2-Nitrobenzoesäureamids in 50 ml THF und 50 ml Methanol in Gegenwart einer Spatelspitze 10 % Palladium auf Kohle bei RT und Normaldruck hydriert. Man saugt vom Katalysator ab, engt die Reaktionsmischung ein und erhält das entsprechende 2-Aminobenzoesäureamid.

Auf diese Weise wurde u.a. folgende Vorstufe synthetisiert:

| Vorstufe | Struktur | Masse |
|---|---|---|
| 4a | 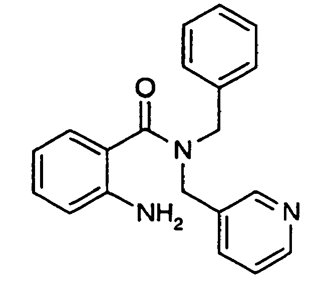 | 318 (M+1) |

Allgemeine Vorschrift 4 B: Darstellung von 2-Aminobenzoesäureamiden aus lsatosäureanhydrid

Eine Lösung von 20 mmol Isatosäureanhydrid und 22 mmol des entsprechenden Amins in 75 ml DMF wird bis zur vollständigen Umsetzung auf 60°C erhitzt. Die Reaktionsmischung wird mit 100 ml Wasser versetzt und das Produkt wird abgesaugt oder durch Extraktion isoliert.

### Vorstufe 4 b: (S)-2-Amino-N-(1-phenyl-propyl)-benzamid

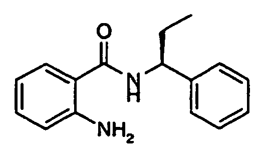

Gemäß der allgemeinen Vorschrift 4 B wurden aus 3 g (S)-1-Phenylpropylamin und 3,2 g Isatoanhydrid nach 2 h bei 60°C 3,4 g der Titelverbindung erhalten.

### Allgemeine Vorschrift 5: Umsetzung von Sulfonylaminobenzoylchloriden mit Aminen

Zu einer Lösung von 0,66 mmol des jeweiligen Amins und 0,9 mmol Triethylamin in 3 ml Methylenchlorid werden 0,6 mmol des jeweiligen Sulfonylaminobenzoylchlorids hinzugefügt und der Ansatz wird über Nacht bei Raumtemperatur gerührt. Die Reaktionsmischung wird mit 5 ml Wasser und 10 ml Methylenchlorid verdünnt und die organische Phase wird nacheinander mit 1 M Salzsäurelösung und gesättigter Natriumbicarbonatlösung gewaschen. Nach Trocknen über Magnesiumsulfat wird die Lösung im Vakuum eingeengt und das Produkt, falls erforderlich, über präparative HPLC oder Säulenchromatographie gereinigt.

### Allgemeine Vorschrift 6: Umsetzung von Sulfonylaminobenzoesäuren mit Aminen

Zu einer Lösung von 0,42 mmol der entsprechenden Sulfonylaminobenzoesäure, 0,44 mmol HOBT und 0,44 mmol EDAC in 5 ml THF werden bei 0°C 0,44 mmol des jeweiligen Amins zugetropft und es wird 4 bis 12 h bei RT gerührt. Die Reaktionsmischung wird mit EE verdünnt und mit verdünnter Salzsäure und Natriumbicarbonatlösung gewaschen. Nach Trocknen über Magnesiumsulfat und Einengen im Vakuum erhält man das entsprechende Amid, das falls erforderlich über präparative HPLC gereinigt wird.

### Allgemeine Vorschrift 7: Umsetzung von 2-Aminobenzoesäureamiden mit Sulfonylchloriden

Zu einer Lösung von 0,2 mmol des entsprechenden 2-Aminobenzoesäureamids (Vorstufe 4) und 0,6 mmol Pyridin in 5 ml Methylenchlorid wird eine Lösung von 0,3 mmol des entsprechenden Sulfonylchlorids in 2 ml Methylenchlorid bei 0°C zugetropft, und es wird über Nacht bei RT gerührt. Die organische Phase wird mit Wasser, verdünnter Salzsäure und Natriumbicarbonatlösung gewaschen und das erhaltene Rohprodukt wird, falls erforderlich, über präparative HPLC gereinigt.

### Beispiel 1: (S)-N-(1-Phenyl-propyl)-2-(chinolin-8-sulfonylamino)-benzamid

### a) 2-(Chinolin-8-sulfonylamino)-benzoesäure

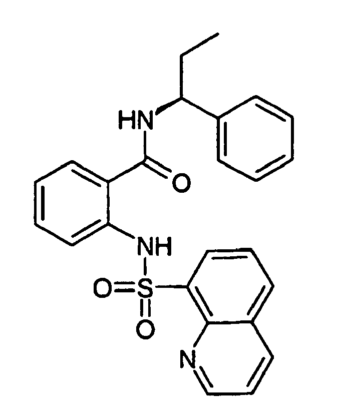

Zu einer Lösung aus 1.32 g Na₂CO₃ in 10 ml Wasser wurden bei 60°C 690 mg Anthranilsäure portionsweise zugegeben. 10 Minuten wurde bei dieser Temperatur gerührt, anschließend bei 70°C portionsweise 1.25 g 8-Chinolinsulfonylchlorid zugegeben. 5 Stunden wurde bei 70°C gerührt, anschließend weitere 230 mg 8-Chinolinsulfonylchlorid zugegeben. 2 Stunden wurde bei 70°C gerührt, anschließend ließ man das Reaktionsgemisch auf RT abkühlen. Mit einer 2N wässrigen HCl-Lösung wurde auf pH 1 gestellt und die Suspension eine weitere Stunde bei RT gerührt. Dann wurde der Niederschlag abfiltriert, unter Feinvakuum bei 60°C getrocknet und man erhielt 1.57 g eines farblosen, amorphen Feststoffs.
MS (ESI): 329 (M+H)⁺

### b) 2-(Chinolin-8-sulfonylamino)-benzoylchlorid

100 mg 2-(Chinolin-8-sulfonylamino)-benzoesäure wurden in 1 ml SOCl₂ gelöst und 4½ Stunden unter Rückfluss gekocht. Die flüchtigen Bestandteile wurden anschließend im Vakuum entfernt, der Rückstand in 10 ml Toluol aufgenommen und anschließend erneut die flüchtigen Bestandteile im Vakuum entfernt. Man erhielt 120 mg des Säurechlorids, das ohne Reinigung weiter umgesetzt wurde.

### c) (S)-N-(1-Phenyl-propyl)-2-(chinolin-8-sulfonylamino)-benzamid

120 mg 2-(Chinolin-8-sulfonylamino)-benzoylchlorid wurden in 4 ml CH₂Cl₂ suspendiert und bei RT 85 µl Triethylamin zugegeben. Anschließend wurde eine Lösung von 41 mg (S)-1-Phenylpropylamin in 2 ml CH₂Cl₂ zugegeben und 18 Stunden bei RT gerührt. Das Reaktionsgemisch wurde mit 50 ml CH₂Cl₂ verdünnt und 2 mal mit je 20 ml einer gesättigten wäßrigen Na₂CO₃-Lösung gewaschen. Die wässrige Phase wurde anschließend mit weiteren 20 ml CH₂Cl₂ extrahiert, die vereinigten organischen Phasen über Na₂SO₄ getrocknet und das Solvens im Vakuum entfernt. Chromatographie des Rückstandes an Kieselgel mit MTB/DIP 1:1 lieferte 77 mg eines amorphen Feststoffs.
R_{f} (MTB/DIP 1:1) = 0.31 MS (ES): 446 (M+H)⁺

Die Titelverbindungen der Beispiele 2-11 wurden analog Beispiel 1 synthetisiert:

### Beispiel 2: (R)-N-(1-Phenyl-propyl)-2-(chinolin-8-sulfonylamino)-benzamid

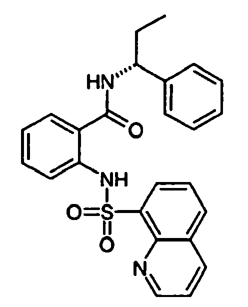

R_{f} (MTB/DIP 1:1) = 0.31 MS (ES): 446 (M+H)⁺

### Beispiel 3: (R)-N-(1-Phenyl-ethyl)-2-(chinolin-8-sulfonylamino)-benzamid

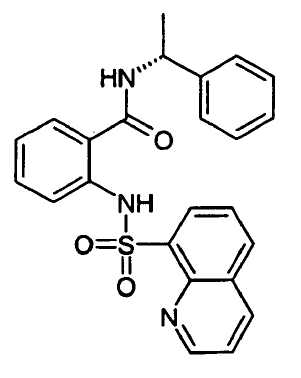

R_{f} (MTB/DIP 1:1) = 0.25 MS (ES) : 432 (M+H)⁺

### Beispiel 4: (S)-N-(1 -Phenyl-ethyl)-2-(chinolin-8-sulfonylamino)-benzamid

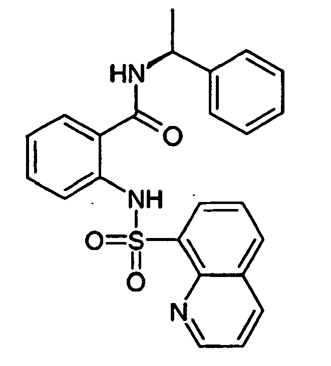

R_{f} (MTB/DIP 1:1) = 0.25 MS (ES) : 432 (M+H)⁺

### Beispiel 5: (S)-N-[1-(4-Chlor-phenyl)-ethyl]-2-(chinolin-8-sulfonylamino)-benzamid

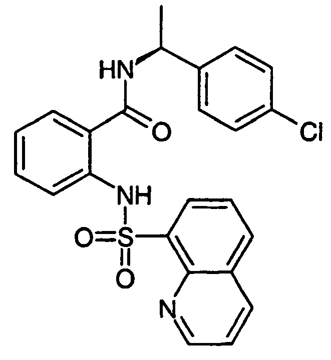

R_{f} (MTB/DIP 1:1)= 0.23 MS (ES): 466 (M+H)⁺

### Beispiel 6: (R)-N-[1-(4-Chlor-phenyl)-ethyl]-2-(chinolin-8-sulfonylamino)-benzamid

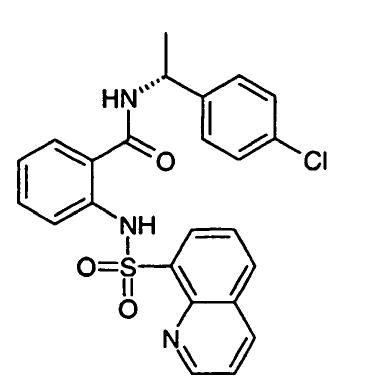

R_{f} (MTB/DIP 1:1) 0.23 MS (ES): 466 (M+H)⁺

### Beispiel 7: 4-Chlor-N-pyrazin-2-ytmethyl-2-(Chinolin-8-sulfonylamino)-benzamid

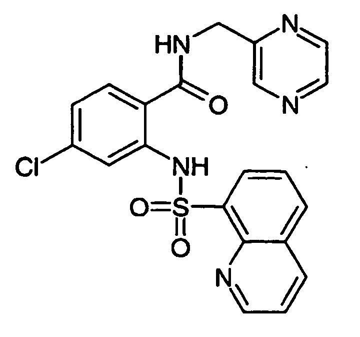

R_{f} (EE)= 0.10 MS (ES): 454 (M+H)⁺

### Beispiel 8: N-(2-Benzyloxy-ethyl)-5-fluoro-2-(Chinolin-8-sulfonyfamino)-benzamid ,

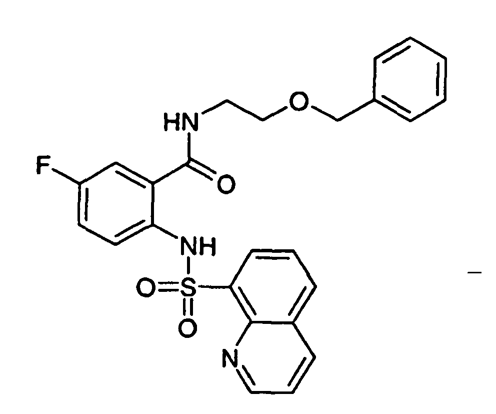

R_{f} (MTB/DIP 1:1) = 0.24 MS (ES) : 480 (M+H)⁺

### Beispiel 9: N-(2-Benzyloxy-ethyl)-2-(chinolin-8-sulfonylamino)-benzamid

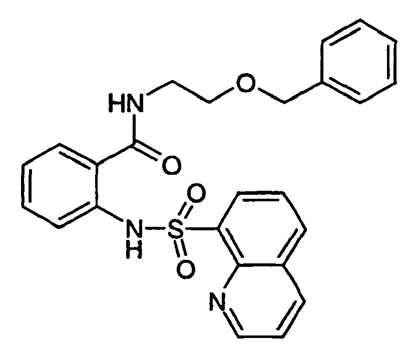

R_{f} (MTB) = 0.36 MS (ES) : 462 (M+H)⁺

### Beispiel 10: N-(2-Benzyloxy-ethyl)-5-methoxy-2-(chinolin-8-sulfonylamino)-benzamid

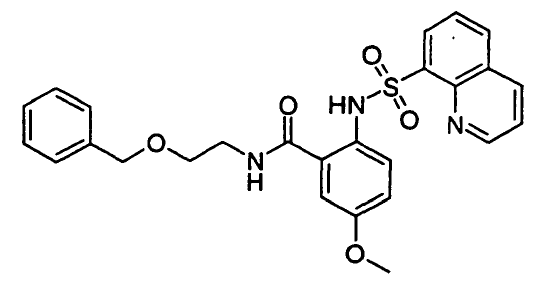

MS (ES) : 492 (M+H)⁺

### Beispiel 11: 5-Fluor-N-(2-phenoxy-ethyl)-2-(Chinolin-8-sulfonylamino)-benzamide

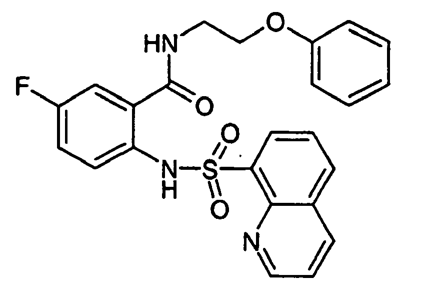

R_{f} (MTB/DIP 1:1) = 0.29 MS (ES) : 466 (M+H)⁺

### Beispiel 12: N-Benzyl-5-methoxy-N-(1-methyl-1 H-imidazol-2-ylmethyl)-2-(chinolin-8-sulfonylamino)-benzamid

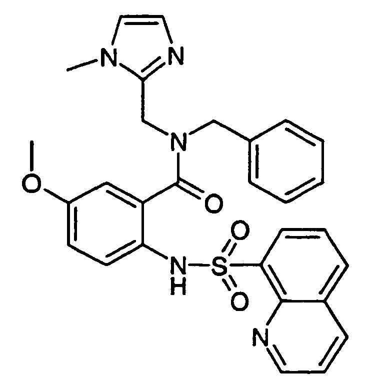

### a) Benzyl-(1 -methyl-1 H-imidazol-2-ylmethyl)-amin

19.4 g (0.18 mol) Benzylamin wurden in 200 ml Methanol gelöst, mit 10 g (0.09 mol) 2-Formyl-1-methylimidazol , 11.4 g Natriumcyanborhydrid (0.18 mol) sowie 10.9 g (0.18 mol) Eisessig versetzt und 16 h bei RT gerührt. Die Lösung wurde eingeengt, mit EE aufgenommen und zweimal mit NaHCO₃-Lösung gewaschen. Die organische Phase wurde getrocknet, eingeengt und noch vorhandenes Benzylamin im Feinvakuum abdestilliert. Der Rückstand wurde in Diethylether/THF 1:1 gelöst und mit einer gesättigten Lösung von HCl in Diethylether versetzt. Das ausgefallene Hydrochlorid (20.5 g) wurde abgesaugt, mit Diethylether gewaschen und im Vakuum getrocknet.
MS (ES) : 202 (M+H)⁺

### b) N-Benryl-5-methoxy-N-(1-methyl-1H-imidazol-2-ylmethyl)-2-(chinolin-8-sulfonylamino)-benzamid

66 mg Benzyl-(1-methyl-1 H-imidazal-2-ylmethyl)-amin wurden wie unter 1c) beschrieben umgesetzt und man erhielt 78 mg der Titelverbindung als amorphen Feststoff.
R_{f} (EE) = 0.09 MS (ES) : 542 (M+H)⁺

### Beispiel 13: N-(Phenyl-pyridin-3-yl-methyl)-2-(chinolin-8-sulfonylamino)-benzamid

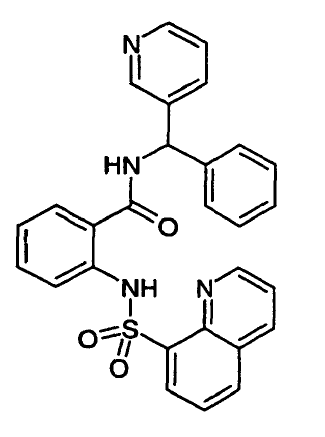

Analog Beispiel 1 wurden 120 mg Phenyl-pyridin-3-yl-methylamin (Synthesis 1976, 593) mit 450 mg 2-(Chinolin-8-sulfonylamino)-benzoylchlorid umgesetzt und man erhielt 130 mg eines amorphen Feststoffs.
R_{f} (EE) = 0.29 MS (ES) : 495 (M+H)⁺

### Beispiel 14: N-Benzyl-N-pyridin-3-ylmethyl-2-(chinolin-8-sulfonylamino)-benzamid

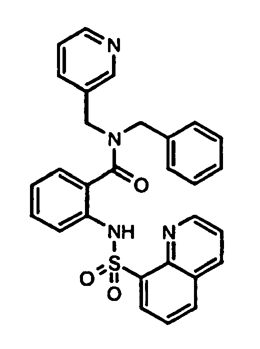

Analog Beispiel 1 wurden 99 mg N-Benzyl-N-(3-pyridylmethyl)amin (Vorstufe 3b) mit 87 mg 2-(Chinolin-8-sulfonylamino)-benzoylchlorid umgesetzt und man erhielt 66 mg eines amorphen weißen Feststoffs.
MS (ES) : 509 (M+H)⁺

### Beispiel 15: N-Cyclohexyl-2-(chinolin-8-sulfonylamino)-benzamid

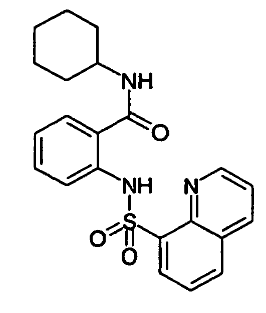

Analog Beispiel 1 wurden 50 mg Cyclohexylamin mit 87 mg 2-(Chinolin-8-sulfonylamino)-benzoylchlorid umgesetzt und man erhielt 59 mg eines amorphen weißen Feststoffs.
MS (ES) : 410 (M+H)⁺

Die Titelverbindungen der Beispiele 16-44 wurden analog Beispiel 1 synthetisiert:

### Beispiel 16: N-(1-Benzyl-propyl)-2-(chinolin-8-sulfonylamino)-benzamid

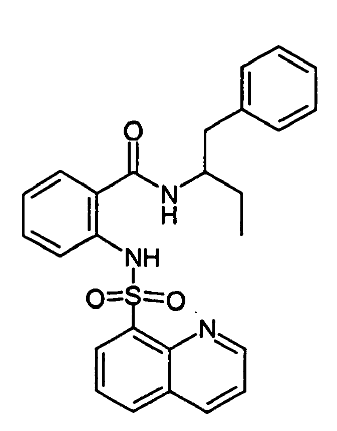

Die Titelverbindung wurde erhalten aus 2-(Chinolin-8-sulfonylamino)-benzoylchlorid (Beispiel 1 b) und 1-Benrylpropylamin (Vorstufe 31).
MS (ES) : 460 (M+H)⁺

### Beispiel 17: (S)-5-Chlor-2-(5-chlor-thiophen-2-sulfonylamino)-N-(1-phenyl-propyl)-benzamid

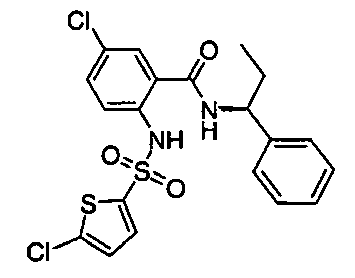

MS (ES) : 469 (M+H)⁺

### Beispiel 18: N-(1-Pyridin-3-yl-propyl)-2-(chinolin-8-sulfonylamino)-benzamid

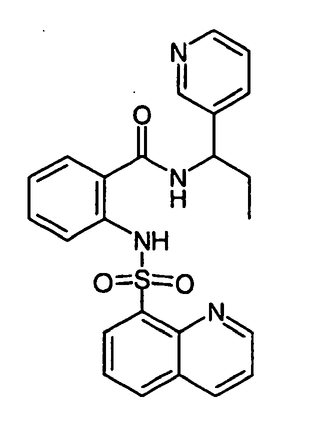

Die Titelverbindung wurde erhalten aus 2-(Chinolin-8-sulfonylamino)-benzoylchlorid (Beispiel 1 b) und 1-Pyridin-3-yl-propylamin (Vorstufe 3n).
MS (ES) : 447 (M+H)⁺

### Beispiel 19: N-Benzyl-N-(1-methyl-1 H-imidazol-2-ylmethyl)-2-(chinolin-8-sulfonylamino)-benzamid

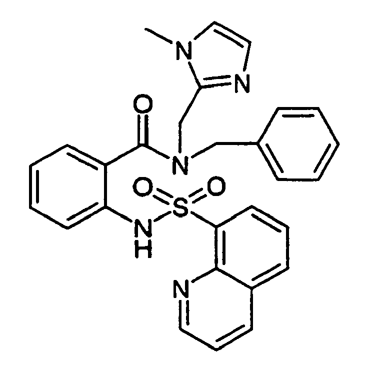

MS (ES) : 512 (M+H)⁺

### Beispiel 20: N-Benzyl-N-furan-2-ylmethyl-2-(chinolin-8-sulfonylamino)-benzamid

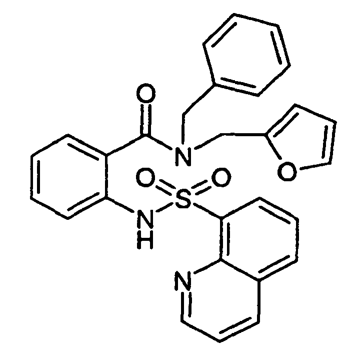

MS (ES) : 498 (M+H)⁺

### Beispiel 21: N-Cyclopropyl-N-pyridin-3-ylmethyl-2-(chinolin-8-sulfonylamino)-benzamid

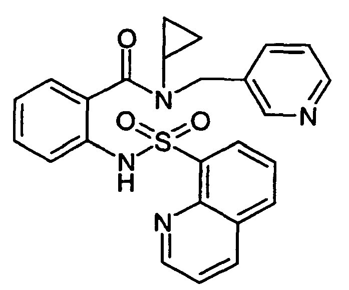

MS (ES) : 459 (M+H)⁺

### Beispiel 22: N-Benzyl-N-cyclopropyl-2-(chinolin-8-sulfonylamino)-benzamid

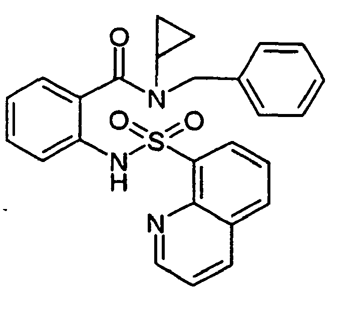

MS (ES) : 458 (M+H)⁺

### Beispiel 23: N-Benzyl-N-pyridin-2-ylmethyl-2-(chinolin-8-sulfonylamino)-benzamid

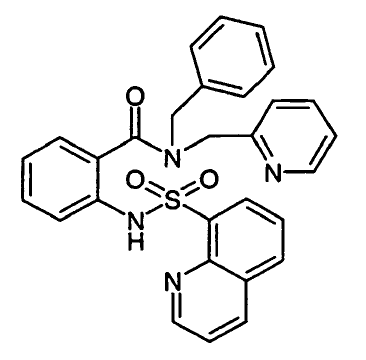

MS (ES) : 509 (M+H)⁺

### Beispiel 24: (R)- 2-(Chinolin-8-sulfonylamino)-N-(1-p-tolyl-ethyl)-benzamid

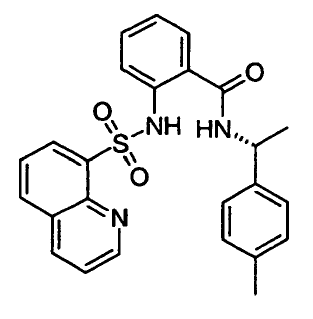

MS (ES) : 446 (M+H)⁺

### Beispiel 25: N-[1-(4-Fluor-phenyl)-ethyl]-2-(chinolin-8-sulfonylamino)-benzamid

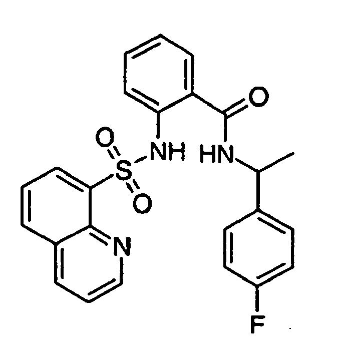

MS (ES) : 450 (M+H)⁺

### Beispiel 26: (R)- N-[1-(4-Methoxy-phenyl)-ethyl]-2-(chinolin-8-sulfonylamino)-benzamid

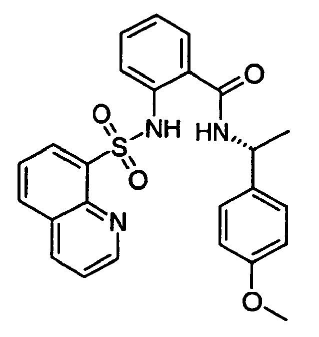

MS (ES) : 462 (M+H)⁺

### Beispiel 27: N-(1-Methyl-1-phenyl-ethyl)-2-(chinolin-8-sulfonylamino)-benzamid

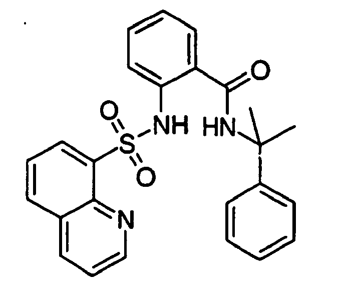

MS (ES) : 446 (M+H)⁺

### Beispiel 28: N-Indan-1-yl-2-(chinolin-8-sulfonylamino)-benzamid

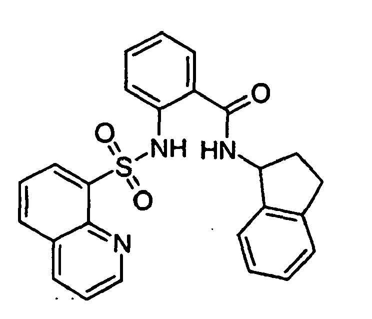

MS (ES) : 444 (M+H)⁺

### Beispiel 29: Chinolin-8-sulfonsäure [2-(3,4-dihydro-1 H-isoquinoline-2-carbonyl)-phenyl]-amid

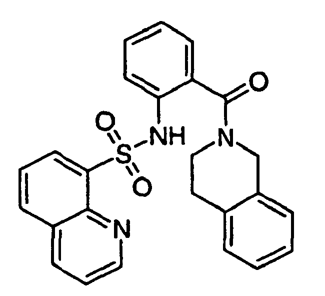

MS (ES) : 444 (M+H)⁺

### Beispiel 30: N-[2-(4-Fluor-phenyl)-1,1-dimethyl-ethyl]-2-(chinolin-8-sulfonylamino)-benzamid

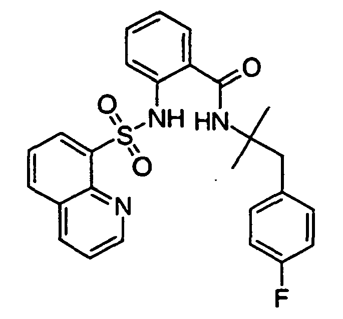

MS (ES) : 478 (M+H)⁺

### Beispiel 31: N-(1-Phenyl-butyl)-2-(chinolin-8-sulfonylamino)-benzamid

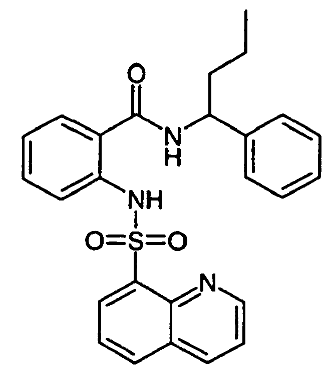

MS (ES) : 460 (M+H)⁺

### Beispiel 32: (S)- 2-(Chinolin-8-sulfonylamino)-N-(1-p-tolyl-ethyl)-benzamid

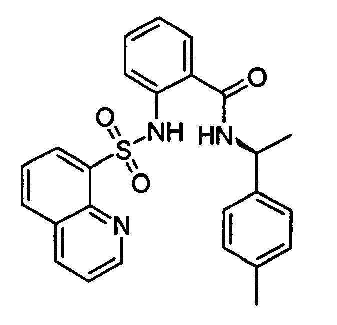

MS (ES) : 446 (M+H)⁺

### Beispiel 33 (S)- N-[1-(4-Methoxy-phenyl)-ethyl]-2-(chinolin-8-sulfonylamino)-benzamid

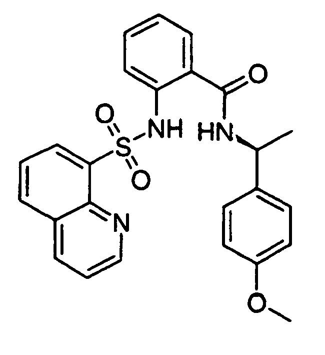

MS (ES) : 462 (M+H)⁺

### Beispiel 34: (S)-N-[1-(3-Methoxy-phenyl)-ethyl]-2-(chinolin-8-sulfonylamino)-benzamid

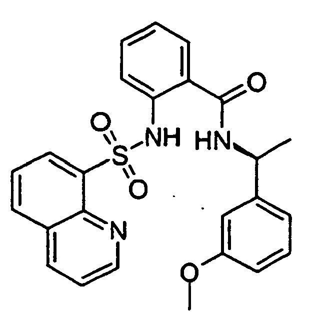

MS (ES) : 462 (M+H)⁺

### Beispiel 35 (R)-N-(2-Hydroxy-1-phenyl-ethyl)-2-(chinolin-8-sulfonylamino)-benzamid

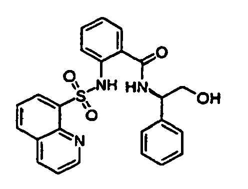

MS (ES) : 448 (M+H)⁺

### Beispiel 36: (S)- N-(1-Cyclohexyl-ethyl)-2-(chinolin-8-sulfonylamino)-benzamid

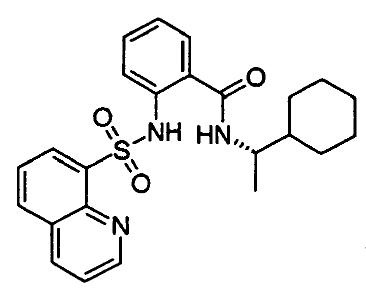

MS (ES) : 438 (M+H)⁺

### Beispiel 37: N-(2-Phenyl-cyclopropyl)-2-(chinolin-8-sulfonylamino)-benzamid

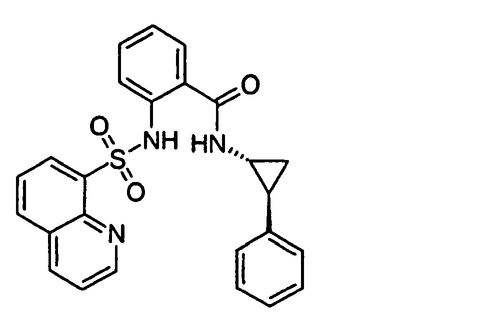

MS (ES) : 444 (M+H)⁺

### Beispiel 38: N-[1-(2-Fluor-phenyl)-propyl]-2-(chinolin-8-sulfonylamino)-benzamid

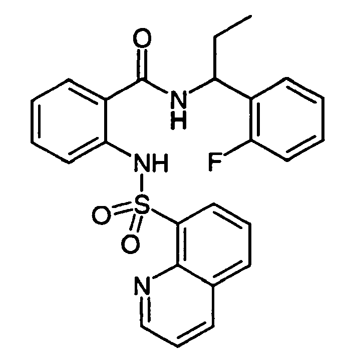

MS (ES) : 464 (M+H)⁺

### Beispiel 39: N-(2-Methoxy-1-phenyl-ethyl)-2-(chinolin-8-sulfonylamino)-benzamid

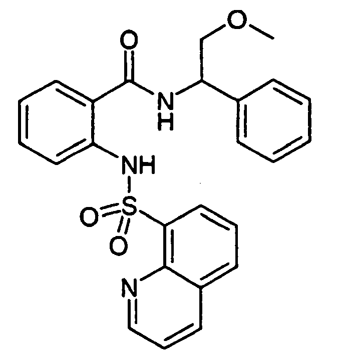

MS (ES) : 462 (M+H)⁺

### Beispiel 40: N-[1-(4-Chloro-phenyl)-propyl]-2-(chinolin-8-sulfonylamino)-benzamid

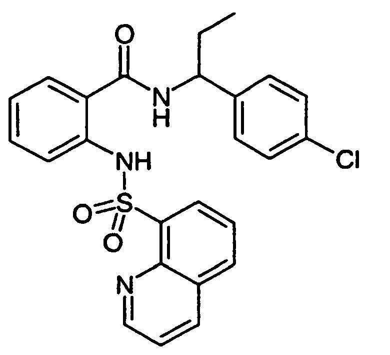

MS (ES) : 480 (M+H)⁺

### Beispiel 41: N-Cyclopropyl-N-phenyl-2-(chinolin-8-sulfonylamino)-benzamid

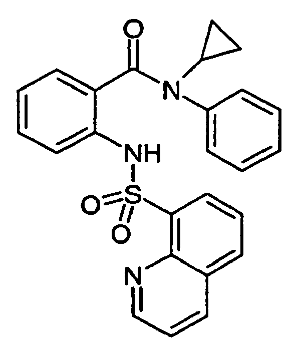

MS (ES) : 444 (M+H)⁺

### Beispiel 42: N-(2-Isopropyl-5-methyt-cyclohexyl)-2-(chinolin-8-sulfonylamino)-benzamid

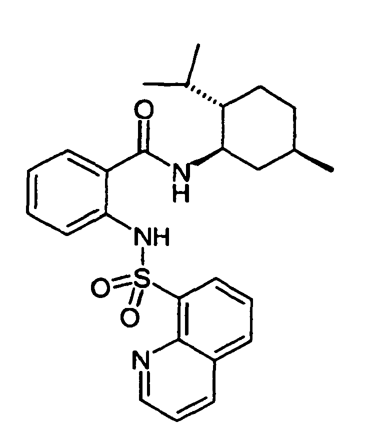

MS (ES) : 466 (M+H)⁺

### Beispiel 43: N-(Cyclopropyl-phenyl-methyl)-2-(chinolin-8-sulfonylamino)-benzamid

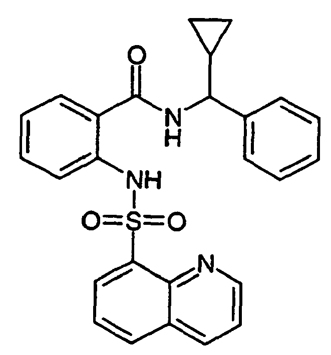

MS (ES) : 458 (M+H)⁺

### Beispiel 44: N-[1-(4-Fluoro-phenyl)-propyl]-2-(chinolin-8-sulfonylamino)-benzamid

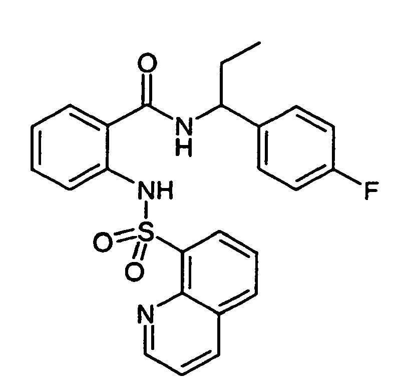

MS (ES) : 464 (M+H)⁺

Die Titelverbindungen der Beispiele 45 - 51 wurden aus (S)-2-Amino-N-(1-phenylpropyl)-benzamid (Vorstufe 4b) nach der allgemeinen Vorschrift 7 hergestellt:

### Beispiel 45: (S)-N-(1-Phenyl-propyl)-2-(thiophen-2-sulfonylamino)-benzamid

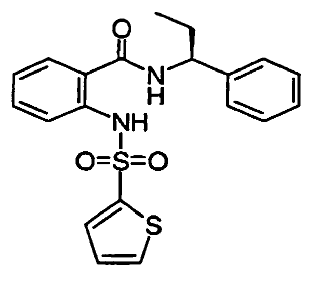

MS (ES) : 401 (M+H)⁺

### Beispiel 46: 2-(3,5-Dimethyl-isoxazol-4-sulfonylamino)-N-(1-phenyl-propyl)-benzamid

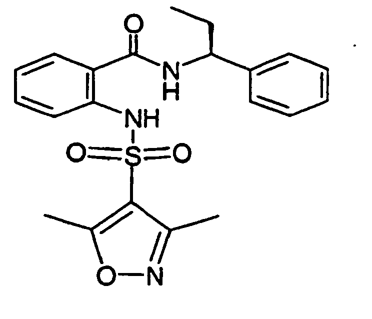

MS (ES) : 414 (M+H)⁺

### Beispiel 47: (S)-2-(Isochinolin-5-sulfonylamino)-N-(1-phenyl-propyl)-benzamid

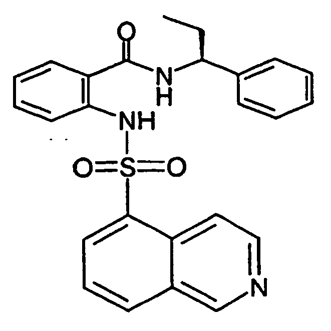

MS (ES) : 446 (M+H)⁺

### Beispiel 48: 2-(Benzo[1,2,5]oxadiazol-4-sulfonylamino)-N-(1-phenyl-propyl)-benzamid

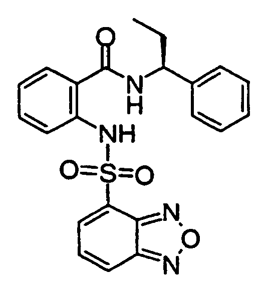

MS (ES) : 437 (M+H)⁺

### Beispiel 49: 2-(5-Chlor-thiophen-2-sulfonylamino)-N-(1-phenyl-propyl)-benzamid

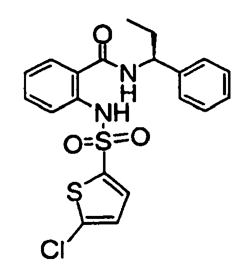

MS (ES) : 435 (M+H)⁺

### Beispiel 50: 2-(2-Methyl-chinolin-8-sulfonylamino)-N-(1-phenyl-propyl)-benzamid

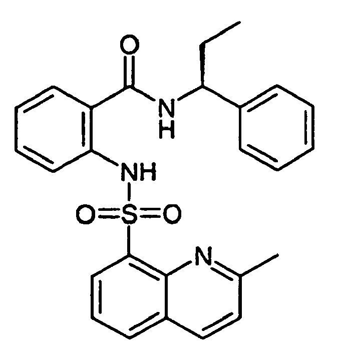

MS (ES) : 460 (M+H)⁺

### Beispiel 51: (S)-2-(4-Chlor-chinolin-8-sulfonylamino)-N-(1-phenyl-propyl)-benzamid

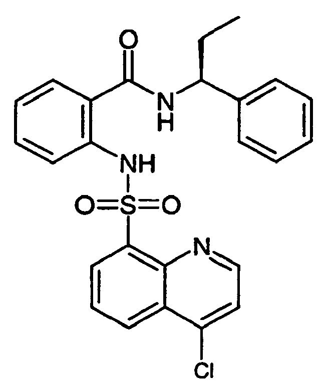

MS (ES) : 480 (M+H)⁺

### Beispiel 52: (S)-5-Fluor-2-(chinolin-8-sulfonylamino)- N-(1-phenyl-propyl)-benzamid

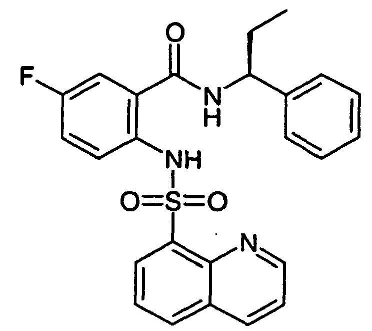

### a) 5-Fluor-2-(chinolin-8-sulfonylamino)-benzoesäure

Eine Reaktionsmischung aus 10,0 g (64 mmol) 5-Fluor-2-amino-benzoesäure, 16,3 g (193 mmol) Natriumhydrogencarbonat und 16,3 g 8-Chinolinsulfonylchlorid in 325 ml Wasser und 325 ml Essigester wurde über Nacht bei RT gerührt. Die wässerige Phase wurde abgetrennt und 1-mal mit 50 ml Essigester extrahiert. Anschließend wurde die wässerige Phase mit konz. Salzsäure sauer gestellt und 2 h gerührt. Der ausgefallene Niederschlag wurde abgesaugt, im Vakuum getrocknet und man erhielt 19,5 g 5-Fluor-2-(chinolin-8-sulfonylamino)-benzoesäure.

### b) 5-Fluor-2-(chinolin-8-sulfonylamino)- N-(1-phenyl-propyl)-benzamid

Aus 5,5 g (15,9 mmol) 5-Fluor-2-(chinolin-8-sulfonylamino)-benzoesäure und 2,3 g (₁6,₇ mmol) (S)-Phenylpropylamin wurden gemäß der allgemeinen Vorschrift 6 5,7 g der Titelverbindung erhalten.
Fp.: 163°C; MS (ES) : 464 (M+H)⁺

### (S)-5-Fluor-2-(chinolin-8-sulfonylamino)- N-(1-phenyl-propyl)-benzamid- Natriumsalz

Zu einer Lösung von 5 g der Verbindung des Beispiels 52 in 120 ml Essigester wurden 2 ml einer 30-prozentigen Natriummethanolatlösung gegeben. Das ausgefallene Natriumsalz wurde abgesaugt und aus 25 ml Ethanol umkristallisiert und man erhielt 3,3 g der Titelverbindung.

Die Titelverbindungen der Beispiele 53 - 58 wurden aus den entsprechenden Vorstufen 1 und (S)-Phenylpropylamin gemäß der allgemeinen Vorschrift 6 hergestellt:

### Beispiel 53: (S)-5-Methoxy-2-(chinolin-8-sulfonylamino)- N-(1-phenyl-propyl)-benzamid

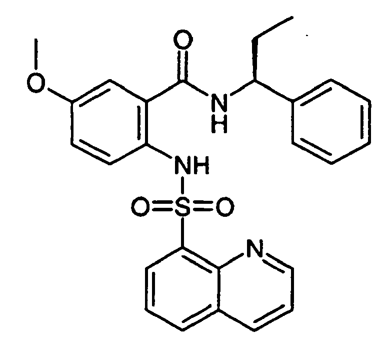

MS (ES) : 476 (M+H)⁺

### Beispiel 54: (S)-4-Fluor-2-(chinolin-8-sulfonylamino)- N-(1-phenyl-propyl)-benzamid

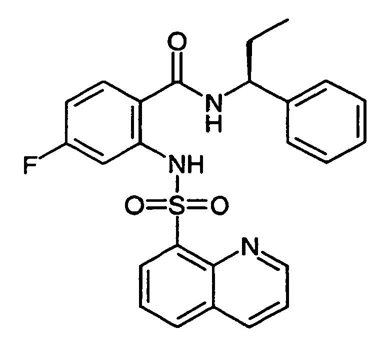

MS (ES) : 464 (M+H)⁺

### Beispiel 55: (S)-6-Chlor-2-(chinolin-8-sulfonylamino)- N-(1-phenyl-propyl)-benzamid

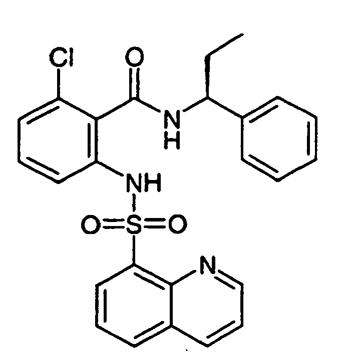

MS (ES) : 480 (M+H)⁺

### Beispiel 56: (S)-6-Fluor-2-(chinolin-8-sulfonylamino)- N-(1-phenyl-propyl)-benzamid

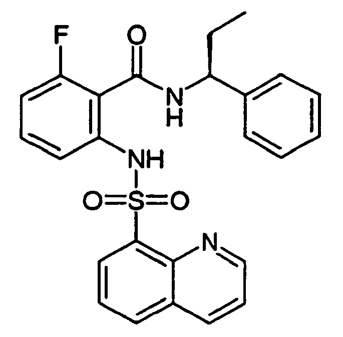

MS (ES) : 464 (M+H)⁺

### Beispiel 57: (S)-3-Chlor-2-(chinolin-8-sulfonylamino)- N-(1-phenyl-propyl)-benzamid

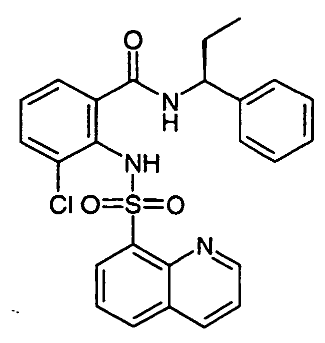

MS (ES) : 480 (M+H)⁺

### Beispiel 58: (S)-5-Chlor-2-(chinolin-8-sulfonylamino)- N-(1-phenyl-propyl)-benzamid

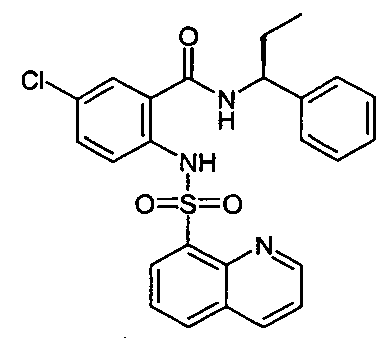

MS (ES) : 480 (M+H)⁺

Die Titelverbindungen der Beispiele 59 - 60 wurden aus den entsprechenden Vorstufen 1 und a-Cyclopropylbenzylamin (Vorstufe 3o) gemäß der allgemeinen Vorschrift 6 hergestellt:

### Beispiel 59: N-(Cyclopropyl-phenyl-methyl)-5-fluor-2-(chinolin-8-sulfonylamino)-benzamid

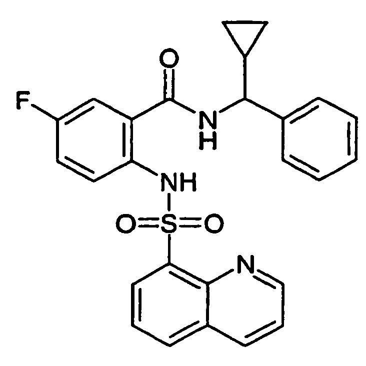

MS (ES) : 476 (M+H)⁺

### Beispiel 60: N-(Cyclopropyl-phenyl-methyl)-5-methoxy-2-(chinolin-8-sulfonylamino)-benzamid

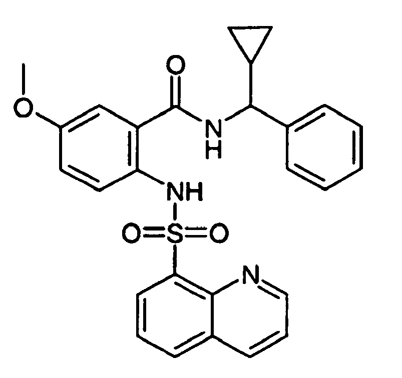

MS (ES) : 488 (M+H)⁺

### Beispiel 61: (R)-5-Fluor-2-(chinolin-8-sulfonylamino)- N-(1-phenyl-propyl)-benzamid

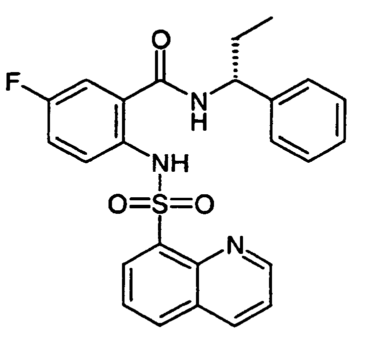

Die Titelverbindung wurde analog Beispiel 52 aus (R)-Phenylpropylamin erhalten.
MS (ES) : 464 (M+H)⁺

Die Titelverbindungen der Beispiele 62 - 63 wurden aus den entsprechenden Vorstufen 1 und 1-(5-Methyl-furan-2-yl)-propylamin (Vorstufe 3r) gemäß der allgemeinen Vorschrift 5 hergestellt:

### Beispiel 62: N-[1-(5-Methyl-furan-2-yl)-propyl]-2-(chinolin-8-sulfonylamino)-benzamid

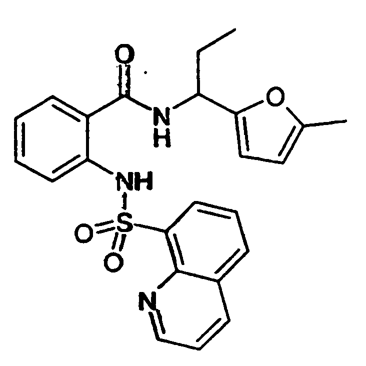

MS (ES) : 450 (M+H)⁺

### Beispiel 63: 5-Fluor-N-[1-(5-methyl-furan-2-yl)-propyl]-2-(chinolin-8-sulfonylamino)-benzamid

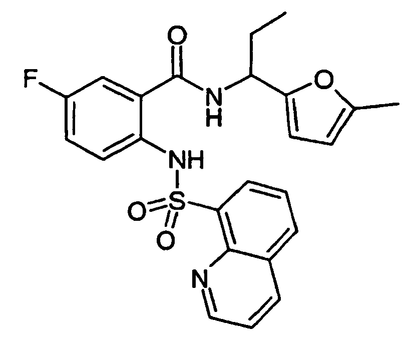

MS (ES) : 468 (M+H)⁺

Die Titelverbindungen der Beispiele 64 - 66 wurden aus den entsprechenden Vorstufen 1 und 1-Phenyl-prop-2-ynylamin (Vorstufe 3s) gemäß der allgemeinen Vorschrift 5 hergestellt:

### Beispiel 64: N-(1-Phenyl-prlop-2-ynyl)-2-(chinolin-8-sulfonylamino)-benzamid

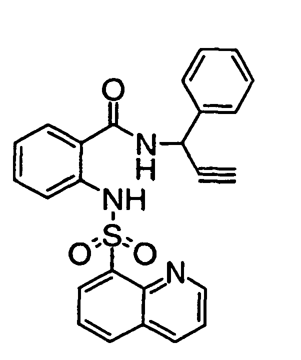

MS (ES) : 442 (M+H)⁺

### Beispiel 65: 5-Fluor-N-(1-phenyl-prop-2-ynyl)-2-(chinolin-8-sulfonylamino)-benzamid

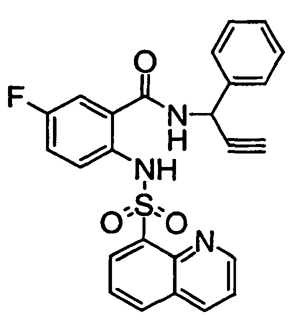

MS (ES) : 460 (M+H)⁺

### Beispiel 66: 5-Methoxy-N-(1-phenyl-prop-2-ynyl)-2-(chinolin-8-sulfonylamino)-benzamid

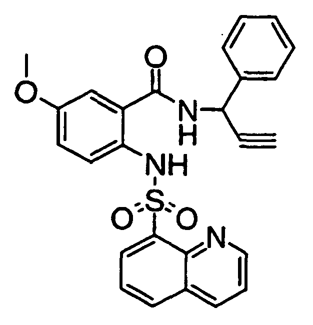

MS (ES) : 472 (M+H)⁺

### Beispiel 67: N-(1-Phenyl-propyl)-2-(pyridin-2-sulfonylamino)-benzamid

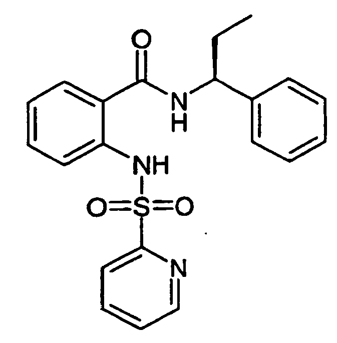

a) Pyridin-2-sulfonsäurechlorid (analog J. Org. Chem. 54, 2, 1989, 389-393). 60.4 mmol 2-Mercaptopyridin werden in 100 mL Salzsäure (20 %) gelöst und auf 2 - 5 °C abgekühlt. Anschließend wird Chlorgas 30 min. so durch die Lösung geleitet, dass die Temperatur 5 °C nicht übersteigt. Dann werden weitere 50 mL Wasser zugegeben.
   Die wässerige Phase wird mit Ether (3 x 100 mL) extrahiert, mit ges. Natriumhydrogencarbonatlösung gewaschen, getrocknet (Na₂SO₄) und konzentriert.
   Ausbeute: 4,52 g (42 %).
b) Gemäß der allgemeinen Vorschrift 7 wurden aus 100 mg (S)-2-Amino-N-(1-phenylpropyl)-benzamid und 70 mg Pyridin-2-sulfonsäurechlorid 11 mg N-(1-Phenyl-propyl)-2-(pyridin-2-sulfonylamino)-benzamid als weisser Feststoff erhalten.
   MS (ES): 396 (M+H)⁺

### Beispiel 68: 5-Methoxy-N-(1-phenyl-propyl)-2-(pyridin-2-sulfonylamino)-benzamid

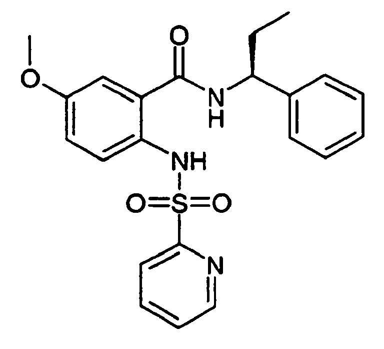

Gemäß der allgemeinen Vorschrift 7 wurden aus 100 mg (S)-2-Amino-5-methoxy-N-(1-phenyl-propyl)-benzamid und 62 mg Pyridin-2-sulfonsäurechlorid 30 mg der Titelverbindung als weisser Feststoff erhalten.
MS (ES) : 426 (M+H)⁺

### Beispiel 69: 5-Methoxy-2-(6-methyl-pyridin-3-sulfonylamino)-N-(1-phenyl-propyl)-benzamid

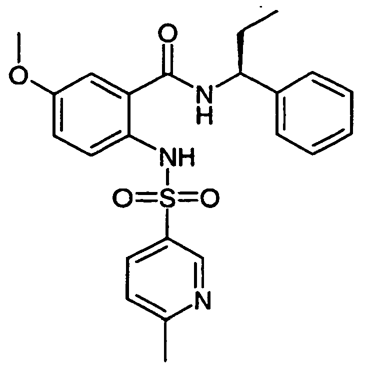

a) 6-Methyl-pyridin-3-sulfonsäure (analog J. Amer. Chem. Soc. 65, 1943, 2233-2236). Zu 0,408 mol Oleum (20 % freies Schwefeltrioxid) werden tropfenweise innerhalb von 10 min. unter Eiskühlung 0.1 mol 2-Picolin gegeben. Anschließend wird 0.843 mmol Quecksilbersulfat hinzugefügt und 24 h bei 230 °C gerührt. Dann wird die Schwefelsäure unter Vakuum abdestilliert. Unter Zugabe von 200 mL Acetonitril fällt das Produkt aus. Es wird abgesaugt, mit etwas Acetonitril nachgewaschen und bei 100 °C getrocknet. Ausbeute: 8.16 g (48 %).
b) 6-Methyl-pyridin-3-sulfonsäurechlorid (analog J. Org. Chem. 54, 2, 1989, 389-393). 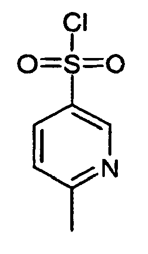
   Das Gemisch aus 47.1 mmol 6-Methyl-pyridin-3-sulfonsäure und 56.5 mmol Phosphorpentachlorid wird in 80 mL Phosphoroxidchlorid suspendiert und 24 h bei 120 °C gerührt. Die Lösung wird unter Vakuum konzentriert und unter Kühlung vorsichtig Wasser hinzugegeben. Die wässerige Phase wird dann mit Ether (3 x 100 mL) extrahiert, getrocknet (Na₂SO₄) und konzentriert. Ausbeute: 0.6 g (7 %).
c) Gemäß der allgemeinen Vorschrift 7 wurden aus 445 mg (S)-2-Amino-5-methoxy-N-(1-phenyl-propyl)-benzamid und 300 mg 6-Methyl-pyridin-3-sulfonsäurechlorid 67 mg 5-Methoxy-2-(6-methyl-pyridin-3-sulfonylamino)-N-(1-phenyl-propyl)-benzamid als weisser Feststoff erhalten.
MS (ES) : 440 (M+H)⁺

### Beispiel 70: 5-Methyl-N-[1-(5-methylfuran-2-yl)-propyl]-2-(pyridin-2-sulfonylamino)-benzamid

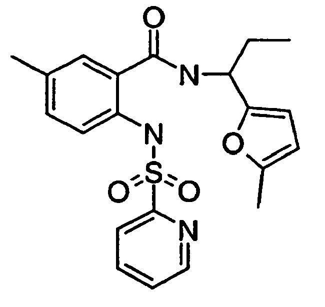

### a) 5-Methyl-N-[1-(5-methylfuran-2-yl)-propyl]-2-nitrobenzamid

2 g (10 mmol) 2-Nitro-5-methylbenzoesäurechlorid und 1,39 g (10 mmol) 1-(5-Methyl-furan-2-yl)-propylamin (= Vorstufe 3r) wurden zusammen mit 1,3 ml DIPEA in 20 ml Dichloromethan 18 h bei Raumtemperatur zur Reaktion gebracht. Die Mischung wurde mit Dichlormethan verdünnt, gewaschen, über Na₂SO₄ getrocknet und an Kieselgel chromatographisch gereinigt. Man erhielt 1,14g (3,8 mmol) eines hellgelben Feststoffs.

### b) 2-Amino-5-methyl-N-[1-(5-methylfuran-2-yl)-propyl]-benzamid

1,14 g (3,8 mmol) 5-Methyl-N-[1-(5-methylfuran-2-yl)-propyl]-2-nitro-benzamid (siehe a) wurden in 20 ml Methanol gelöst, mit 1g Palladium auf Kohle (10 %) bei Raumtemperatur unter Normaldruck hydriert. Nach Filtrieren und Einengen erhielt man 0.9 g (3,3 mmol) Feststoff.

### c) 5-Methyl-N-[1-(5-methylfuran-2-yl)-propyl]-2-(pyridin-2-sulfonylamino)- benzamid

100 mg (0,37 mmol) 2-Amino-5-methyl-N-[1-(5-methylfuran-2-yl)-propyl]-benzamid (siehe b) und 117 mg (0,66 mmol) 2-Pyridinsulfonylchlorid Hydrochlorid wurden in 1 ml Pyridin gelöst und 18 h bei Raumtemperatur zur Reaktion gebracht. Die Reaktionsmischung wurde eingeengt und mittels präparativer HPLC die Beispielverbindung 70 (61 mg, 0,12 mmol) als Trifluoracetat nach Gefriertrocknung isoliert.

Analog den zuvor beschriebenen Beispielen wurden auch folgende Verbindungen erhalten:

| Beispiel | Struktur | Masse (ES) |
|---|---|---|
| 71 | 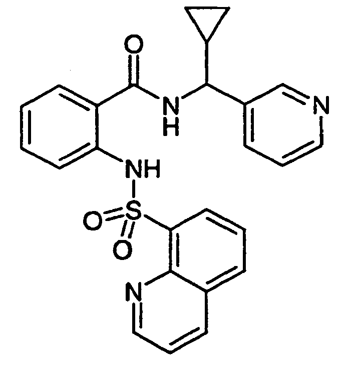 | 459 (M+1) |
| 72 | 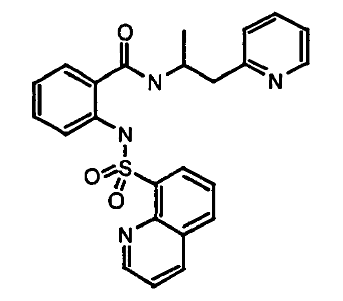 | 447 (M+1) |
| 73 | 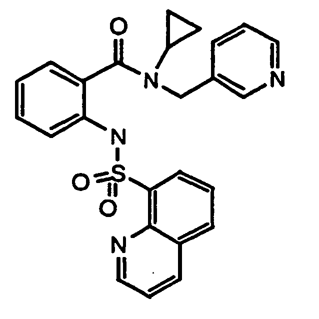 | 459 (M+1) |
| 74 | 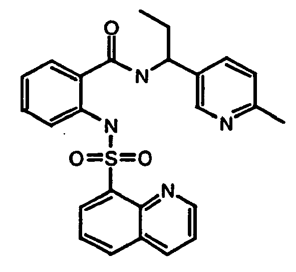 | 461 (M+1) |
| 75 | 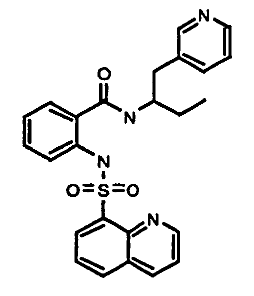 | 461 (M+1) |
| 76 | 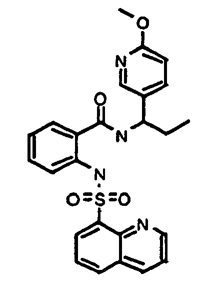 | 477(M+1) |
| 77 | 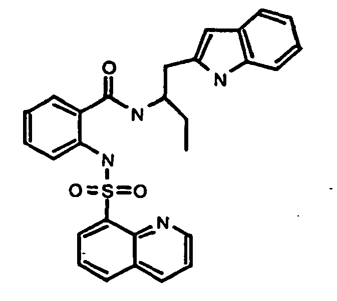 | 499 (M+1) |
| 78 | 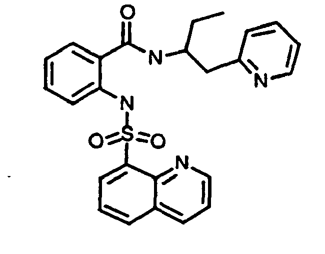 | 461 (M+1) |
| 79 | 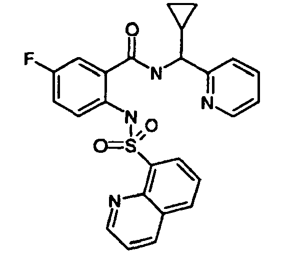 | 477 (M+1) |
| 80 | 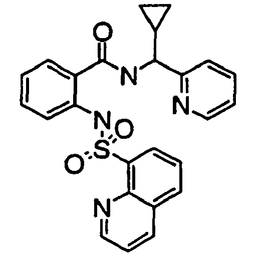 | 459 (M+1) |
| 81 | 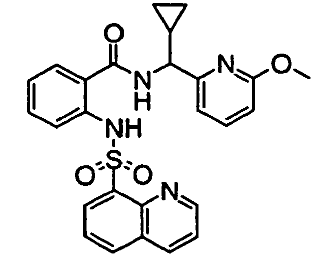 | 489 (M+1) |
| 82 | 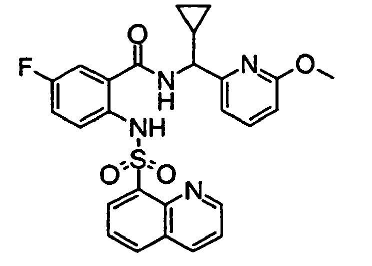 | 507 (M+1) |
| 83 | 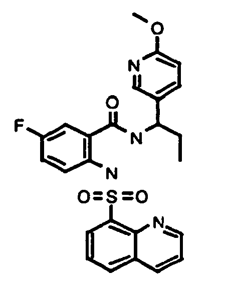 | 495 (M+1) |
| 84 | 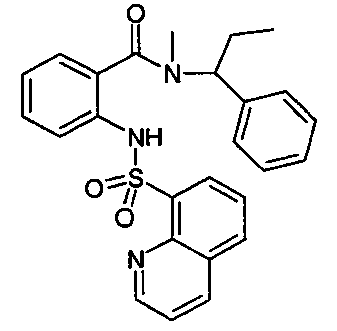 | 460 (M+1) |

## Patentansprüche

1. Verbindungen der Formel I, 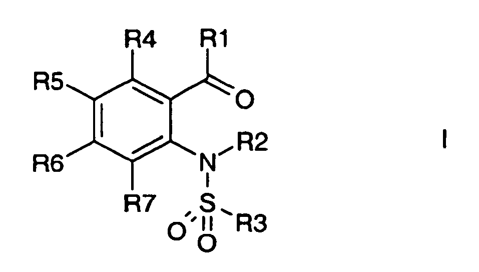 worin bedeuten:
R(1) 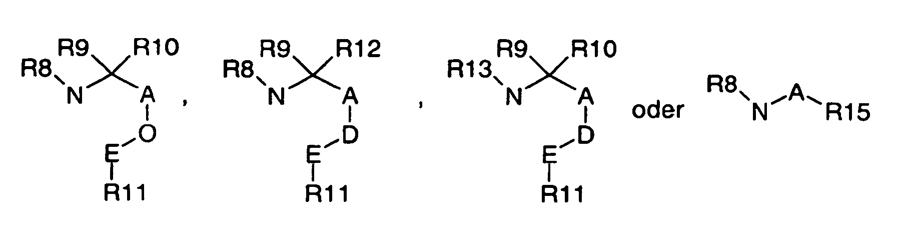
A -CₙH₂n-;
n = 0, 1, 2, 3, 4 oder 5;
D eine Bindung oder -O-;
E -CₘH₂ₘ-;
m = 0, 1, 2, 3, 4 oder 5;
R(8) Wasserstoff , Alkyl mit 1, 2, 3 oder 4 C-Atomen oder CₚH₂ₚ-R(14);
p 0, 1, 2, 3, 4 oder 5;
R(14) Phenyl, Naphthyl oder Heteroaryl,
wobei Phenyl, Naphthyl und Heteroaryl unsubstituiert sind oder substituiert mit 1, 2 oder 3 Substituenten ausgewählt aus der Gruppe bestehend aus F, Cl, Br, l, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(9) Wasserstoff oder Alkyl mit 1, 2, 3, 4, 5 oder 6 C-Atomen;
R(10) Wasserstoff, Alkyl mit 1, 2, 3 oder 4 C-Atomen, Phenyl, Naphthyl oder Heteroaryl,
wobei Phenyl, Naphthyl und Heteroaryl unsubstituiert sind oder substituiert mit 1, 2 oder 3 Substituenten ausgewählt aus der Gruppe bestehend aus F, Cl, Br, l, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(11) Cycloalkyl mit 3, 4, 5 oder 6 C-Atomen, Phenyl, Naphthyl, Thienyl, Furyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl,
wobei Phenyl, Naphthyl, Thienyl, Furyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl unsubstituiert sind oder substituiert mit 1, 2 oder 3 Substituenten ausgewählt aus der Gruppe bestehend aus F, Cl, Br, l, CF₃, OCF₃, NO₂, CN, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(12) Alkyl mit 1, 2, 3 oder 4 C-Atomen, Alkinyl mit 1, 2, 3 oder 4 C-Atomen, Cycloalkyl mit 3, 4, 5 oder 6 C-Atomen, Phenyl, Naphthyl oder Heteroaryl,
wobei Phenyl, Naphthyl und Heteroaryl unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(13) CₚH₂ₚ-R(14);
p 0, 1, 2, 3, 4 oder 5;
R(15) Cycloalkyl mit 3, 4, 5, 6, 7 oder 8 C-Atomen;
R(2) Wasserstoff oder Alkyl mit 1, 2, 3 oder 4 C-Atomen;
R(3) Heteroaryl,
wobei Heteroaryl unsubstituiert ist oder substituiert mit 1, 2 oder 3 Substituenten ausgewählt aus der Gruppe bestehend aus F, Cl, Br, l, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(4), R(5), R(6) und R(7) unabhängig voneinander Wasserstoff, F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
sowie ihre pharmazeutisch verträglichen Salze.

2. Verbindungen der Formel I nach Anspruch 1, worin bedeuten:
R(1) 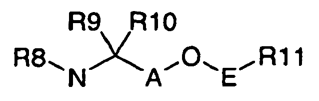
A -CₙH₂ₙ-;
n 0, 1, 2 oder 3;
E -CₘH₂ₘ-;
m 0, 1, 2 oder 3;
R(8) Wasserstoff , Alkyl mit 1, 2 oder 3 C-Atomen oder CₚH₂ₚ-R(14);
p 0, 1, 2, oder 3;
R(14) Phenyl, Naphthyl oder Heteroaryl,
wobei Phenyl, Naphthyl und Heteroaryl unsubstituiert sind oder substituiert mit 1, 2 oder 3 Substituenten ausgewählt aus der Gruppe bestehend aus F, Cl, CF₃, OCF₃, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(9) Wasserstoff oder Alkyl mit 1, 2, 3 oder 4 C-Atomen;
R(10) Wasserstoff, Alkyl mit 1, 2 oder 3 C-Atomen, Phenyl, Naphthyl oder Heteroaryl,
wobei Phenyl, Naphthyl und Heteroaryl unsubstituiert sind oder substituiert mit 1, 2 oder 3 Substituenten ausgewählt aus der Gruppe bestehend aus F, Cl, CF₃, OCF₃, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(11) Phenyl, Naphthyl, Thienyl, Furyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl,
wobei Phenyl, Naphthyl, Thienyl, Furyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl unsubstituiert sind oder substituiert mit 1, 2 oder 3 Substituenten ausgewählt aus der Gruppe bestehend aus F, Cl, CF₃, OCF₃, CN, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(2) Wasserstoff oder Alkyl mit 1, 2 oder 3 C-Atomen;
R(3) Heteroaryl,
wobei Heteroaryl unsubstituiert ist oder substituiert mit 1, 2 oder 3 Substituenten ausgewählt aus der Gruppe bestehend aus F, Cl, CF₃, OCF₃, CN, COOMe. CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(4), R(5), R(6) und R(7) unabhängig voneinander Wasserstoff, F, Cl, CF₃, OCF₃, CN, COMe, OH, Alkyl mit 1, 2, 3 oder 4 C-Atomen, Methoxy, Ethoxy, Dimethylamino, Sulfamoyl, Methylsulfonyl und Methylsulfonylamino;
sowie ihre pharmazeutisch verträglichen Salze.

3. Verbindungen der Formel I nach Ansprüchen 1 oder 2, worin bedeuten:
R(1) 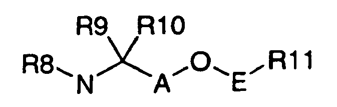
A -CₙH₂ₙ-;
n 0 oder 1;
E -CₘH₂ₘ-;
m 0 oder 1;
R(8) Wasserstoff , Alkyl mit 1, 2 oder 3 C-Atomen oder CpH₂ₚ-R(14);
p 0 oder 1;
R(14) Phenyl, Naphthyl oder Heteroaryl,
wobei Phenyl, Naphthyl und Heteroaryl unsubstituiert sind oder substituiert mit 1 oder 2 Substituenten ausgewählt aus der Gruppe bestehend aus F, Cl, CF₃, OCF₃, CN, COMe, Alkyl mit 1, 2, 3 oder 4 C-Atomen, Methoxy, Ethoxy, Dimethylamino, Sulfamoyl, Methylsulfonyl und Methylsulfonylamino;
R(9) Wasserstoff, Methyl oder Ethyl;
R(10) Wasserstoff, Alkyl mit 1, 2 oder 3 C-Atomen, Phenyl, Naphthyl oder Heteroaryl,
wobei Phenyl, Naphthyl und Heteroaryl unsubstituiert sind oder substituiert mit 1 oder 2 Substituenten ausgewählt aus der Gruppe bestehend aus F, Cl, CF₃, OCF₃, CN, COMe, Alkyl mit 1, 2, 3 oder 4 C-Atomen, Methoxy, Ethoxy, Dimethylamino, Sulfamoyl, Methylsulfonyl und Methylsulfonylamino;
R(11) Phenyl, Naphthyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl,
wobei Phenyl, Naphthyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl unsubstituiert sind oder substituiert mit 1 oder 2 Substituenten ausgewählt aus der Gruppe bestehend aus F, Cl, CF₃, OCF₃, CN, COMe, Alkyl mit 1, 2, 3 oder 4 C-Atomen, Methoxy, Ethoxy, Dimethylamino, Sulfamoyl, Methylsulfonyl und Methylsulfonylamino;
R(2) Wasserstoff, Methyl oder Ethyl;
R(3) Heteroaryl,
wobei Heteroaryl unsubstituiert ist oder substituiert mit 1 oder 2 Substituenten ausgewählt aus der Gruppe bestehend aus F, Cl, CF₃, OCF₃, CN, COMe, Alkyl mit 1, 2, 3 oder 4 C-Atomen, Methoxy, Ethoxy, Dimethylamino, Sulfamoyl, Methylsulfonyl und Methylsulfonylamino;
R(4), R(5), R(6) und R(7) unabhängig voneinander Wasserstoff, F, Cl, CF₃, OCF₃, CN, COMe, OH, Alkyl mit 1, 2, 3 oder 4 C-Atomen, Methoxy, Ethoxy, Dimethylamino, Sulfamoyl, Methylsulfonyl und Methylsulfonylamino;
sowie ihre pharmazeutisch verträglichen Salze.

4. Verbindungen 1 nach Ansprüchen 1 bis 3, worin bedeuten
R(1) 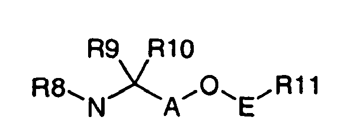
A -CₙH₂ₙ-;
n 0 oder 1;
E -CₘH₂ₘ-;
m 0 oder 1;
R(8) Wasserstoff, Alkyl mit 1, 2 oder 3 C-Atomen oder CₚH₂ₚ-R(14);
p 0 oder 1;
R(14) Phenyl, Naphthyl oder Heteroaryl,
wobei Phenyl, Naphthyl und Heteroaryl unsubstituiert sind oder substituiert mit 1 oder 2 Substituenten ausgewählt aus der Gruppe bestehend aus F, Cl, CF₃, OCF₃, CN, COMe, Methyl, Methoxy, Dimethylamino, Sulfamoyl oder Methylsulfonyl;
R(9) Wasserstoff, Methyl oder Ethyl;
R(10) Wasserstoff, Alkyl mit 1, 2 oder 3 C-Atomen, Phenyl, Naphthyl oder Heteroaryl,
wobei Phenyl, Naphthyl und Heteroaryl unsubstituiert sind oder substituiert mit 1 oder 2 Substituenten ausgewählt aus der Gruppe bestehend aus F, Cl, CF₃, OCF₃, CN, COMe, Methyl, Methoxy, Ethoxy, Dimethylamino, Sulfamoyl oder Methylsulfonyl;
R(11) Phenyl, Naphthyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl,
wobei Phenyl, Naphthyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl unsubstituiert sind oder substituiert mit 1 oder 2 Substituenten ausgewählt aus der Gruppe bestehend aus F, Cl, CF₃, OCF₃, CN, COMe, Methyl, Methoxy, Ethoxy, Dimethylamino, Sulfamoyl oder Methylsulfonyl;
R(2) Wasserstoff;
R(3) Heteroaryl,
wobei Heteroaryl unsubstituiert ist oder substituiert mit 1 oder 2 Substituenten ausgewählt aus der Gruppe bestehend aus F, Cl, CF₃, OCF₃, CN, COMe, Methyl, Methoxy, Ethoxy, Dimethylamino, Sulfamoyl oder Methylsulfonyl;
R(4) Wasserstoff, F; Cl, CF₃, Methyl oder Methoxy;
R(5) Wasserstoff, F, Cl, CF₃, Methyl, Methoxy, COMe, OCF₃, CN oder OH;
R(6) Wasserstoff, F, Cl, CF₃, Methyl oder Methoxy;
R(7) Wasserstoff, F, Cl, CF₃, Methyl, Ethyl, Methoxy oder OH;
sowie ihre pharmazeutisch verträglichen Salze.

5. Verbindungen der Formel I nach Anspruch 1, worin bedeuten:
R(1) 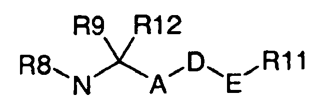
A -CₙH₂ₙ-;
n 0, 1, 2 oder 3;
D eine Bindung oder -O-;
E -CₘH₂ₘ-;
m 0,1, 2 oder 3;
R(8) Wasserstoff, Alkyl mit 1, 2 oder 3 C-Atomen oder CₚH₂ₚ-R(14)
p 0, 1, 2, oder 3;
R(14) Phenyl, Naphthyl oder Heteroaryl,
wobei Phenyl, Naphthyl und Heteroaryl unsubstituiert sind oder substituiert mit 1, 2 oder 3 Substituenten ausgewählt aus der Gruppe bestehend aus F, Cl, CF₃, OCF₃, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(9) Wasserstoff oder Alkyl mit 1, 2, 3 oder 4 C-Atomen;
R(11) Phenyl, Naphthyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl
wobei Phenyl, Naphthyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl unsubstituiert sind oder substituiert mit 1, 2 oder 3 Substituenten ausgewählt aus der Gruppe bestehend aus F, Cl, CF₃, OCF₃, CN, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(12) Alkyl mit 1, 2 oder 3 C-Atomen, Alkinyl mit 1, 2 oder 3 C-Atomen, Cycloalkyl mit 3, 4, 5 oder 6 C-Atomen, Phenyl, Naphthyl oder Heteroaryl,
wobei Phenyl, Naphthyl und Heteroaryl unsubstituiert sind oder substituiert mit 1, 2 oder 3 Substituenten ausgewählt aus der Gruppe bestehend aus F, Cl, CF₃, OCF₃, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(2) Wasserstoff oder Alkyl mit 1, 2 oder 3 C-Atomen;
R(3) Heteroaryl,
wobei Heteroaryl unsubstituiert ist oder substituiert mit 1, 2 oder 3 Substituenten ausgewählt aus der Gruppe bestehend aus F, Cl, CF₃, OCF₃, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(4), R(5), R(6) und R(7) unabhängig voneinander Wasserstoff, F, Cl, CF₃, OCF₃, CN, COMe, OH, Alkyl mit 1, 2, 3 oder 4 C-Atomen, Methoxy, Ethoxy, Dimethylamino, Sulfamoyl, Methylsulfonyl und Methylsulfonylamino;
und ihre pharmakologisch verträglichen Salze.

6. Verbindungen I nach Ansprüchen 1 und/oder 5, worin bedeuten:
R(1) 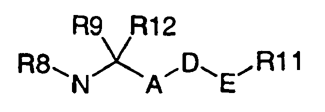
A CₙH₂ₙ-;
n 0 oder 1;
D eine Bindung oder -O-;
E -CₘH₂ₘ-;
m 0 oder 1;
R(8) Wasserstoff , Alkyl mit 1, 2 oder 3 C-Atomen oder CₚH₂ₚ-R(14)
p 0 oder 1;
R(14) Phenyl, Naphthyl oder Heteroaryl,
wobei Phenyl, Naphthyl und Heteroaryl unsubstituiert sind oder substituiert mit 1 oder 2 Substituenten ausgewählt aus der Gruppe bestehend aus F, Cl, CF₃, OCF₃, CN, COMe, Alkyl mit 1, 2, 3 oder 4 C-Atomen, Methoxy, Ethoxy, Dimethylamino, Sulfamoyl, Methylsulfonyl und Methylsulfonylamino;
R(9) Wasserstoff. Methyl oder Ethyl;
R(11) Phenyl, Naphthyl, Pyridyl, Pyrazinyl, Pyrimidinyl, Pyridazinyl, lndolyl, lndazolyl, Chinolyl, Isochinolyl, Phthalazinyl, Chinoxalinyl, Chinazolinyl oder Cinnolinyl,
wobei Phenyl, Naphthyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl unsubstituiert sind oder substituiert mit 1 oder 2 Substituenten ausgewählt aus der Gruppe bestehend aus F, Cl, CF₃, OCF₃, CN, COMe, Alkyl mit 1, 2, 3 oder 4 C-Atomen, Methoxy, Ethoxy, Dimethylamino, Sulfamoyl, Methylsulfonyl und Methylsulfonylamino;
R(12) Alkyl mit 1, 2 oder 3 C-Atomen, Ethinyl, Cyclopropyl, Phenyl, Naphthyl oder Heteroaryl,
wobei Phenyl, Naphthyl und Heteroaryl unsubstituiert sind oder substituiert mit 1 oder 2 Substituenten ausgewählt aus der Gruppe bestehend aus F, Cl, CF₃, OCF₃, CN, COMe, Alkyl mit 1, 2, 3 oder 4 C-Atomen, Methoxy, Ethoxy, Dimethylamino, Sulfamoyl, Methylsulfonyl und Methylsulfonylamino;
R(2) Wasserstoff, Methyl oder Ethyl;
R(3) Heteroaryl,
wobei Heteroaryl unsubstituiert ist oder substituiert mit 1 oder 2 Substituenten ausgewählt aus der Gruppe bestehend aus F, Cl, CF₃, OCF₃, CN, COMe, alkyl mit 1, 2, 3 oder 4 C-Atomen, Methoxy, Ethoxy, Dimethylamino, Sulfamoyl, Methylsulfonyl und Methylsulfonylamino;
R(4), R(5), R(6) und R(7) unabhängig voneinander Wasserstoff, F, Cl, CF₃, OCF₃, CN, COMe, OH, Alkyl mit 1, 2, 3 oder 4 C-Atomen, Methoxy, Ethoxy, Dimethylamino, Sulfamoyl, Methylsulfonyl und Methylsulfonylamino;
und ihre pharmakologisch verträglichen Salze.

7. Verbindungen der Formel I nach Ansprüchen 1, 5 und/oder 6, worin bedeuten: R(1) 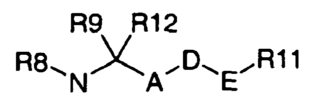
A -CₙH₂ₙ-;
n 0 oder 1;
D eine Bindung oder -O-;
E -CₘH₂ₘ-;
m 0 oder 1;
R(8) Wasserstoff , Alkyl mit 1, 2 oder 3 C-Atomen oder CₚH₂ₚ-R(14);
p 0 oder 1;
R(14) Phenyl, Naphthyl oder Heteroaryl,
wobei Phenyl, Naphthyl und Heteroaryl unsubstituiert sind oder substituiert mit 1 oder 2 Substituenten ausgewählt aus der Gruppe bestehend aus F, Cl, CF₃, OCF₃, CN, COMe, Methyl, Methoxy, Ethoxy, Dimethylamino, Sulfamoyl, Methylsulfonyl und Methylsulfonylamino;
R(9) Wasserstoff, Ethyl oder Methyl;
R(11) Phenyl, Naphthyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl,
wobei Phenyl, Naphthyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl unsubstituiert sind oder substituiert mit 1 oder 2 Substituenten ausgewählt aus der Gruppe bestehend aus F, Cl, CF₃, OCF₃, CN, COMe, Methoxy, Ethoxy, Dimethylamino, Sulfamoyl und Methylsulfonyl;
R(12) Alkyl mit 1, 2 oder 3 C-Atomen, Ethinyl, Cyclopropyl, Phenyl, Naphthyl oder Heteroaryl,
wobei Phenyl, Naphthyl und Heteroaryl unsubstituiert sind oder substituiert mit 1 oder 2 Substituenten ausgewählt aus der Gruppe bestehend aus F, Cl, CF₃, OCF₃, CN, COMe, Ethoxy, Dimethylamino, Sulfamoyl, Methylsulfonyl und Methylsulfonylamino;
R(2) Wasserstoff;
R(3) Heteroaryl,
wobei Heteroaryl unsubstituiert ist oder substituiert mit 1 oder 2 Substituenten ausgewählt aus der Gruppe bestehend aus F, Cl, CF₃, OCF₃, CN, COMe, Methyl, Methoxy, Ethoxy, Dimethylamino, Sulfamoyl und Methylsulfonyl;
R(4) Wasserstoff, F, Cl, CF₃, Methyl oder Methoxy;
R(5) Wasserstoff, F, Cl, CF₃, Methyl, Methoxy, COMe, OCF₃, CN oder OH;
R(6) Wasserstoff, F, Cl, CF₃, Methyl oder Methoxy;
R(7) Wasserstoff, F, Cl, CF₃, Methyl, Methoxy oder OH;
und ihre pharmakologisch verträglichen Salze.

8. Verbindungen der Formel I nach Anspruch 1, worin bedeuten:
R(1) 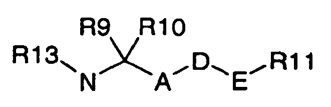 worin bedeuten:
A -CₙH₂ₙ-
n = 0, 1, 2 oder 3;
D eine Bindung oder -O-;
E -CₘH₂ₘ-
m 0, 1, 2 oder 3;
R(9) Wasserstoff oder Alkyl mit 1, 2, 3 oder 4 C-Atomen;
R(10) Wasserstoff, Alkyl mit 1, 2 oder 3 C-Atomen, Phenyl, Naphthyl oder Heteroaryl
wobei Phenyl, Naphthyl und Heteroaryl unsubstituiert sind oder substituiert mit 1, 2 oder 3 Substituenten ausgewählt aus der Gruppe bestehend aus F, Cl, CF₃, OCF₃, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(11) Phenyl, Naphthyl, Thienyl, Furanyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl,
wobei Phenyl, Naphthyl, Thienyl, Furanyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl unsubstituiert sind oder substituiert mit 1, 2 oder 3 Substituenten ausgewählt aus der Gruppe bestehend aus F, Cl, CF₃, OCF₃, CN, COMe, NH₂, OH, Alkyl mit 1, 2,3 oder 4 C-Atomen, Alkoxy mit 1, 2, 3 oder 4 C-Atomen, Dimethylamino, Sulfamoyl, Methylsulfonyl und Methylsulfonylamino;
R(13) CₚH₂ₚ-R(14);
p 0, 1, 2 oder 3;
R(14) Phenyl, Naphthyl oder Heteroaryl,
wobei Phenyl, Naphthyl und Heteroaryl unsubstituiert sind oder substituiert mit 1, 2 oder 3 Substituenten ausgewählt aus der Gruppe bestehend aus F, Cl, CF₃, OCF₃, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(2) Wasserstoff oder Alkyl mit 1, 2 oder 3 C-Atomen;
R(3) Heteroaryl,
wobei Heteroaryl unsubstituiert ist oder substituiert mit 1, 2 oder 3 Substituenten ausgewählt aus der Gruppe bestehend aus F, Cl, CF₃, OCF₃, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(4), R(5), R(6) und R(7) unabhängig voneinander Wasserstoff, F, Cl, CF₃, OCF₃, CN, COMe, OH, Alkyl mit 1, 2, 3 oder 4 C-Atomen, Methoxy, Ethoxy, Dimethylamino, Sulfamoyl, Methylsulfonyl und Methylsulfonylamino;
sowie ihre pharmazeutisch verträglichen Salze.

9. Verbindungen der Formel I nach Ansprüchen 1 und /oder 8, worin bedeuten:
R(1) 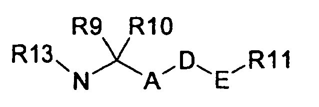
A -CₙH₂ₙ-;
n 0 oder 1;
D eine Bindung oder -O-;
E -CₘH₂m-
m 0 oder 1;
R(9) Wasserstoff, Methyl oder Ethyl;
R(10) Wasserstoff, Alkyl mit 1, 2 oder 3 C-Atomen, Phenyl, Naphthyl oder Heteroaryl,
wobei Phenyl, Naphthyl und Heteroaryl unsubstituiert sind oder substituiert mit 1 oder 2 Substituenten ausgewählt aus der Gruppe bestehend aus F, Cl, CF₃, OCF₃, CN, COMe, NH₂, OH, Alkyl mit 1, 2, 3 oder 4 C-Atomen, Methoxy, Ethoxy, Dimethylamino, Sulfamoyl, Methylsulfonyl und Methylsulfonylamino;
R(11) Phenyl, Naphthyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl,
wobei Phenyl, Naphthyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl unsubstituiert sind oder substituiert mit 1 oder 2 Substituenten ausgewählt aus der Gruppe bestehend aus F, Cl, CF₃, OCF₃, CN, COMe, Alkyl mit 1, 2, 3 oder 4 C-Atomen, Methoxy, Ethoxy, Dimethylamino, Sulfamoyl, Methylsulfonyl und Methylsulfonylamino;
R(13) CₚH₂ₚ-R(14);
p 0 oder 1;
R(14) Phenyl, Naphthyl oder Heteroaryl,
wobei Phenyl, Naphthyl und Heteroaryl unsubstituiert sind oder substituiert mit 1 oder 2 Substituenten ausgewählt aus der Gruppe bestehend aus F, Cl, CF₃, OCF₃, CN, COMe, Alkyl mit 1, 2, 3 oder 4 C-Atomen, Methoxy, Ethoxy, Dimethylamino, Sulfamoyl, Methylsulfonyl und Methylsulfonylamino;
R(2) Wasserstoff, Methyl oder Ethyl;
R(3) Heteroaryl,
wobei Heteroaryl unsubstituiert ist oder substituiert mit 1 oder 2 Substituenten ausgewählt aus der Gruppe bestehend aus F, Cl, CF₃, OCF₃, CN, COMe, Alkyl mit 1, 2, 3 oder 4 C-Atomen, Methoxy, Ethoxy, Dimethylamino, Sulfamoyl, Methylsulfonyl und Methylsulfonylamino;
R(4), R(5), R(6) und R(7) unabhängig voneinander Wasserstoff, F, Cl, CF₃, OCF₃, CN, COMe, OH, Alkyl mit 1, 2, 3 oder 4 C-Atomen, Methoxy, Ethoxy, Dimethylamino, Sulfamoyl, Methylsulfonyl und Methylsulfonylamino;
sowie ihre pharmazeutisch verträglichen Salze.

10. Verbindungen der Formel nach Ansprüchen 1, 8 und/oder 9, worin bedeuten
R(1) 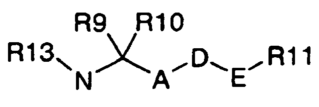
A -CₙH₂ₙ₋;
n 0 oder 1;
D eine Bindung oder -O-;
E -CₘH₂ₘ-
m 0 oder 1;
R(9) Wasserstoff, Methyl oder Ethyl;
R(10) Wasserstoff, Alkyl mit 1, 2 oder 3 C-Atomen, Phenyl, Naphthyl oder Heteroaryl,
wobei Phenyl, Naphthyl und Heteroaryl unsubstituiert sind oder substituiert mit 1 oder 2 Substituenten ausgewählt aus der Gruppe bestehend aus F, Cl, CF₃, OCF₃, CN, COMe, Methyl, Methoxy, Ethoxy, Dimethylamino, Sulfamoyl, Methylsulfonyl und Methylsulfonylamino;
R(11) Phenyl, Naphthyl, Pyridyl, Pyrazinyl, Pyrimidinyl, Pyridazinyl, Indolyl, Indazolyl, Chinolyl, lsochinolyl, Phthalazinyl, Chinoxalinyl, Chinazolinyl oder Cinnolinyl,
wobei Phenyl, Naphthyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl unsubstituiert sind oder substituiert mit 1 oder 2 Substituenten ausgewählt aus der Gruppe bestehend aus F, Cl, CF₃, OCF₃, CN, COMe, Methyl, Methoxy, Ethoxy, Dimethylamino, Sulfamoyl und Methylsulfonyl;
R(13) CₚH₂ₚ-R(14);
p 0 oder 1;
R(14) Phenyl, Naphthyl oder Heteroaryl,
wobei Phenyl, Naphthyl und Heteroaryl unsubstituiert sind oder substituiert mit 1 oder 2 Substituenten ausgewählt aus der Gruppe bestehend aus F, Cl, CF₃, OCF₃, CN, COMe, Methoxy, Ethoxy, Dimethylamino, Sulfamoyl und Methylsulfonyl;
R(2) Wasserstoff;
R(3) Heteroaryl,
wobei Heteroaryl unsubstituiert ist oder substituiert mit 1, 2 oder 3 Substituenten ausgewählt aus der Gruppe bestehend aus F, Cl, CF₃, OCF₃, CN, COMe, Methoxy, Ethoxy, Dimethylamino, Sulfamoyl und Methylsulfonyl;
R(4) Wasserstoff, F, Cl, CF₃, Methyl oder Methoxy;
R(5) Wasserstoff, F, Cl, CF₃, Methyl, Methoxy, COMe, OCF₃, CN oder OH;
R(6) Wasserstoff, F, Cl, CF₃, Methyl, Methoxy oder OH
R(7) Wasserstoff, F, Cl, CF₃, Methyl, Ethyl, Methoxy oder OH;
sowie ihre pharmazeutisch verträglichen Salze.

11. Verbindungen der Formel I nach Anspruch 1, worin bedeuten:
R(1) 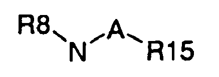
A -CₙH₂n-;
n =0,1,2 oder 3
R(8) Wasserstoff, Alkyl mit 1, 2 oder 3 C-Atomen oder CₚH₂ₚ-R(14);
p 0, 1, 2, oder 3;
R(14) Phenyl, Naphthyl oder Heteroaryl,
wobei Phenyl, Naphthyl und Heteroaryl unsubstituiert sind oder substituiert mit 1, 2 oder 3 Substituenten ausgewählt aus der Gruppe bestehend aus F, Cl, CF₃, OCF₃, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(2) Wasserstoff oder Alkyl mit 1, 2 oder 3 C-Atomen;
R(3) Heteroaryl,
wobei Heteroaryl unsubstituiert ist oder substituiert mit 1, 2 oder 3 Substituenten ausgewählt aus der Gruppe bestehend aus F, Cl, CF₃, OCF₃, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(4), R(5), R(6) und R(7) unabhängig voneinander Wasserstoff, F, Cl, CF₃, OCF₃, CN, COMe, OH, Alkyl mit 1, 2, 3 oder 4 C-Atomen, Methoxy, Ethoxy, Dimethylamino, Sulfamoyl, Methylsulfonyl und Methylsulfonylamino;
R(15) Cycloalkyl mit 3, 4, 5, 6 oder 7 C-Atomen;
sowie ihre pharmazeutisch verträglichen Salze.

12. Verbindungen der Formel I nach Ansprüchen 1 und/oder 11, worin bedeuten:
R(1) 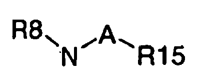
A -CₙH₂n-;
n 0 oder 1;
R(8) Wasserstoff , Alkyl mit 1, 2 oder 3 C-Atomen oder CₚH₂ₚ-R(14);
p 0 oder 1;
R(14) Phenyl, Naphthyl oder Heteroaryl,
wobei Phenyl, Naphthyl und Heteroaryl unsubstituiert sind oder substituiert mit 1 oder 2 Substituenten ausgewählt aus der Gruppe bestehend aus F, Cl, CF₃, OCF₃, CN, COMe, Alkyl mit 1, 2, 3 oder 4 C-Atomen, Methoxy, Ethoxy, Dimethylamino, Sulfamoyl, Methylsulfonyl und Methylsulfonylamino;
R(2) Wasserstoff, Methyl oder Ethyl;
R(3) Heteroaryl,
wobei Heteroaryl unsubstituiert ist oder substituiert mit 1 oder 2 Substituenten ausgewählt aus der Gruppe bestehend aus F, Cl, CF₃, OCF₃, CN, COMe, Alkyl mit 1, 2, 3 oder 4 C-Atomen, Methoxy, Ethoxy, Dimethylamino, Sulfamoyl, Methylsulfonyl und Methylsulfonylamino;
R(4), R(5), R(6) und R(7) unabhängig voneinander Wasserstoff, F, Cl, CF₃, OCF₃, CN, COMe, OH, Alkyl mit 1, 2, 3 oder 4 C-Atomen, Methoxy, Ethoxy, Dimethylamino, Sulfamoyl, Methylsulfonyl und Methylsulfonylamino; R(15) Cycloalkyl mit 3, 4, 5, 6 oder 7 C-Atomen;
sowie ihre pharmazeutisch verträglichen Salze.

13. Verbindungen der Formel I nach Ansprüchen 1, 11 und/oder 12, worin bedeuten:
R(1) 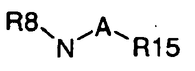
A -CₙH₂ₙ-;
n 0 oder 1;
R(8) Wasserstoff , Alkyl mit 1, 2 oder 3 C-Atomen oder CₚH₂ₚ-R(14);
p 0 oder 1;
R(14) Phenyl, Naphthyl oder Heteroaryl,
wobei Phenyl, Naphthyl und Heteroaryl unsubstituiert sind oder substituiert mit 1 oder 2 Substituenten ausgewählt aus der Gruppe bestehend aus F, Cl, CF₃, OCF₃, CN, COMe, Methyl, Methoxy, Ethoxy, Dimethylamino, Sulfamoyl und Methylsulfonyl;
R(2) Wasserstoff;
R(3) Heteroaryl,
wobei Heteroaryl unsubstituiert ist oder substituiert mit 1 oder 2 Substituenten ausgewählt aus der Gruppe bestehend aus F, Cl, CF₃, OCF₃, CN, COMe, Methyl, Methoxy, Ethoxy, Dimethylamino, Sulfamoyl oder Methylsulfonyl;
R(4) Wasserstoff, F, Cl, CF₃, Methyl oder Methoxy;
R(5) Wasserstoff, F, Cl, CF₃, Methyl, Methoxy, COMe, OCF₃; CN oder OH;
R(6) Wasserstoff, F, Cl, CF₃, Methyl, Methoxy oder OH;
R(7) Wasserstoff, F, Cl, CF₃, Methyl, Ethyl, Methoxy oder OH;
R(15) Cycloalkyl mit 3, 4, 5, 6 oder 7 C-Atomen;
sowie ihre pharmazeutisch verträglichen Salze.

14. Verbindung der Formel I nach einem oder mehreren der Ansprüche 1, 5, 6 oder 7, die ist: 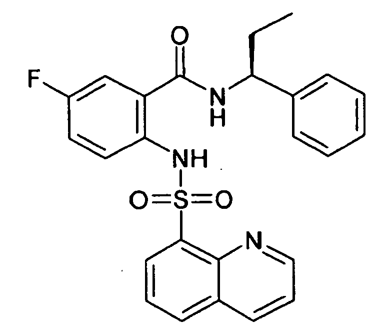 sowie ihre pharmazeutisch verträglichen Salze.

15. Verbindungen der Formel I nach einem oder mehreren der Ansprüche 1 bis 14 und ihre physiologisch verträglichen Salze zur Anwendung als Arzneimittel.

16. Pharmazeutische Zubereitung, enthaltend eine wirksame Menge mindestens einer Verbindung der Formel I nach einem oder mehreren der Ansprüche 1 bis 14 und/oder eines physiologisch verträglichen Salzes davon als Wirkstoff, zusammen mit pharmazeutisch annehmbaren Träger- und Zusatzstoffen und gegebenenfalls noch einem oder mehreren anderen pharmakologischen Wirkstoffen.

17. Verwendung einer Verbindung der Formel I nach einem oder mehreren der Ansprüche 1 bis 14 und/oder eines physiologisch verträglichen Salzes davon zur Herstellung eines Medikaments zur Therapie oder Prophylaxe von Herzrhythmusstörungen, die durch Aktionspotential-Verlängerung behoben werden können.

18. Verwendung einer Verbindung der Formel I nach einem oder mehreren der Ansprüche 1 bis 14 und/oder eines physiologisch verträglichen Salzes davon zur Herstellung eines Medikaments zur Therapie oder Prophylaxe von Reentry-Arrhythmien.

19. Verwendung einer Verbindung der Formel I nach einem oder mehreren der Ansprüche 1 bis 14 und/oder eines physiologisch verträglichen Salzes davon zur Herstellung eines Medikaments zur Therapie oder Prophylaxe von supraventrikulären Arrhythmien.

20. Verwendung einer Verbindung der Formel I nach einem oder mehreren der Ansprüche 1 bis 14 und/oder eines physiologisch verträglichen Salzes davon zur Herstellung eines Medikaments zur Therapie oder Prophylaxe von atrialer Fibrillation oder atrialem Flattern.

21. Pharmazeutische Zubereitung, enthaltend eine wirksame Menge mindestens einer Verbindung der Formel I nach einem oder mehreren der Ansprüche 1 bis 14 und/oder eines physiologisch verträglichen Salzes davon sowie eines Beta-blockers als Wirkstoffe, zusammen mit pharmazeutisch annehmbaren Träger- und Zusatzstoffen.

## Claims

1. A compound of the formula I 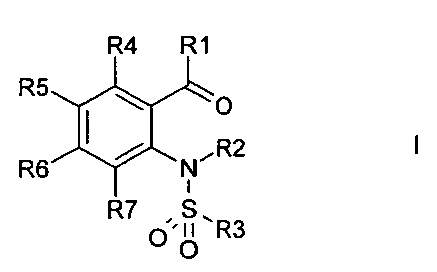 in which:
R(1) is 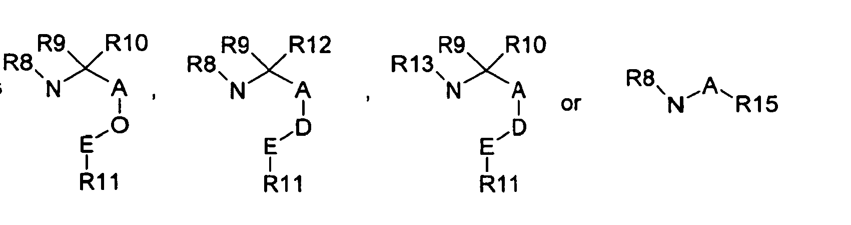
A is -CₙH₂ₙ-;
n = 0, 1, 2, 3, 4 or 5;
D is a bond or -O-;
E is -CₘH₂ₘ-;
m = 0, 1, 2, 3, 4 or 5;
R(8) is hydrogen, alkyl having 1, 2, 3 or 4 carbon atoms or CpH₂p-R(14);
p is 0, 1, 2, 3, 4 or 5;
R(14) is phenyl, naphthyl or heteroaryl,
where phenyl, naphthyl and heteroaryl are unsubstituted or substituted by 1, 2 or 3 substituents selected from the group consisting of F, Cl, Br, l, CF₃, OCF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(9) is hydrogen or alkyl having 1, 2, 3, 4, 5 or 6 carbon atoms;
R(10) is hydrogen, alkyl having 1, 2, 3 or 4 carbon atoms, phenyl, naphthyl or heteroaryl,
where phenyl, naphthyl and heteroaryl are unsubstituted or substituted by 1, 2 or 3 substituents selected from the group consisting of F, Cl, Br, l, CF₃, OCF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(11) is cycloalkyl having 3, 4, 5 or 6 carbon atoms, phenyl, naphthyl, thienyl, furyl, pyridyl, pyrazinyl, pyrimidinyl, pyridazinyl, indolyl, indazolyl, quinolyl, isoquinolyl, phthalazinyl, quinoxalinyl, quinazolinyl or cinnolinyl,
where phenyl, naphthyl, thienyl, furyl, pyridyl, pyrazinyl, pyrimidinyl, pyridazinyl, indolyl, indazolyl, quinolyl, isoquinolyl, phthalazinyl, quinoxalinyl, quinazolinyl or cinnolinyl are unsubstituted or substituted by 1, 2 or 3 substituents selected from the group consisting of F, Cl, Br, l, CF₃, OCF₃, NO₂, CN, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(12) is alkyl having 1, 2, 3 or 4 carbon atoms, alkynyl having 1, 2, 3 or 4 carbon atoms, cycloalkyl having 3, 4, 5 or 6 carbon atoms, phenyl, naphthyl or heteroaryl,
where phenyl, naphthyl and heteroaryl are unsubstituted or substituted by 1, 2 or 3 substituents selected from the group consisting of F, Cl, Br, I, CF₃, OCF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(13) is CₚH₂ₚ-R(14);
p is 0, 1, 2, 3, 4 or 5;
R(15) is cycloalkyl having 3, 4, 5, 6, 7 or 8 carbon atoms;
R(2) is hydrogen or alkyl having 1, 2, 3 or 4 carbon atoms;
R(3) is heteroaryl,
where heteroaryl is unsubstituted or substituted by 1, 2 or 3 substituents selected from the group consisting of F, Cl, Br, I, CF₃, OCF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(4), R(5), R(6) and R(7) are, independently of one another, hydrogen, F, Cl, Br, I, CF₃, OCF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
and the pharmaceutically acceptable salts thereof.

2. A compound of the formula I as claimed in claim 1, in which:
R(1) is 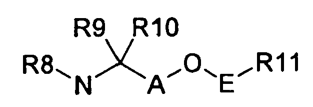
A is -CₙH₂ₙ-;
n is 0, 1, 2 or 3;
E is -CₘH₂ₘ-;
m is 0, 1, 2 or 3;
R(8) is hydrogen, alkyl having 1, 2 or 3 carbon atoms or CₚH₂ₚ-R(14);
p is 0, 1, 2 or 3;
R(14) is phenyl, naphthyl or heteroaryl,
where phenyl, naphthyl and heteroaryl are unsubstituted or substituted by 1, 2 or 3 substituents selected from the group consisting of F, Cl, CF₃, OCF₃, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(9) is hydrogen or alkyl having 1, 2, 3 or 4 carbon atoms;
R(10) is hydrogen, alkyl having 1, 2 or 3 carbon atoms, phenyl, naphthyl or heteroaryl,
where phenyl, naphthyl and heteroaryl are unsubstituted or substituted by 1, 2 or 3 substituents selected from the group consisting of F, Cl, CF₃, OCF₃, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(11) is phenyl, naphthyl, thienyl, furyl, pyridyl, pyrazinyl, pyrimidinyl, pyridazinyl, indolyl, indazolyl, quinolyl, isoquinolyl, phthalazinyl, quinoxalinyl, quinazolinyl or cinnolinyl, where phenyl, naphthyl, thienyl, furyl, pyridyl, pyrazinyl, pyrimidinyl, pyridazinyl, indolyl, indazolyl, quinolyl, isoquinolyl, phthalazinyl, quinoxalinyl, quinazolinyl or cinnolinyl are unsubstituted or substituted by 1, 2 or 3 substituents selected from the group consisting of F, Cl, CF₃, OCF₃, CN, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(2) is hydrogen or alkyl having 1, 2 or 3 carbon atoms;
R(3) is heteroaryl,
where heteroaryl is unsubstituted or substituted by 1, 2 or 3 substituents selected from the group consisting of F, Cl, CF₃, OCF₃, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(4), R(5), R(6) and R(7) are, independently of one another, hydrogen, F, Cl, CF₃, OCF₃, CN, COMe, OH, alkyl having 1, 2, 3 or 4 carbon atoms, methoxy, ethoxy, dimethylamino, sulfamoyl, methylsulfonyl and methylsulfonylamino;
and the pharmaceutically acceptable salts thereof.

3. A compound of the formula I as claimed in claim 1 or 2, in which:
R(1) is 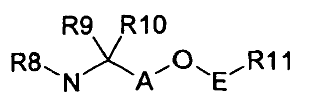
A is -CₙH₂ₙ-;
n is 0 or 1;
E is -CₘH₂ₘ-;
m is 0 or 1;
R(8) is hydrogen, alkyl having 1, 2 or 3 carbon atoms or CₚH₂ₚ-R(14);
p is 0 or 1;
R(14) is phenyl, naphthyl or heteroaryl,
where phenyl, naphthyl and heteroaryl are unsubstituted or substituted by 1 or 2 substituents selected from the group consisting of F, Cl, CF₃, OCF₃, CN, COMe, alkyl having 1, 2, 3 or 4 carbon atoms, methoxy, ethoxy, dimethylamino, sulfamoyl, methylsulfonyl and methylsulfonylamino;
R(9) is hydrogen, methyl or ethyl;
R(10) is hydrogen, alkyl having 1, 2 or 3 carbon atoms, phenyl, naphthyl or heteroaryl,
where phenyl, naphthyl and heteroaryl are unsubstituted or substituted by 1 or 2 substituents selected from the group consisting of F, Cl, CF₃, OCF₃, CN, COMe, alkyl having 1, 2, 3 or 4 carbon atoms, methoxy, ethoxy, dimethylamino, sulfamoyl, methylsulfonyl and methylsulfonylamino;
R(11) is phenyl, naphthyl, pyridyl, pyrazinyl, pyrimidinyl, pyridazinyl, indolyl, indazolyl, quinolyl, isoquinolyl, phthalazinyl, quinoxalinyl, quinazolinyl or cinnolinyl,
where phenyl, naphthyl, pyridyl, pyrazinyl, pyrimidinyl, pyridazinyl, indolyl, indazolyl, quinolyl, isoquinolyl, phthalazinyl, quinoxalinyl, quinazolinyl or cinnolinyl are unsubstituted or substituted by 1 or 2 substituents selected from the group consisting of F, Cl, CF₃, OCF₃, CN, COMe, alkyl having 1, 2, 3 or 4 carbon atoms, methoxy, ethoxy, dimethylamino, sulfamoyl, methylsulfonyl and methylsulfonylamino;
R(2) is hydrogen, methyl or ethyl;
R(3) is heteroaryl,
where heteroaryl is unsubstituted or substituted by 1 or 2 substituents selected from the group consisting of F, Cl, CF₃, OCF₃, CN, COMe, alkyl having 1, 2, 3 or 4 carbon atoms, methoxy, ethoxy, dimethylamino, sulfamoyl, methylsulfonyl and methylsulfonylamino;
R(4), R(5), R(6) and R(7) are, independently of one another, hydrogen, F, Cl, CF₃, OCF₃, CN, COMe, OH, alkyl having 1, 2, 3 or 4 carbon atoms, methoxy, ethoxy, dimethylamino, sulfamoyl, methylsulfonyl and methylsulfonylamino;
and the pharmaceutically acceptable salts thereof.

4. A compound I as claimed in claims 1 to 3, in which
R(1) is 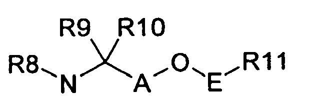
A is -CₙH₂ₙ-;
n is 0 or 1;
E is -CₘH₂ₘ-;
m is 0 or 1;
R(8) is hydrogen, alkyl having 1, 2 or 3 carbon atoms or CₚH₂ₚ-R(14);
p is 0 or 1;
R(14) is phenyl, naphthyl or heteroaryl,
where phenyl, naphthyl and heteroaryl are unsubstituted or substituted by 1 or 2 substituents selected from the group consisting of F, Cl, CF₃, OCF₃, CN, COMe, methyl, methoxy, dimethylamino, sulfamoyl or methylsulfonyl;
R(9) is hydrogen, methyl or ethyl;
R(10) is hydrogen, alkyl having 1, 2 or 3 carbon atoms, phenyl, naphthyl or heteroaryl,
where phenyl, naphthyl and heteroaryl are unsubstituted or substituted by 1 or 2 substituents selected from the group consisting of F, Cl, CF₃, OCF₃, CN, COMe, methyl, methoxy, ethoxy, dimethylamino, sulfamoyl or methylsulfonyl;
R(11) is phenyl, naphthyl, pyridyl, pyrazinyl, pyrimidinyl, pyridazinyl, indolyl, indazolyl, quinolyl, isoquinolyl, phthalazinyl, quinoxalinyl, quinazolinyl or cinnolinyl,
where phenyl, naphthyl, pyridyl, pyrazinyl, pyrimidinyl, pyridazinyl, indolyl, indazolyl, quinolyl, isoquinolyl, phthalazinyl, quinoxalinyl, quinazolinyl or cinnolinyl are unsubstituted or substituted by 1 or 2 substituents selected from the group consisting of F, Cl, CF₃, OCF₃, CN, COMe, methyl, methoxy, ethoxy, dimethylamino, sulfamoyl or methylsulfonyl;
R(2) is hydrogen;
R(3) is heteroaryl,
where heteroaryl is unsubstituted or substituted by 1 or 2 substituents selected from the group consisting of F, Cl, CF₃, OCF₃, CN, COMe, methyl, methoxy, ethoxy, dimethylamino, sulfamoyl or methylsulfonyl;
R(4) is hydrogen, F, Cl, CF₃, methyl or methoxy;
R(5) is hydrogen, F, Cl, CF₃, methyl, methoxy, COMe, OCF₃, CN or OH;
R(6) is hydrogen, F, Cl, CF₃, methyl or methoxy;
R(7) is hydrogen, F, Cl, CF₃, methyl, ethyl, methoxy or OH;
and the pharmaceutically acceptable salts thereof.

5. A compound of the formula I as claimed in claim 1, in which:
R(1) is 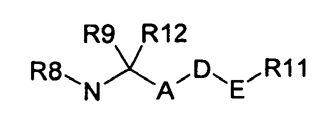
A is -CₙH₂ₙ-;
n is 0, 1, 2 or 3;
D is a bond or -O-;
E is -CₘH₂ₘ-;
m is 0, 1, 2 or 3;
R(8) is hydrogen, alkyl having 1, 2 or 3 carbon atoms or CₚH₂ₚ-R(14)
p is 0, 1, 2, or 3;
R(14) is phenyl, naphthyl or heteroaryl,
where phenyl, naphthyl and heteroaryl are unsubstituted or substituted by 1, 2 or 3 substituents selected from the group consisting of F, Cl, CF₃, OCF₃, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(9) is hydrogen or alkyl having 1, 2, 3 or 4 carbon atoms;
R(11) is phenyl, naphthyl, pyridyl, pyrazinyl, pyrimidinyl, pyridazinyl, indolyl, indazolyl, quinolyl, isoquinolyl, phthalazinyl, quinoxalinyl, quinazolinyl or cinnolinyl
where phenyl, naphthyl, pyridyl, pyrazinyl, pyrimidinyl, pyridazinyl, indolyl, indazolyl, quinolyl, isoquinolyl, phthalazinyl, quinoxalinyl, quinazolinyl or cinnolinyl are unsubstituted or substituted by 1, 2 or 3 substituents selected from the group consisting of F, Cl, CF₃, OCF₃, CN, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(12) is alkyl having 1, 2 or 3 carbon atoms, alkynyl having 1, 2 or 3 carbon atoms, cycloalkyl having 3, 4, 5 or 6 carbon atoms, phenyl, naphthyl or heteroaryl,
where phenyl, naphthyl and heteroaryl are unsubstituted or substituted by 1, 2 or 3 substituents selected from the group consisting of F, Cl, CF₃, OCF₃, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(2) is hydrogen or alkyl having 1, 2 or 3 carbon atoms;
R(3) is heteroaryl,
where heteroaryl is unsubstituted or substituted by 1, 2 or 3 substituents selected from the group consisting of F, Cl, CF₃, OCF₃, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(4), R(5), R(6) and R(7) are, independently of one another, hydrogen, F, Cl, CF₃, OCF₃, CN, COMe, OH, alkyl having 1, 2, 3 or 4 carbon atoms, methoxy, ethoxy, dimethylamino, sulfamoyl, methylsulfonyl and methylsulfonylamino;
and the pharmacologically acceptable salts thereof.

6. A compound I as claimed in claims 1 and/or 5, in which:
R(1) is 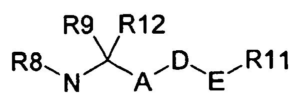
A is -CₙH₂ₙ-;
n is 0 or 1;
D is a bond or -O-;
E is -CₘH₂ₘ-;
m is 0 or 1;
R(8) is hydrogen, alkyl having 1, 2 or 3 carbon atoms or CₚH₂ₚ-R(14)
p is 0 or 1;
R(14) is phenyl, naphthyl or heteroaryl,
where phenyl, naphthyl and heteroaryl are unsubstituted or substituted by 1 or 2 substituents selected from the group consisting of F, Cl, CF₃, OCF₃, CN, COMe, alkyl having 1, 2, 3 or 4 carbon atoms, methoxy, ethoxy, dimethylamino, sulfamoyl, methylsulfonyl and methylsulfonylamino;
R(9) is hydrogen, methyl or ethyl;
R(11) is phenyl, naphthyl, pyridyl, pyrazinyl, pyrimidinyl, pyridazinyl, indolyl, indazolyl, quinolyl, isoquinolyl, phthalazinyl, quinoxalinyl, quinazolinyl or cinnolinyl,
where phenyl, naphthyl, pyridyl, pyrazinyl, pyrimidinyl, pyridazinyl, indolyl, indazolyl, quinolyl, isoquinolyl, phthalazinyl, quinoxalinyl, quinazolinyl or cinnolinyl are unsubstituted or substituted by 1 or 2 substituents selected from the group consisting of F, Cl, CF₃, OCF₃, CN, COMe, alkyl having 1, 2, 3 or 4 carbon atoms, methoxy, ethoxy, dimethylamino, sulfamoyl, methylsulfonyl and methylsulfonylamino;
R(12) is alkyl having 1, 2 or 3 carbon atoms, ethynyl, cyclopropyl, phenyl, naphthyl or heteroaryl,
where phenyl, naphthyl and heteroaryl are unsubstituted or substituted by 1 or 2 substituents selected from the group consisting of F, Cl, CF₃, OCF₃, CN, COMe, alkyl having 1, 2, 3 or 4 carbon atoms, methoxy, ethoxy, dimethylamino, sulfamoyl, methylsulfonyl and methylsulfonylamino;
R(2) is hydrogen, methyl or ethyl;
R(3) is heteroaryl,
where heteroaryl is unsubstituted or substituted by 1 or 2 substituents selected from the group consisting of F, Cl, CF₃, OCF₃, CN, COMe, alkyl having 1, 2, 3 or 4 carbon atoms, methoxy, ethoxy, dimethylamino, sulfamoyl, methylsulfonyl and methylsulfonylamino;
R(4), R(5), R(6) and R(7) are, independently of one another, hydrogen, F, Cl, CF₃, OCF₃, CN, COMe, OH, alkyl having 1, 2, 3 or 4 carbon atoms, methoxy, ethoxy, dimethylamino, sulfamoyl, methylsulfonyl and methylsulfonylamino;
and the pharmacologically acceptable salts thereof.

7. A compound of the formula I as claimed in claims 1, 5 and/or 6, in which:
R(1) is 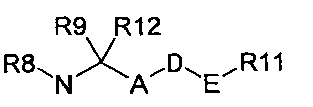
A is -CₙH₂ₙ-;
n is 0 or 1;
D is a bond or -O-;
E is -CₘH₂ₘ-;
m is 0 or 1;
R(8) is hydrogen, alkyl having 1, 2 or 3 carbon atoms or CₚH₂ₚ-R(14);
p is 0 or 1;
R(14) is phenyl, naphthyl or heteroaryl,
where phenyl, naphthyl and heteroaryl are unsubstituted or substituted by 1 or 2 substituents selected from the group consisting of F, Cl, CF₃, OCF₃, CN, COMe, methyl, methoxy, ethoxy, dimethylamino, sulfamoyl, methylsulfonyl and methylsulfonylamino;
R(9) is hydrogen, ethyl or methyl;
R(11) is phenyl, naphthyl, pyridyl, pyrazinyl, pyrimidinyl, pyridazinyl, indolyl, indazolyl, quinolyl, isoquinolyl, phthalazinyl, quinoxalinyl, quinazolinyl or cinnolinyl,
where phenyl, naphthyl, pyridyl, pyrazinyl, pyrimidinyl, pyridazinyl, indolyl, indazolyl, quinolyl, isoquinolyl, phthalazinyl, quinoxalinyl, quinazolinyl or cinnolinyl are unsubstituted or substituted by 1 or 2 substituents selected from the group consisting of F, Cl, CF₃, OCF₃, CN, COMe, methoxy, ethoxy, dimethylamino, sulfamoyl and methylsulfonyl;
R(12) is alkyl having 1, 2 or 3 carbon atoms, ethynyl, cyclopropyl, phenyl, naphthyl or heteroaryl,
where phenyl, naphthyl and heteroaryl are unsubstituted or substituted by 1 or 2 substituents selected from the group consisting of F, Cl, CF₃, OCF₃, CN, COMe, ethoxy, dimethylamino, sulfamoyl, methylsulfonyl and methylsulfonylamino;
R(2) is hydrogen;
R(3) is heteroaryl,
where heteroaryl is unsubstituted or substituted by 1 or 2 substituents selected from the group consisting of F, Cl, CF₃, OCF₃, CN, COMe, methyl, methoxy, ethoxy, dimethylamino, sulfamoyl and methylsulfonyl;
R(4) is hydrogen, F, Cl, CF₃, methyl or methoxy;
R(5) is hydrogen, F, Cl, CF₃, methyl, methoxy, COMe, OCF₃, CN or OH;
R(6) is hydrogen, F, Cl, CF₃, methyl or methoxy;
R(7) is hydrogen, F, Cl, CF₃, methyl, methoxy or OH;
and the pharmacologically acceptable salts thereof.

8. A compound of the formula I as claimed in claim 1, in which:
R(1) is 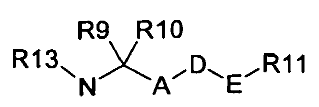 in which:
A is -CₙH₂ₙ-
n = 0, 1, 2 or 3;
D is a bond or -O-;
E is -CₘH₂ₘ-
m is 0, 1, 2 or 3;
R(9) is hydrogen or alkyl having 1, 2, 3 or 4 carbon atoms;
R(10) is hydrogen, alkyl having 1, 2 or 3 carbon atoms, phenyl, naphthyl or heteroaryl
where phenyl, naphthyl and heteroaryl are unsubstituted or substituted by 1, 2 or 3 substituents selected from the group consisting of F, Cl, CF₃, OCF₃, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(11) is phenyl, naphthyl, thienyl, furanyl, pyridyl, pyrazinyl, pyrimidinyl, pyridazinyl, indolyl, indazolyl, quinolyl, isoquinolyl, phthalazinyl, quinoxalinyl, quinazolinyl or cinnolinyl,
where phenyl, naphthyl, thienyl, furanyl, pyridyl, pyrazinyl, pyrimidinyl, pyridazinyl, indolyl, indazolyl, quinolyl, isoquinolyl, phthalazinyl, quinoxalinyl, quinazolinyl or cinnolinyl are unsubstituted or substituted by 1, 2 or 3 substituents selected from the group consisting of F, Cl, CF₃, OCF₃, CN, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(13) is CₚH₂ₚ-R(14);
p is 0, 1, 2 or 3;
R(14) is phenyl, naphthyl or heteroaryl,
where phenyl, naphthyl and heteroaryl are unsubstituted or substituted by 1, 2 or 3 substituents selected from the group consisting of F, Cl, CF₃, OCF₃, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(2) is hydrogen or alkyl having 1, 2 or 3 carbon atoms;
R(3) is heteroaryl,
where heteroaryl is unsubstituted or substituted by 1, 2 or 3 substituents selected from the group consisting of F, Cl, CF₃, OCF₃, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(4), R(5), R(6) and R(7) are, independently of one another, hydrogen, F, Cl, CF₃, OCF₃, CN, COMe, OH, alkyl having 1, 2, 3 or 4 carbon atoms, methoxy, ethoxy, dimethylamino, sulfamoyl, methylsulfonyl and methylsulfonylamino;
and the pharmaceutically acceptable salts thereof.

9. A compound of the formula I as claimed in claims 1 and /or 8, in which:
R(1) is 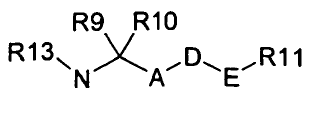
A is -CₙH₂ₙ-;
n is 0 or 1;
D is a bond or -O-;
E is -CₘH₂ₘ-
m is 0 or 1;
R(9) is hydrogen, methyl or ethyl;
R(10) is hydrogen, alkyl having 1, 2 or 3 carbon atoms, phenyl, naphthyl or heteroaryl,
where phenyl, naphthyl and heteroaryl are unsubstituted or substituted by 1 or 2 substituents selected from the group consisting of F, Cl, CF₃, OCF₃, CN, COMe, NH₂, OH, alkyl having 1, 2, 3 or 4 carbon atoms, methoxy, ethoxy, dimethylamino, sulfamoyl, methylsulfonyl and methylsulfonylamino;
R(11) is phenyl, naphthyl, pyridyl, pyrazinyl, pyrimidinyl, pyridazinyl, indolyl, indazolyl, quinolyl, isoquinolyl, phthalazinyl, quinoxalinyl, quinazolinyl or cinnolinyl,
where phenyl, naphthyl, pyridyl, pyrazinyl, pyrimidinyl, pyridazinyl, indolyl, indazolyl, quinolyl, isoquinolyl, phthalazinyl, quinoxalinyl, quinazolinyl or cinnolinyl are unsubstituted or substituted by 1 or 2 substituents selected from the group consisting of F, Cl, CF₃, OCF₃, CN, COMe, alkyl having 1, 2, 3 or 4 carbon atoms, methoxy, ethoxy, dimethylamino, sulfamoyl, methylsulfonyl and methylsulfonylamino;
R(13) is CₚH₂ₚ-R(14);
p is 0 or 1;
R(14) is phenyl, naphthyl or heteroaryl,
where phenyl, naphthyl and heteroaryl are unsubstituted or substituted by 1 or 2 substituents selected from the group consisting of F, Cl, CF₃, OCF₃, CN, COMe, alkyl having 1, 2, 3 or 4 carbon atoms, methoxy, ethoxy, dimethylamino, sulfamoyl, methylsulfonyl and methylsulfonylamino;
R(2) is hydrogen, methyl or ethyl;
R(3) is heteroaryl,
where heteroaryl is unsubstituted or substituted by 1 or 2 substituents selected from the group consisting of F, Cl, CF₃, OCF₃, CN, COMe, alkyl having 1, 2, 3 or 4 carbon atoms, methoxy, ethoxy, dimethylamino, sulfamoyl, methylsulfonyl and methylsulfonylamino;
R(4), R(5), R(6) and R(7) are, independently of one another, hydrogen, F, Cl, CF₃, OCF₃, CN, COMe, OH, alkyl having 1, 2, 3 or 4 carbon atoms, methoxy, ethoxy, dimethylamino, sulfamoyl, methylsulfonyl and methylsulfonylamino;
and the pharmaceutically acceptable salts thereof.

10. A compound of the formula I as claimed in claims 1, 8 and/or 9, in which
R(1) is 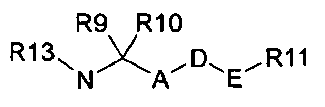
A is -CₙH₂ₙ-;
n is 0 or 1;
D is a bond or -O-;
E is -CₘH₂ₘ-
m is 0 or 1;
R(9) is hydrogen, methyl or ethyl;
R(10) is hydrogen, alkyl having 1, 2 or 3 carbon atoms, phenyl, naphthyl or heteroaryl,
where phenyl, naphthyl and heteroaryl are unsubstituted or substituted by 1 or 2 substituents selected from the group consisting of F, Cl, CF₃, OCF₃, CN, COMe, methyl, methoxy, ethoxy, dimethylamino, sulfamoyl, methylsulfonyl and methylsulfonylamino;
R(11) is phenyl, naphthyl, pyridyl, pyrazinyl, pyrimidinyl, pyridazinyl, indolyl, indazolyl, quinolyl, isoquinolyl, phthalazinyl, quinoxalinyl, quinazolinyl or cinnolinyl,
where phenyl, naphthyl, pyridyl, pyrazinyl, pyrimidinyl, pyridazinyl, indolyl, indazolyl, quinolyl, isoquinolyl, phthalazinyl, quinoxalinyl, quinazolinyl or cinnolinyl are unsubstituted or substituted by 1 or 2 substituents selected from the group consisting of F, Cl, CF₃, OCF₃, CN, COMe, methyl, methoxy, ethoxy, dimethylamino, sulfamoyl and methylsulfonyl;
R(13) is CₚH₂ₚ-R(14);
p is 0 or 1;
R(14) is phenyl, naphthyl or heteroaryl,
where phenyl, naphthyl and heteroaryl are unsubstituted or substituted by 1 or 2 substituents selected from the group consisting of F, Cl, CF₃, OCF₃, CN, COMe, methoxy, ethoxy, dimethylamino, sulfamoyl and methylsulfonyl;
R(2) is hydrogen;
R(3) is heteroaryl,
where heteroaryl is unsubstituted or substituted by 1, 2 or 3 substituents selected from the group consisting of F, Cl, CF₃, OCF₃, CN, COMe, methoxy, ethoxy, dimethylamino, sulfamoyl and methylsulfonyl;
R(4) is hydrogen, F, Cl, CF₃, methyl or methoxy;
R(5) is hydrogen, F, Cl, CF₃, methyl, methoxy, COMe, OCF₃, CN or OH;
R(6) is hydrogen, F, Cl, CF₃, methyl, methoxy or OH
R(7) is hydrogen, F, Cl, CF₃, methyl, ethyl, methoxy or OH;
and the pharmaceutically acceptable salts thereof.

11. A compound of the formula I as claimed in claim 1, in which:
R(1) is 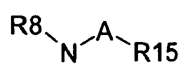
A is -CₙH₂ₙ-;
n = 0, 1, 2 or 3
R(8) is hydrogen, alkyl having 1, 2 or 3 carbon atoms or CₚH₂ₚ-R(14);
p is 0, 1, 2, or 3;
R(14) is phenyl, naphthyl or heteroaryl,
where phenyl, naphthyl and heteroaryl are unsubstituted or substituted by 1, 2 or 3 substituents selected from the group consisting of F, Cl, CF₃, OCF₃, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(2) is hydrogen or alkyl having 1, 2 or 3 carbon atoms;
R(3) is heteroaryl,
where heteroaryl is unsubstituted or substituted by 1, 2 or 3 substituents selected from the group consisting of F, Cl, CF₃, OCF₃, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(4), R(5), R(6) and R(7) are, independently of one another, hydrogen, F, Cl, CF₃, OCF₃, CN, COMe, OH, alkyl having 1, 2, 3 or 4 carbon atoms, methoxy, ethoxy, dimethylamino, sulfamoyl, methylsulfonyl and methylsulfonylamino;
R(15) is cycloalkyl having 3, 4, 5, 6 or 7 carbon atoms;
and the pharmaceutically acceptable salts thereof.

12. A compound of the formula I as claimed in claims 1 and/or 11, in which:
R(1) is 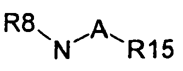
A is -CₙH₂ₙ-;
n is 0 or 1;
R(8) is hydrogen, alkyl having 1, 2 or 3 carbon atoms or CₚH₂ₚ-R(14);
p is 0 or 1;
R(14) is phenyl, naphthyl or heteroaryl,
where phenyl, naphthyl and heteroaryl are unsubstituted or substituted by 1 or 2 substituents selected from the group consisting of F, Cl, CF₃, OCF₃, CN, COMe, alkyl having 1, 2, 3 or 4 carbon atoms, methoxy, ethoxy, dimethylamino, sulfamoyl, methylsulfonyl and methylsulfonylamino;
R(2) is hydrogen, methyl or ethyl;
R(3) is heteroaryl,
where heteroaryl is unsubstituted or substituted by 1 or 2 substituents selected from the group consisting of F, Cl, CF₃, OCF₃, CN, COMe, alkyl having 1, 2, 3 or 4 carbon atoms, methoxy, ethoxy, dimethylamino, sulfamoyl, methylsulfonyl and methylsulfonylamino;
R(4), R(5), R(6) and R(7) are, independently of one another, hydrogen, F, Cl, CF₃, OCF₃, CN, COMe, OH, alkyl having 1, 2, 3 or 4 carbon atoms, methoxy, ethoxy, dimethylamino, sulfamoyl, methylsulfonyl and methylsulfonylamino;
R(15) is cycloalkyl having 3, 4, 5, 6 or 7 carbon atoms;
and the pharmaceutically acceptable salts thereof.

13. A compound of the formula I as claimed in claims 1, 11 and/or 12, in which:
R(1) is 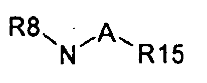
A is -CₙH₂n-;
n is 0 or 1;
R(8) is hydrogen, alkyl having 1, 2 or 3 carbon atoms or CₚH₂ₚ-R(14);
p is 0 or 1;
R(14) is phenyl, naphthyl or heteroaryl,
where phenyl, naphthyl and heteroaryl are unsubstituted or substituted by 1 or 2 substituents selected from the group consisting of F, Cl, CF₃, OCF₃, CN, COMe, methyl, methoxy, ethoxy, dimethylamino, sulfamoyl and methylsulfonyl;
R(2) is hydrogen;
R(3) is heteroaryl,
where heteroaryl is unsubstituted or substituted by 1 or 2 substituents selected from the group consisting of F, Cl, CF₃, OCF₃, CN, COMe, methyl, methoxy, ethoxy, dimethylamino, sulfamoyl or methylsulfonyl;
R(4) is hydrogen, F, Cl, CF₃, methyl or methoxy;
R(5) is hydrogen, F, Cl, CF₃, methyl, methoxy, COMe, OCF₃; CN or OH;
R(6) is hydrogen, F, Cl, CF₃, methyl, methoxy or OH;
R(7) is hydrogen, F, Cl, CF₃, methyl, ethyl, methoxy or OH; R(15) is cycloalkyl having 3, 4, 5, 6 or 7 carbon atoms;
and the pharmaceutically acceptable salts thereof.

14. A compound of the formula I as claimed in one or more of claims 1, 5, 6 or 7, which is: 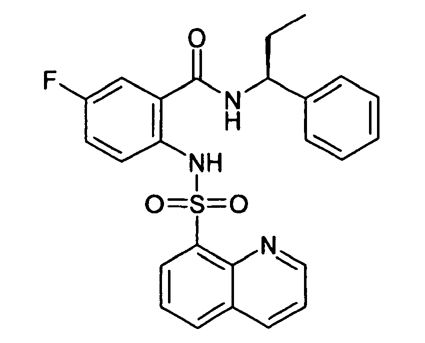 or its sodium salt.

15. A compound of the formula I as claimed in one or more of claims 1 to 14 and the physiologically tolerated salts thereof for use as pharmaceutical.

16. A pharmaceutical preparation comprising an effective amount of at least one compound of the formula I as claimed in one or more of claims 1 to 14 and/or a physiologically tolerated salt thereof as active ingredient together with pharmaceutically acceptable carriers and additives and, where appropriate, also one or more other pharmacological active ingredients.

17. The use of a compound of the formula I as claimed in one or more of claims 1 to 14 and/or of a physiologically tolerated salt thereof for producing a medicament for the therapy or prophylaxis of cardiac arrhythmias which can be abolished by prolongation of the action potential.

18. The use of a compound of the formula I as claimed in one or more of claims 1 to 14 and/or of a physiologically tolerated salt thereof for producing a medicament for the therapy and prophylaxis of reentry arrhythmias.

19. The use of a compound of the formula I as claimed in one or more of claims 1 to 14 and/or of a physiologically tolerated salt thereof for producing a medicament for the therapy and prophylaxis of supraventricular arrhythmias.

20. The use of a compound of the formula I as claimed in one or more of claims 1 to 14 and/or of a physiologically tolerated salt thereof for producing a medicament for the therapy and prophylaxis of atrial fibrillation or atrial flutter.

21. A pharmaceutical preparation comprising an effective amount of at least one compound of the formula I as claimed in one or more of claims 1 to 14 and/or of a physiologically tolerated salt thereof plus a beta-blocker as active ingredients, together with pharmaceutically acceptable carriers and additives.

## Revendications

1. Composés de formule I 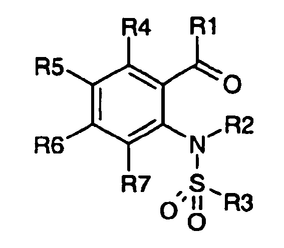 dans laquelle
R(1) signifie 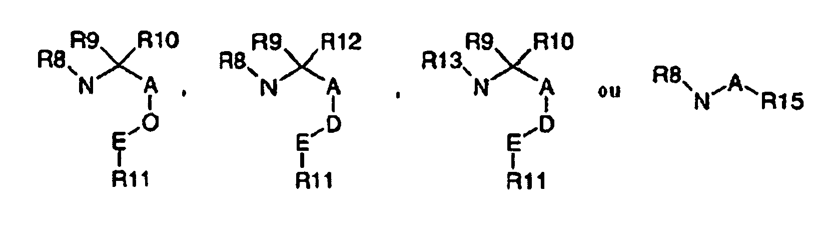
A signifie -CₙH₂ₙ-;
n=0, 1, 2,3,4ou5;
D signifie une liaison ou -O- ;
E signifie -CₘH₂ₘ- ;
m=0, 1,2,3,4 ou 5;
R(8) signifie hydrogène, alkyle comprenant 1, 2, 3 ou 4 atomes de carbone ou CₚH₂ₚ-R(14);
p signifie 0, 1, 2, 3, 4 ou 5 ;
R(14) signifie phényle, naphtyle ou hétéroaryle,
phényle, naphtyle et hétéroaryle étant non substitués ou substitués par 1, 2 ou 3 substituants choisis dans le groupe constitué par F, Cl, Br, I, CF₃, OCF₃, NO₂, CN, COOMe, CONH₂, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(9) signifie hydrogène ou alkyle comprenant 1, 2, 3, 4, 5 ou 6 atomes de carbone ;
R(10) signifie hydrogène, alkyle comprenant 1, 2, 3 ou 4 atomes de carbone, phényle, naphtyle ou hétéroaryle,
phényle, naphtyle et hétéroaryle étant non substitués ou substitués par 1, 2 ou 3 substituants choisis dans le groupe constitué par F, CI, Br, l, CF₃, OCF₃, NO₂, CN, COOMe, CONH₂, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(11) signifie cycloalkyle comprenant 3, 4, 5 ou 6 atomes de carbone, phényle, naphtyle, thiényle, furyle, pyridyle, pyrazinyle, pyrimidinyle, pyridazinyle, indolyle, indazolyle, quinoléyle, isoquinoléyle, phtalazinyle, quinoxalinyle, quinazolinyle ou cinnolinyle,
phényle, naphtyle, thiényle, furyle, pyridyle, pyrazinyle, pyrimidinyle, pyridazinyle, indolyle, indazolyle, quinoléyle, isoquinoléyle, phtalazinyle, quinoxalinyle, quinazolinyle ou cinnolinyle étant non substitués ou substitués par 1, 2 ou 3 substituants choisis dans le groupe formé par F, CI, Br, 1, CF₃, OCF₃, NO₂, CN, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(12) signifie alkyle comprenant 1, 2, 3 ou 4 atomes de carbone, alcynyle comprenant 1, 2, 3 ou 4 atomes de carbone, cycloalkyle comprenant 3, 4, 5 ou 6 atomes de carbone, phényle, naphtyle ou hétéroaryle,
phényle, naphtyle et hétéroaryle étant non substitués ou substitués par 1, 2 ou 3 substituants choisis dans le groupe constitué par F, Cl, Br, l, CF₃, OCF₃, NO₂, CN, COOMe, CONH₂, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(13) signifie CₚH₂ₚ-R(14) ;
p signifie 0, 1, 2, 3, 4 ou 5 ;
R(15) signifie cycloalkyle comprenant 3, 4, 5, 6, 7 ou 8 atomes de carbone ;
R(2) signifie hydrogène ou alkyle comprenant 1, 2, 3 ou 4 atomes de carbone ;
R(3) signifie hétéroaryle,
hétéroaryle étant non substitué ou substitué par 1, 2 ou 3. substituants choisis dans le groupe constitué par F, CI, Br, 1, CF₃, OCF₃, NO₂, CN, COOMe, CONH₂, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(4), R(5), R(6) et R(7) signifient indépendamment l'un de l'autre hydrogène, F, CI, Br, l, CF₃, OCF₃, NO₂, CN, COOMe, CONH₂, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino;
ainsi que leurs sels pharmaceutiquement acceptables.

2. Composés de formule 1 selon la revendication 1, dans laquelle :
R(1) signifie 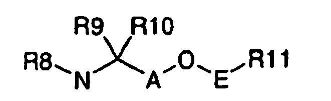
A signifie -CₙH₂ₙ- ;
n signifie 0, 1, 2 ou 3 ;
E signifie -CₘH₂ₘ- ;
m 0, 1, 2 ou 3;
R(8) signifie hydrogène, alkyle comprenant 1, 2 ou 3 atomes de carbone ou CₚH₂ₚ-R(14) ;
p signifie 0, 1, 2 ou 3 ;
R(14) signifie phényle, naphtyle ou hétéroaryle,
phényle, naphtyle et hétéroaryle étant non substitués ou substitués par 1, 2 ou 3 substituants choisis dans le groupe constitué par F, CI, CF₃, OCF₃, CN, COOMe, CONH₂, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(9) signifie hydrogène ou alkyle comprenant 1, 2, 3 ou 4 atomes de carbone ;
R(10) signifie hydrogène, alkyle comprenant 1, 2 ou 3 atomes de carbone, phényle, naphtyle ou hétéroaryle,
phényle, naphtyle et hétéroaryle étant non substitués ou substitués par 1, 2 ou 3 substituants choisis dans le groupe constitué par F, CI, CF₃, OCF₃, CN, COOMe, CONH₂, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(11) signifie phényle, naphtyle, thiényle, furyle, pyridyle, pyrazinyle, pyrimidinyle, pyridazinyle, indolyle, indazolyle, quinoléyle, isoquinoléyle, phtalazinyle, quinoxalinyle, quinazolinyle ou cinnolinyle,
phényle, naphtyle, thiényle, furyle, pyridyle, pyrazinyle, pyrimidinyle, pyridazinyle, indolyle, indazolyle, quinoléyle, isoquinoléyle, phtalazinyle, quinoxalinyle, quinazolinyle ou cinnolinyle étant non substitués ou substitués par 1, 2 ou 3 substituants choisis dans le groupe formé par F, CI, CF₃, OCF₃, CN, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(2) signifie hydrogène ou alkyle comprenant 1, 2 ou 3 atomes de carbone ;
R(3) signifie hétéroaryle,
hétéroaryle étant non substitué ou substitué par 1, 2 ou 3 substituants choisis dans le groupe constitué par F, Cl, CF₃, OCF₃, CN, COOMe, CONH₂, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(4), R(5), R(6) et R(7) signifient, indépendamment l'un de l'autre, hydrogène, F, Cl, CF₃, OCF₃, CN, COMe, OH, alkyle comprenant 1, 2, 3 ou 4 atomes de carbone, méthoxy, éthoxy, diméthylamino, sulfamoyle, méthylsulfonyle et méthylsulfonylamino ;
ainsi que leurs sels pharmaceutiquement acceptables.

3. Composés de formule 1 selon les revendications 1 ou 2, dans laquelle :
R(1) signifie 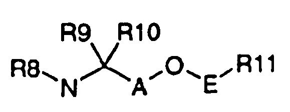
A signifie -CₙH₂ₙ-;
n signifie 0 ou 1
E signifie -CₘH₂ₘ- ;
m signifie 0 ou 1 ;
R(8) signifie hydrogène, alkyle comprenant 1, 2 ou 3 atomes de carbone ou CₚH₂ₚ-R(14);
p signifie 0 ou 1 ;
R(14) signifie phényle, naphtyle ou hétéroaryle,
phényle, naphtyle et hétéroaryle étant non substitués ou substitués par 1 ou 2 substituants choisis dans le groupe constitué par F, CI, CF₃, OCF₃, CN, COMe, alkyle comprenant 1, 2, 3 ou 4 atomes de carbone, méthoxy, éthoxy, diméthylamino, sulfamoyle, méthylsulfonyle et méthylsulfonylamino ;
R(9) signifie hydrogène, méthyle ou éthyle ;
R(10) signifie hydrogène, alkyle comprenant 1, 2 ou 3 atomes de carbone, phényle, naphtyle ou hétéroaryle,
phényle, naphtyle et hétéroaryle étant non substitués ou substitués par 1 ou 2 substituants choisis dans le groupe constitué par F, CI, CF₃, OCF₃, CN, COMe, alkyle comprenant 1, 2, 3 ou 4 atomes de carbone, méthoxy, éthoxy, diméthylamino, sulfamoyle, méthylsulfonyle et méthylsulfonylamino ;
R(11) signifie phényle, naphtyle, pyridyle, pyrazinyle, pyrimidinyle, pyridazinyle, indolyle, indazolyle, quinoléyle, isoquinoléyle, phtalazinyle, quinoxalinyle, quinazolinyle ou cinnolinyle, phényle, naphtyle, pyridyle, pyrazinyle, pyrimidinyle, pyridazinyle, indolyle, indazolyle, quinoléyle, isoquinoléyle, phtalazinyle, quinoxalinyle, quinazolinyle ou cinnolinyle étant non substitués ou substitués par 1 ou 2 substituants choisis dans le groupe formé par F, CI, CF₃, OCF₃, CN, COMe, alkyle comprenant 1, 2, 3 ou 4 atomes de carbone, méthoxy, éthoxy, diméthylamino, sulfamoyle, méthylsulfonyle et méthylsulfonylamino ;
R(2) signifie hydrogène, méthyle ou éthyle ;
R(3) signifie hétéroaryle,
hétéroaryle étant non substitué ou substitué par 1 ou 2 substituants choisis dans le groupe constitué par F, Cl, CF₃, OCF₃, CN, COMe, alkyle comprenant 1, 2, 3 ou 4 atomes de carbone, méthoxy, éthoxy, diméthylamino, sulfamoyle, méthylsulfonyle et méthylsulfonylamino ;
R(4), R(5), R(6) et R(7) signifient, indépendamment l'un de l'autre, hydrogène, F, Cl, CF₃, OCF₃, CN, COMe, OH, alkyle comprenant 1, 2, 3 ou 4 atomes de carbone, méthoxy, éthoxy, diméthylamino, sulfamoyle, méthylsulfonyle et méthylsulfonylamino ;
ainsi que leurs sels pharmaceutiquement acceptables.

4. Composés I selon les revendications 1 à 3, dans laquelle
R(1) signifie 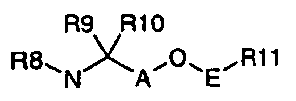
A signifie -CₙH₂ₙ- ;
n = 0 ou 1;
E signifie -CₘH₂ₘ-;
m signifie 0 ou 1 ;
R(8) signifie hydrogène, alkyle comprenant 1, 2 ou 3 atomes de carbone ou CₚH₂ₚ-R(14);
p signifie 0 ou 1 ;
R(14) signifie phényle, naphtyle ou hétéroaryle,
phényle, naphtyle et hétéroaryle étant non substitués ou substitués par 1 ou 2 substituants choisis dans le groupe constitué par F, Cl, CF₃, OCF₃, CN, COMe, méthyle, méthoxy, diméthylamino, sulfamoyle ou méthylsulfonyle ;
R(9) signifie hydrogène, méthyle ou éthyle ;
R(10) signifie hydrogène, alkyle comprenant 1, 2 ou 3 atomes de carbone, phényle, naphtyle ou hétéroaryle,
phényle, naphtyle et hétéroaryle étant non substitués ou substitués par 1 ou 2 substituants choisis dans le groupe constitué par F, Cl, CF₃, OCF₃, CN, COMe, méthyle, méthoxy, éthoxy, diméthylamino, sulfamoyle ou méthylsulfonyle ;
R(11) signifie phényle, naphtyle, pyridyle, pyrazinyle, pyrimidinyle, pyridazinyle, indolyle, indazolyle, quinoléyle, isoquinoléyle, phtalazinyle, quinoxalinyle, quinazolinyle ou cinnolinyle,
phényle, naphtyle, pyridyle, pyrazinyle, pyrimidinyle, pyridazinyle, indolyle, indazolyle, quinoléyle, isoquinoléyle, phtalazinyle, quinoxalinyle, quinazolinyle ou cinnolinyle étant non substitués ou substitués par 1 ou 2 substituants choisis dans le groupe formé par F, Cl, CF₃, OCF₃, CN, COMe, méthyle, méthoxy, éthoxy, diméthylamino, sulfamoyle ou méthylsulfonyle ;
R(2) signifie hydrogène ;
R(3) signifie hétéroaryle,
hétéroaryle étant non substitué ou substitué par 1 ou 2 substituants choisis dans le groupe constitué par F, Cl, CF₃, OCF₃, CN, COMe, méthyle, méthoxy, éthoxy, diméthylamino, sulfamoyle ou méthylsulfonyle ;
R(4) signifie hydrogène, F, Cl, CF₃, méthyle ou méthoxy ;
R(5) signifie hydrogène, F, Cl, CF₃, méthyle, méthoxy, COMe, OCF₃, CN ou OH ;
R(6) signifie hydrogène, F, Cl, CF₃, méthyle ou méthoxy ;
R(7) signifie hydrogène, F, CI, CF₃, méthyle, éthyle, méthoxy ou OH;
ainsi que leurs sels pharmaceutiquement acceptables.

5. Composés de formule 1 selon la revendication 1, dans laquelle :
R(1) signifie 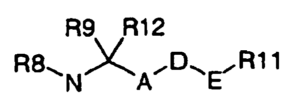
A signifie -CₙH₂ₙ- ;
n signifie 0, 1, 2 ou 3 ;
D signifie une liaison ou -O- ;
E signifie -CₘH₂ₘ- ;
m signifie 0, 1, 2 ou 3 ;
R(8) signifie hydrogène, alkyle comprenant 1, 2 ou 3 atomes de carbone ou CₚH₂ₚ-R(14);
P signifie 0, 1, 2 ou 3 ;
R(14) signifie phényle, naphtyle ou hétéroaryle,
phényle, naphtyle et hétéroaryle étant non substitués ou substitués par 1, 2 ou 3 substituants choisis dans le groupe constitué par F, Cl, CF₃, OCF₃, CN, COOMe, CONH₂, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(9) signifie hydrogène ou alkyle comprenant 1, 2, 3 ou 4 atomes de carbone ;
R(11) signifie phényle, naphtyle, pyridyle, pyrazinyle, pyrimidinyle, pyridazinyle, indolyle, indazolyle, quinoléyle, isoquinoléyle, phtalazinyle, quinoxalinyle, quinazolinyle ou cinnolinyle,
phényle, naphtyle, pyridyle, pyrazinyle, pyrimidinyle, pyridazinyle, indolyle, indazolyle, quinoléyle, isoquinoléyle, phtalazinyle, quinoxalinyle, quinazolinyle ou cinnolinyle étant non substitués ou substitués par 1, 2 ou 3 substituants choisis dans le groupe formé par F, CI, CF₃, OCF₃, CN, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(12) signifie alkyle comprenant 1, 2 ou 3 atomes de carbone, alcynyle comprenant 1, 2 ou 3 atomes de carbone, cycloalkyle comprenant 3, 4, 5 ou 6 atomes de carbone, phényle, naphtyle ou hétéroaryle,
phényle, naphtyle et hétéroaryle étant non substitués ou substitués par 1, 2 ou 3 substituants choisis dans le groupe constitué par F, CI, CF₃, OCF₃, CN, COOMe, CONH₂, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(2) signifie hydrogène ou alkyle comprenant 1, 2 ou 3 atomes de carbone ;
R(3) signifie hétéroaryle,
hétéroaryle étant non substitué ou substitué par 1, 2 ou 3 substituants choisis dans le groupe constitué par F, Cl, CF₃, OCF₃, CN, COOMe, CONH₂, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(4), R(5), R(6) et R(7) signifient, indépendamment l'un de l'autre, hydrogène, F, Cl, CF₃, OCF₃, CN, COMe, OH, alkyle comprenant 1, 2, 3 ou 4 atomes de carbone, méthoxy, éthoxy, diméthylamino, sulfamoyle, méthylsulfonyle et méthylsulfonylamino ;
et leurs sels pharmacologiquement acceptables.

6. Composés I selon les revendications 1 et/ou 5, dans lesquels :
R(1) signifie 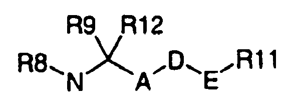
A signifie -CₙH₂ₙ-;
n= 0 ou 1
D signifie une liaison ou -O- ;
E signifie -CₘH₂ₘ-;
m signifie 0 ou 1 ;
R(8) signifie hydrogène, alkyle comprenant 1, 2 ou 3 atomes de carbone ou CₚH₂ₚ-R(14);
P signifie 0 ou 1 ;
R(14) signifie phényle, naphtyle ou hétéroaryle,
phényle, naphtyle et hétéroaryle étant non substitués ou substitués par 1 ou 2 substituants choisis dans le groupe constitué par F, CI, CF₃, OCF₃, CN, COMe, alkyle comprenant 1, 2, 3 ou 4 atomes de carbone, méthoxy, éthoxy, diméthylamino, sulfamoyle, méthylsulfonyle et méthylsulfonylamino ;
R(9) signifie hydrogène, méthyle ou éthyle ;
R(11) signifie phényle, naphtyle, pyridyle, pyrazinyle, pyrimidinyle, pyridazinyle, indolyle, indazolyle, quinoléyle, isoquinoléyle, phtalazinyle, quinoxalinyle, quinazolinyle ou cinnolinyle,
phényle, naphtyle, pyridyle, pyrazinyle, pyrimidinyle, pyridazinyle, indolyle, indazolyle, quinoléyle, isoquinoléyle, phtalazinyle, quinoxalinyle, quinazolinyle ou cinnolinyle étant non substitués ou substitués par 1 ou 2 substituants choisis dans le groupe formé par F, CI, CF₃, OCF₃, CN, COMe, alkyle comprenant 1, 2, 3 ou 4 atomes de carbone, méthoxy, éthoxy, diméthylamino, sulfamoyle, méthylsulfonyle et méthylsulfonylamino ;
R(12) signifie alkyle comprenant 1, 2 ou 3 atomes de carbone, éthynyle, cyclopropyle, phényle, naphtyle ou hétéroaryle, phényle, naphtyle et hétéroaryle étant non substitués ou substitués par 1 ou 2 substituants choisis dans le groupe constitué par F, CI, CF₃, OCF₃, CN, COMe, alkyle comprenant 1, 2, 3 ou 4 atomes de carbone, méthoxy, éthoxy, diméthylamino, sulfamoyle, méthylsulfonyle et méthylsulfonylamino ;
R(2) signifie hydrogène, méthyle ou éthyle ;
R(3) signifie hétéroaryle,
hétéroaryle étant non substitué ou substitué par 1 ou 2 substituants choisis dans le groupe constitué par F, CI, CF₃, OCF₃, CN, COMe, alkyle comprenant 1, 2, 3 ou 4 atomes de carbone, méthoxy, éthoxy, diméthylamino, sulfamoyle, méthylsulfonyle et méthylsulfonylamino ;
R(4), R(5), R(6) et R(7) signifient, indépendamment l'un de l'autre, hydrogène, F, Cl, CF₃, OCF₃, CN, COMe, OH, alkyle comprenant 1, 2, 3 ou 4 atomes de carbone, méthoxy, éthoxy, diméthylamino, sulfamoyle, méthylsulfonyle et méthylsulfonylamino ;
et leurs sels pharmacologiquement acceptables.

7. Composés de formule I selon les revendications 1, 5 et/ou 6, dans laquelle :
R(1) signifie 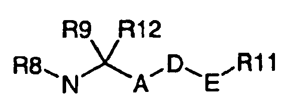
A signifie -CₙH₂ₙ- ;
n signifie 0 ou 1 ;
D signifie une liaison ou -O- ;
E signifie -CₘH₂ₘ- ;
m signifie 0 ou 1 ;
R(8) signifie hydrogène, alkyle comprenant 1, 2 ou 3 atomes de carbone ou CₚH₂ₚ-R(14);
p signifie 0 ou 1 ;
R(14) signifie phényle, naphtyle ou hétéroaryle,
phényle, naphtyle et hétéroaryle étant non substitués ou substitués par 1 ou 2 substituants choisis dans le groupe constitué par F, CI, CF₃, OCF₃, CN, COMe, méthyle, méthoxy, éthoxy, diméthylamino, sulfamoyle, méthylsulfonyle et méthylsulfonyle ;
R(9) signifie hydrogène, éthyle ou méthyle ;
R(11) signifie phényle, naphtyle, pyridyle, pyrazinyle, pyrimidinyle, pyridazinyle, indolyle, indazolyle, quinoléyle, isoquinoléyle, phtalazinyle, quinoxalinyle, quinazolinyle ou cinnolinyle, phényle, naphtyle, pyridyle, pyrazinyle, pyrimidinyle, pyridazinyle, indolyle, indazolyle, quinoléyle, isoquinoléyle, phtalazinyle, quinoxalinyle, quinazolinyle ou cinnolinyle étant non substitués ou substitués par 1 ou 2 substituants choisis dans le groupe formé par F, Cl, CF₃, OCF₃, CN, COMe, méthoxy, éthoxy, diméthylamino, sulfamoyle ou méthylsulfonyle ;
R(12) signifie alkyle comprenant 1, 2 ou 3 atomes de carbone, éthynyle, cyclopropyle, phényle, naphtyle ou hétéroaryle, phényle, naphtyle et hétéroaryle étant non substitués ou substitués par 1 ou 2 substituants choisis dans le groupe constitué par F, CI, CF₃, OCF₃, CN, COMe, éthoxy, diméthylamino, sulfamoyle, méthylsulfonyle et méthylsulfonyle ;
R(2) signifie hydrogène ;
R(3) signifie hétéroaryle,
hétéroaryle étant non substitué ou substitué par 1 ou 2 substituants choisis dans le groupe constitué par F, CI, CF₃, OCF₃, CN, COMe, méthyle, méthoxy, éthoxy, diméthylamino, sulfamoyle ou méthylsulfonyle ;
R(4) signifie hydrogène, F, CI, CF₃, méthyle ou méthoxy ;
R(5) signifie hydrogène, F, CI, CF₃, méthyle, méthoxy, COMe, OCF₃, CN ou OH ;
R(6) signifie hydrogène, F, CI, CF₃, méthyle ou méthoxy ;
R(7) signifie hydrogène, F, CI, CF₃, méthyle, méthoxy ou OH ;
et leurs sels pharmacologiquement acceptables.

8. Composés de formule 1 selon la revendication 1, dans laquelle :
R(1) signifie 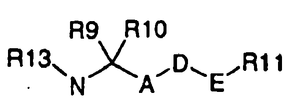 dans laquelle
A signifie -CₙH₂ₙ-
n= 0, 1, 2 ou 3;
D signifie une liaison ou -O- ;
E signifie -CₘH₂ₘ-
m signifie 0, 1, 2 ou 3 ;
R(9) signifie hydrogène ou alkyle comprenant 1, 2, 3 ou 4 atomes de carbone ;
R(10) signifie hydrogène, alkyle comprenant 1, 2 ou 3 atomes de carbone, phényle, naphtyle ou hétéroaryle,
phényle, naphtyle et hétéroaryle étant non substitués ou substitués par 1, 2 ou 3 substituants choisis dans le groupe constitué par F, CI, CF₃, OCF₃, CN, COOMe, CONH₂, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(11) signifie phényle, naphtyle, thiényle, furannyle, pyridyle, pyrazinyle, pyrimidinyle, pyridazinyle, indolyle, indazolyle, quinoléyle, isoquinoléyle, phtalazinyle, quinoxalinyle, quinazolinyle ou cinnolinyle,
phényle, naphtyle, thiényle, furannyle, pyridyle, pyrazinyle, pyrimidinyle, pyridazinyle, indolyle, indazolyle, quinoléyle, isoquinoléyle, phtalazinyle, quinoxalinyle, quinazolinyle ou cinnolinyle étant non substitués ou substitués par 1, 2 ou 3 substituants choisis dans le groupe formé par F, Cl, CF₃, OCF₃, CN, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(13) signifie CₚH₂ₚ-R(14);
p signifie 0, 1, 2 ou 3 ;
R(14) signifie phényle, naphtyle ou hétéroaryle, phényle, naphtyle et hétéroaryle étant non substitués ou substitués par 1, 2 ou 3 substituants choisis dans le groupe constitué par F, Cl, CF₃, OCF₃, CN, COOMe, CONH₂, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(2) signifie hydrogène ou alkyle comprenant 1, 2 ou 3 atomes de carbone ;
R(3) signifie hétéroaryle,
hétéroaryle étant non substitué ou substitué par 1, 2 ou 3 substituants choisis dans le groupe constitué par F, CI, CF₃, OCF₃, CN, COOMe, CONH₂, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(4), R(5), R(6) et R(7) signifient, indépendamment l'un de l'autre, hydrogène, F, Cl, CF₃, OCF₃, CN, COMe, OH, alkyle comprenant 1, 2, 3 ou 4 atomes de carbone, méthoxy, éthoxy, diméthylamino, sulfamoyle, méthylsulfonyle et méthylsulfonylamino ;
ainsi que leurs sels pharmaceutiquement acceptables.

9. Composés de formule I selon les revendications 1 et/ou 8, dans laquelle :
R(1) signifie 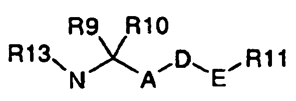
A signifie -CₙH₂ₙ-;
n signifie 0 ou 1 ;
D signifie une liaison ou -O- ;
E signifie -CₘH₂ₘ-
m signifie 0 ou 1 ;
R(9) signifie hydrogène, méthyle ou éthyle ;
R(10) signifie hydrogène, alkyle comprenant 1, 2 ou 3 atomes de
carbone, phényle, naphtyle ou hétéroaryle, phényle, naphtyle et hétéroaryle étant non substitués ou substitués par 1 ou 2 substituants choisis dans le groupe constitué par F, CI, CF₃, OCF₃, CN, COMe, NH₂, OH, alkyle comprenant 1, 2, 3 ou 4 atomes de carbone, méthoxy, éthoxy, diméthylamino, sulfamoyle, méthylsulfonyle et méthylsulfonylamino ;
R(11) signifie phényle, naphtyle, pyridyle, pyrazinyle, pyrimidinyle, pyridazinyle, indolyle, indazolyle, quinoléyle, isoquinoléyle, phtalazinyle, quinoxalinyle, quinazolinyle ou cinnolinyle,
phényle, naphtyle, pyridyle, pyrazinyle, pyrimidinyle, pyridazinyle, indolyle, indazolyle, quinoléyle, isoquinoléyle, phtalazinyle, quinoxalinyle, quinazolinyle ou cinnolinyle étant non substitués ou substitués par 1 ou 2 substituants choisis dans le groupe formé par F, Cl, CF₃, OCF₃, CN, COMe, alkyle comprenant 1, 2, 3 ou 4 atomes de carbone, méthoxy, éthoxy, diméthylamino, sulfamoyle, méthylsulfonyle et méthylsulfonylamino ;
R(13) signifie CₚH₂ₚ-R(14) ;
p signifie 0 ou 1 ;
R(14) signifie phényle, naphtyle ou hétéroaryle,
phényle, naphtyle et hétéroaryle étant non substitués ou substitués par 1 ou 2 substituants choisis dans le groupe constitué par F, CI, CF₃, OCF₃, CN, COMe, alkyle comprenant 1, 2, 3 ou 4 atomes de carbone, méthoxy, éthoxy, diméthylamino, sulfamoyle, méthylsulfonyle et méthylsulfonylamino ;
R(2) signifie hydrogène, méthyle ou éthyle ;
R(3) signifie hétéroaryle,
hétéroaryle étant non substitué ou substitué par 1 ou 2 substituants choisis dans le groupe constitué par F, Cl, CF₃, OCF₃, CN, COMe, alkyle comprenant 1, 2, 3 ou 4 atomes de carbone, méthoxy, éthoxy, diméthylamino, sulfamoyle, méthylsulfonyle et méthylsulfonylamino ;
R(4), R(5), R(6) et R(7) signifient, indépendamment l'un de l'autre, hydrogène, F, Cl, CF₃, OCF₃, CN, COMe, OH, alkyle comprenant 1, 2, 3 ou 4 atomes de carbone, méthoxy, éthoxy, diméthylamino, sulfamoyle, méthylsulfonyle et méthylsulfonylamino ;
ainsi que leurs sels pharmaceutiquement acceptables.

10. Composés de formule 1 selon les revendications 1, 8 et/ou 9, dans laquelle :
R(1) signifie 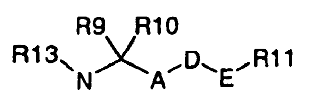
A signifie -CₙH₂ₙ-;
n signifie 0 ou 1 ;
D signifie une liaison ou -O- ;
E signifie -CₘH₂m-
m signifie 0 ou 1 ;
R(9) signifie hydrogène, méthyle ou éthyle ;
R(10) signifie hydrogène, alkyle comprenant 1, 2 ou 3 atomes de carbone, phényle, naphtyle ou hétéroaryle,
phényle, naphtyle et hétéroaryle étant non substitués ou substitués par 1 ou 2 substituants choisis dans le groupe constitué par F, Cl, CF₃, OCF₃, CN, COMe, méthyle, méthoxy, éthoxy, diméthylamino, sulfamoyle, méthylsulfonyle et méthylsulfonyle ;
R(11) signifie phényle, naphtyle, pyridyle, pyrazinyle, pyrimidinyle, pyridazinyle, indolyle, indazolyle, quinoléyle, isoquinoléyle, phtalazinyle, quinoxalinyle, quinazolinyle ou cinnolinyle, phényle, naphtyle, pyridyle, pyrazinyle, pyrimidinyle, pyridazinyle, indolyle, indazolyle, quinoléyle, isoquinoléyle, phtalazinyle, quinoxalinyle, quinazolinyle ou cinnolinyle étant non substitués ou substitués par 1 ou 2 substituants choisis dans le groupe formé par F, CI, CF₃, OCF₃, CN, COMe, méthyle, méthoxy, éthoxy, diméthylamino, sulfamoyle et méthylsulfonyle;
R(13) signifie CₚH₂ₚ-R(14);
p signifie 0 ou 1 ;
R(14) signifie phényle, naphtyle ou hétéroaryle,
phényle, naphtyle et hétéroaryle étant non substitués ou substitués par 1 ou 2 substituants choisis dans le groupe constitué par F, CI, CF₃, OCF₃, CN, COMe, méthoxy, éthoxy, diméthylamino, sulfamoyle et méthylsulfonyle ;
R(2) signifie hydrogène ;
R(3) signifie hétéroaryle,
hétéroaryle étant non substitué ou substitué par 1, 2 ou 3 substituants choisis dans le groupe constitué par F, Cl, CF₃, OCF₃, CN, COMe, méthoxy, éthoxy, diméthylamino, sulfamoyle ou méthylsulfonyle ;
R(4) signifie hydrogène, F, CI, CF₃, méthyle ou méthoxy ;
R(5) signifie hydrogène, F, CI, CF₃, méthyle, méthoxy, COMe, OCF₃, CN ou OH ;
R(6) signifie hydrogène, F, CI, CF₃, méthyle, méthoxy ou OH ;
R(7) signifie hydrogène, F, CI, CF₃, méthyle, éthyle, méthoxy ou OH ;
ainsi que leurs sels pharmaceutiquement acceptables.

11. Composés de formule I selon la revendication 1, dans laquelle :
R(1) signifie 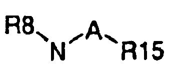
A signifie -CₙH₂ₙ- ;
n = 0, 1, 2 ou 3 ;
R(8) signifie hydrogène, alkyle comprenant 1, 2 ou 3 atomes de carbone ou CₚH₂ₚ-R(14);
p signifie 0, 1, 2 ou 3 ;
R(14) signifie phényle, naphtyle ou hétéroaryle,
phényle, naphtyle et hétéroaryle étant non substitués ou substitués par 1, 2 ou 3 substituants choisis dans le groupe constitué par F, CI, CF₃, OCF₃, CN, COOMe, CONH₂, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(2) signifie hydrogène ou alkyle comprenant 1, 2 ou 3 atomes de carbone ;
R(3) signifie hétéroaryle,
hétéroaryle étant non substitué ou substitué par 1, 2 ou 3 substituants choisis dans le groupe constitué par F, Cl, CF₃, OCF₃, CN, COOMe, CONH₂, COMe, NH₂, OH, alkyle comprenant 1, 2, 3 ou 4 atomes de carbone, alcoxy comprenant 1, 2, 3 ou 4 atomes de carbone, diméthylamino, sulfamoyle, méthylsulfonyle et méthylsulfonylamino ;
R(4), R(5), R(6) et R(7) signifient, indépendamment l'un de l'autre, hydrogène, F, Cl, CF₃, OCF₃, CN, COMe, OH, alkyle comprenant 1, 2, 3 ou 4 atomes de carbone, méthoxy, éthoxy, diméthylamino, sulfamoyle, méthylsulfonyle et méthylsulfonylamino ; R(15) signifie cycloalkyle comprenant 3, 4, 5, 6 ou 7 atomes de carbone ;
ainsi que leurs sels pharmaceutiquement acceptables.

12. Composés de formule I selon les revendications 1 et/ou 11, dans laquelle :
R(1) signifie 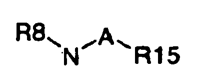
A signifie -C"H₂ₙ ;
n signifie 0 ou 1 ;
R(8) signifie hydrogène, alkyle comprenant 1, 2 ou 3 atomes de carbone ou CₚH₂ₚ-R(14);
p signifie 0 ou 1 ;
R(14) signifie phényle, naphtyle ou hétéroaryle,
phényle, naphtyle et hétéroaryle étant non substitués ou substitués par 1 ou 2 substituants choisis dans le groupe constitué par F, CI, CF₃, OCF₃, CN, COMe, alkyle comprenant 1, 2, 3 ou 4 atomes de carbone, méthoxy, éthoxy, diméthylamino, sulfamoyle, méthylsulfonyle et méthylsulfonylamino ;
R(2) signifie hydrogène, méthyle ou éthyle ;
R(3) signifie hétéroaryle,
hétéroaryle étant non substitué ou substitué par 1 ou 2 substituants choisis dans le groupe constitué par F, CI, CF₃, OCF₃, CN, COMe, alkyle comprenant 1, 2, 3 ou 4 atomes de carbone, méthoxy, éthoxy, diméthylamino, sulfamoyle, méthylsulfonyle et méthylsulfonylamino ;
R(4), R(5), R(6) et R(7) signifient, indépendamment l'un de l'autre, hydrogène, F, CI, CF₃, OCF₃, CN, COMe, OH, alkyle comprenant 1, 2, 3 ou 4 atomes de carbone, méthoxy, éthoxy, diméthylamino, sulfamoyle, méthylsulfonyle et méthylsulfonylamino ; R(15) signifie cycloalkyle comprenant 3, 4, 5, 6 ou 7 atomes de carbone ;
ainsi que leurs sels pharmaceutiquement acceptables.

13. Composés de formule I selon les revendications 1, 11 et/ou 12, dans laquelle :
R(1) signifie 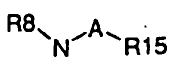
A signifie -CₙH₂ₙ- ;
n signifie 0 ou 1 ;
R(8) signifie hydrogène, alkyle comprenant 1, 2 ou 3 atomes de carbone ou CₚH₂ₚ-R(14) ;
p signifie 0 ou 1 ;
R(14) signifie phényle, naphtyle ou hétéroaryle,
phényle, naphtyle et hétéroaryle étant non substitués ou substitués par 1 ou 2 substituants choisis dans le groupe constitué par F, CI, CF₃, OCF₃, CN, COMe, méthyle, méthoxy, éthoxy, diméthylamino, sulfamoyle et méthylsulfonyle ;
R(2) signifie hydrogène ;
R(3) signifie hétéroaryle,
hétéroaryle étant non substitué ou substitué par 1 ou 2 substituants choisis dans le groupe constitué par F, Cl, CF₃, OCF₃, CN, COMe, méthyle, méthoxy, éthoxy, diméthylamino, sulfamoyle ou méthylsulfonyle ;
R(4) signifie hydrogène, F, Cl, CF₃, méthyle ou méthoxy ;
R(5) signifie hydrogène, F, Cl, CF₃, méthyle, méthoxy, COMe, OCF₃, CN ou OH ;
R(6) signifie hydrogène, F, Cl, CF₃, méthyle, méthoxy ou OH;
R(7) signifie hydrogène, F, Cl, CF₃, méthyle, éthyle, méthoxy ou OH;
R(15) signifie cycloalkyle comprenant 3, 4, 5, 6 ou 7 atomes de carbone ;
ainsi que leurs sels pharmaceutiquement acceptables.

14. Composé de formule I selon l'une ou plusieurs des revendications 1, 5, 6 ou 7, qui est: 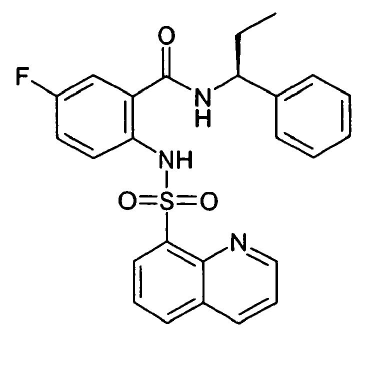 ainsi que leur sel de sodium.

15. Composés de formule I selon l'une ou plusieurs des revendications 1 à 14 et leurs sels physiologiquement acceptables pour une utilisation comme médicament.

16. Préparation pharmaceutique, contenant une quantité active d'au moins un composé de formule I selon l'une ou plusieurs des revendications 1 à 14 et/ou d'un sel physiologiquement acceptable de celui-ci comme substance active avec des supports et des additifs pharmaceutiquement acceptables et le cas échéant encore une ou plusieurs autres substances actives pharmacologiques.

17. Utilisation d'un composé de formule I selon l'une ou plusieurs des revendications 1 à 14 et/ou d'un sel physiologiquement acceptable de celui-ci pour la préparation d'un médicament destiné à la thérapie ou la prophylaxie de troubles du rythme cardiaque, qui peuvent être supprimés par un allongement du potentiel d'action.

18. Utilisation d'un composé de formule I selon l'une ou plusieurs des revendications 1 à 14 et/ou d'un sel physiologiquement acceptable de celui-ci pour la préparation d'un médicament destiné à la thérapie ou la prophylaxie d'arythmies par réentrée.

19. Utilisation d'un composé de formule I selon l'une ou plusieurs des revendications 1 à 14 et/ou d'un sel physiologiquement acceptable de celui-ci pour la préparation d'un médicament destiné à la thérapie ou la prophylaxie d'arythmies supraventriculaires.

20. Utilisation d'un composé de formule I selon l'une ou plusieurs des revendications 1 à 14 et/ou d'un sel physiologiquement acceptable de celui-ci pour la préparation d'un médicament destiné à la thérapie ou la prophylaxie d'une fibrillation atriale ou d'une arythmie atriale.

21. Préparation pharmaceutique, contenant une quantité active d'au moins un composé de formule I selon l'une ou plusieurs des revendications 1 à 14 et/ou d'un sel physiologiquement acceptable de celui-ci ainsi qu'un bêtabloquant comme substances actives, avec des supports et des additifs pharmaceutiquement acceptables.
